# EUROPEAN PATENT SPECIFICATION

(11) **EP 1 682 666 B1**
(45) Date of publication and mention of the grant of the patent: **31.12.2008**
(21) Application number: 04768582.1
(22) Date of filing: 23.09.2004
(51) Int. Cl.: C12N 15/62, C12N 15/49, C07K 14/16, A61K 39/21

(54) **HIV Pharmaceutical vaccines**
Pharmazeitische Impfstoffe gegen HIV
VACCINS PHARMACEUTIQUES CONTRE LE VIH

(30) Priority: 24.09.2003 GB 0322402; 26.09.2003 GB 0322637; 27.10.2003 GB 0325011
(43) Date of publication of application: 26.07.2006
(73) Proprietor: Oxxon Therapeutics Limited, Oxford, Oxfordshire OX14 4SH (GB)
(72) Inventor: SCHNEIDER, Joerg, Barton, Oxford OX3 8BT (GB)
(74) Representative: Harding, Charles Thomas
(86) International application number: PCT/GB2004/004038
(87) International publication number: WO 2005/030964

(56) References cited:
- WO-A-01/47955
- WO-A-02/32943
- KMIECIAK D ET AL: "Enhancement of cellular and humoral immune responses Human Immunodeficiency Virus Type 1 Gag and Pol by a G/P-92 fusion protein expressing highly immunogenic Gag p17/p24 and Pol p51 Antigens" JOURNAL OF HUMAN VIROLOGY, PHILADELPHIA, PA, US, vol. 4, no. 6, 2001, pages 306-316, XP008024317 ISSN: 1090-9508

## Description

### FIELD OF THE INVENTION

The invention relates to polypeptides comprising HIV antigens, in particular to polypeptides comprising mutated HIV sequences, said mutated sequences retaining their naturally occurring CD8+ epitopes. Furthermore, the invention relates to mutated HIV sequences into which extra T helper epitopes have been introduced.

### BACKGROUND TO THE INVENTION

HIV is a pathogenic virus leading to debilitating and fatal immune deficiencies such as AIDS. Although there are certain therapies for conditions such as AIDS which can prolong life expectancy and increase quality of life for affected individuals, the disease is usually terminal.

Eliminating or controlling the virus in HIV infected patients is a problem.

Prevention of infection with HIV is a problem which has thus far been mostly countered by social solutions such as modification of sexual behaviour and/or greater take up of sterile practices for users of hypodermic needles.

Medical prevention of HIV infection remains a high priority area of research. There is a need for effective HIV vaccination strategies.

Even when immune responses are mounted against certain element(s) of an HIV particle, there are problems of immunodominance, viral escape and presentation of viral proteins which can lead to amelioration of the response. Clearly, it is extremely hazardous to render HIV particles for use in vaccines. Furthermore, use of proteins identical to natural proteins may itself be problematic if these proteins have undesirable effects. Furthermore, the proteins may themselves have immunosuppressive qualities and/or may lack sufficient CD8+ T cell epitopes for a suitably strong or broad immune response to be provoked.

Viral escape by mutation of CD8+ T cell epitopes, especially immunodominant CD8+ T cell epitopes, is a significant problem in viral vaccination. Existing vaccines can exhibit poor recruitment of T helper cells and hence produce a narrow and/or weak immune response. Natural viral sequences, subject to evolutionary pressures in vivo, may possess relatively few immunologically significant epitopes. Furthermore, immunodominant effects can skew the clinical importance of certain CD8+ T cell epitopes and focus the response (if any) on these. Clearly, this serves to accentuate immunodominance problems, for example leading to sub selection of viral lines which mutate at this epitope, leading to potentially fatal viral escape (eg. see Barouch et al. 2002 Nature 415 p.335).

WO02/32943 (Nabel and Huang) disclose modifications of HIV ENV, GAG and POL to enhance immunogenicity for genetic immunization. In particular, they focus on modification of glycosylation of ENV, and on nucleic acid constructs encoding delta CFI HIV ENV. Furthermore, specific deletions of ENV cleavage site, fusogenic domain, and spacing of heptad repeats 1 and 2 are disclosed. Immunization with DNA plasmids encoding GAG alone or GAG-POL is described, the best results being alleged for the gag-pol plasmid immunisation. The bulk of WO02/32943 is concerned with the disclosure of specific plasmids listed in table 1 and the claims of WO02/32943. Also claimed are 'analogs' of various parts of these plasmids, and segments having at least 95% sequence identity thereto. To disrupt functions of Nef such as limitation of MHC class I and/or CD4 expression in WO02/32943, point mutations were introduced into the Nef gene from HIV-1 PV22. PV22 corresponds with the HXB2 reference sequence (see example 8) at the positions of mutations. Thus it can be seen that the mutations of WO02/32943 destroy naturally occurring epitopes. For example, amino acid substitutions in the Nef polypeptide of construct VRC4301 are: P69A, P72A, P75A, P78A, D174A and D175A. Each of the 6 mutations lies within a defined epitope with reference to Example 8, and therefore the naturally occurring epitopes have not been retained in this modified Nef gene. In constructs VRC 4306, VRC 4309, VRC 4310, VRC 4311, VRC 4312 and similar construacts in WO02/32943, the reverse transcriptase mutation made (D771H) falls within the YMDD motif (shown in epitope map as RT 183-186) which is an area with a number of recorded epitopes. Thus these epitopes have not been retained in these prior art constructs. Furthermore, synthetic gag/pol/nef genes disclosed in WO02/32943 have been made by truncating fusions such as described for VRC 4312 and similar constructs. With reference to VRC 4312, the synthetic gag gene was ligated in frame with sequences encoding synthetic nef gene that 51 aa were deleted from 5'; 77 aa were deleted from 5' of pol polyprotein, and ligated with 3' of nef in which tag stop codon was deleted. These truncations of pol and nef result in loss of epitopes and so the naturally occurring epitopes have not been retained in terms of full length gag/pol/nef genes.

WO02/022080 (Merck and Co.) disclose modifications of HIV1-Gag, Pol and Nef. The principal modifications described are codon optimisation of the Gag, Pol and Nef genes. There is no mention of mutation of Gag polypeptide, only codon optimisation of the nucleic acid encoding it. Example 17 of WO02/022080 presents various mutations of the Pol protein; nine point mutations are presented, three of which are localised to each of the reverse transcriptase, RNAse and integrase regions of the protein. At least the D112A, D187A, D188A, D445A and D500A mutations of HIV-1 Pol are within human epitopes and therefore the naturally occurring epitopes have not been retained. At least the 'LLAA' mutations of FHV-1 and IRV-1 Nef (ie. L164A, L165A double mutant) are within human epitopes and therefore the naturally occurring epitopes have not been retained in this mutated Nef polypeptide.

WO03/025003 discloses various altered gag gene constructs, and also mention nef and pol gene constructs. The nef gene constructs appear to be truncated removing epitopes from the N-terminus of nef. The gag gene constructs appear not to be mutated.

The present invention seeks, *inter alia*, to overcome some of the problem(s) discussed above.

### SUMMARY OF THE INVENTION

The present invention is based on the design of particularly effective presentation of HIV derived CD8+ T cell epitopes. In this way, the strongest and broadest immune response can be produced.

This is accomplished by altering the context of the polypeptide on which the epitopes are carried. In this way, the natural biological function(s) of the viral polypeptides can be destroyed, advantageously rendering them safe for vaccination use, whilst carefully preserving naturally occurring epitopes which prior art techniques are known to destroy.

In this and other aspects, supplementary (ie. non-naturally occurring) T helper epitopes are also introduced into the antigenic polypeptides, thereby advantageously strengthening and broadening the immune response.

Thus, in one aspect the invention provides a recombinant polypeptide comprising amino acid sequence derived from
(i) an HIV gag gene product comprising the sequence set forth in SEQ ID NO. 2 & SEQ ID NO. 3;
(ii) an HIV pol gene product comprising the sequence set forth in SEQ ID NO. 5; and
(iii) an HIV nef gene product comprising the sequence set forth in SEQ ID NO. 10,

The recombinant polypeptide as described above in which the amino acid sequences are derived from (i) and (ii) and (iii) may be arranged in the order (i) - (ii) - (iii) from the N terminus to the C terminus of the polypeptide.

There is also described a recombinant polypeptide comprising amino acid sequence derived from
(i) an HIV gag gene product;
(ii) an HIV pol gene product; and
(iii) an HIV nef gene product,
said sequence being mutated with respect to the natural sequence of said gene product, and said sequence maintaining substantially all of the naturally occurring CD8+ T cell epitopes of said gene product as defined in p17 and p24 (gag), amino acids 1-440 of RT (pol) and nef shown in Example 8, said polypeptide comprising amino acid sequence having at least 75% identity to SEQ ID NO:9. Preferably said polypeptide comprises amino acid sequence having at least 95% identity to SEQ ID NO:9.

In another aspect, the sequence identity is interepitope sequence identity. This means that the epitopes correspond with 100% sequence identity to the epitopes in the natural or reference sequence, and the sequence identity quoted is for the remaining non-epitope regions of the sequence ie. the interepitope regions. Preferably this is calculated across all interepitope regions present. Preferably 'epitope' refers to the linear amino acid sequence to which the epitope has been mapped, preferably with reference to the epitopes mapped in Example 8.

In another aspect, the invention relates to a recombinant polypeptide as described above comprising SEQ ID NO:9, or a sequence having at least 95% identity thereto.

The recombinant polypeptide as described above may further comprise an antibody recognition tag.

The tag may be an HA tag comprising the sequence as shown in SEQ ID NO:8.

The recombinant polypeptide may further comprise a CD8+ T cell epitope tag.

The recombinant polypeptide may comprise a gp160 derived tag comprising the sequence as shown in SEQ ID NO:7.

The recombinant polypeptide as described above, may comprise the sequence as shown in SEQ ID NO:1.

There is also described a recombinant polypeptide as described above, said polypeptide comprising amino acid sequence derived from an HIV nef gene product, said recombinant polypeptide sequence being mutated to disrupt the function of said nef sequence, said nef sequence further comprising one or more T helper epitopes which are not present in the naturally occurring nef gene.

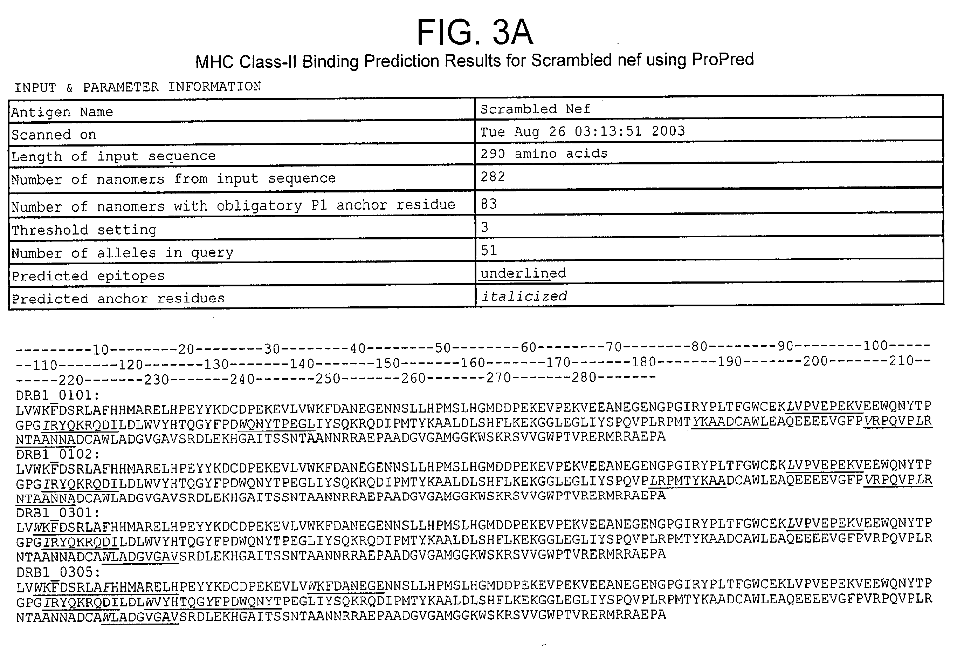

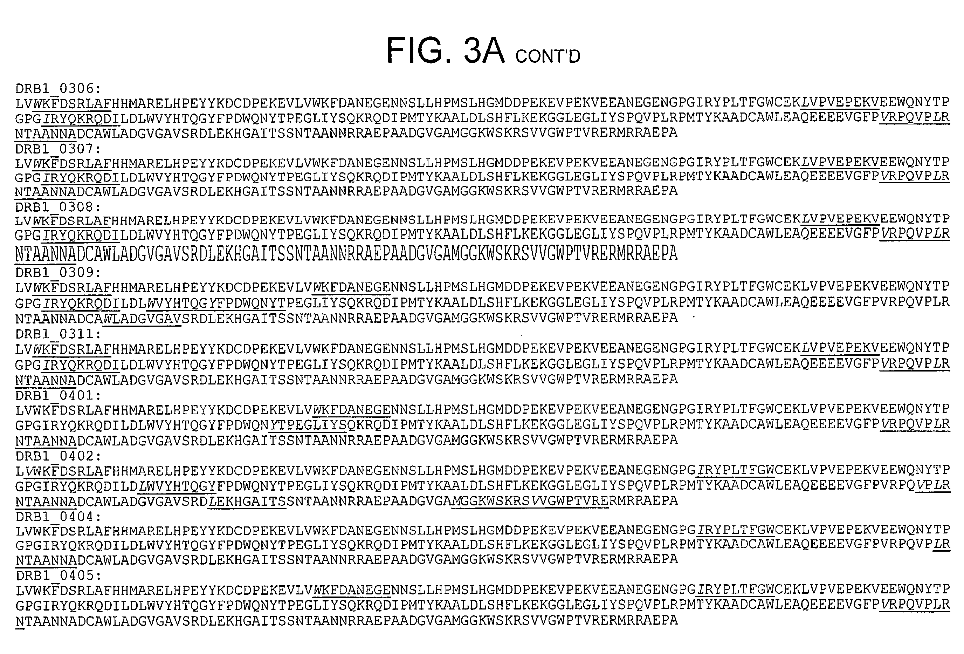

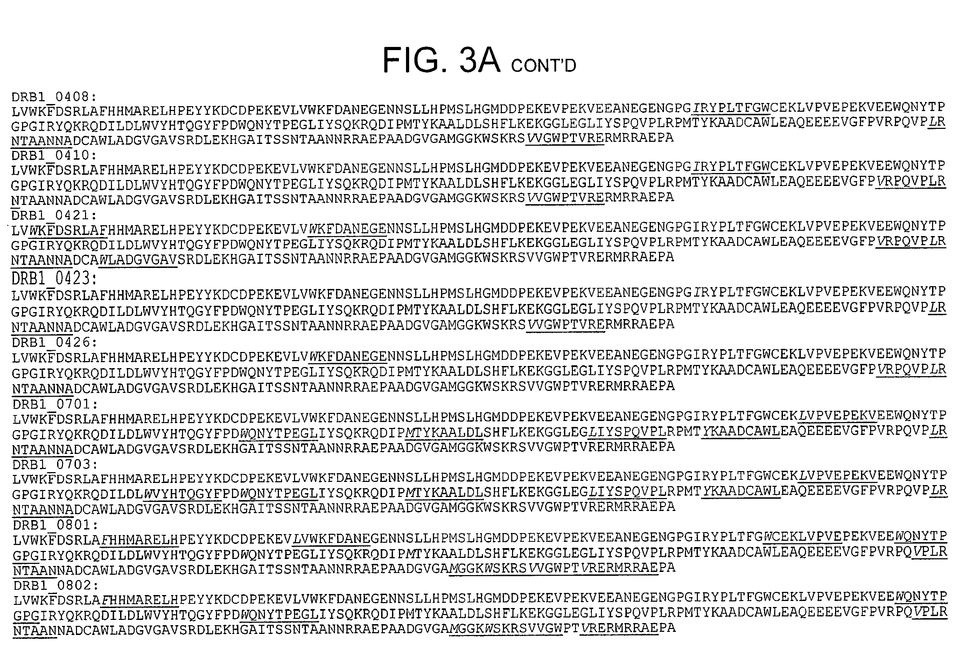

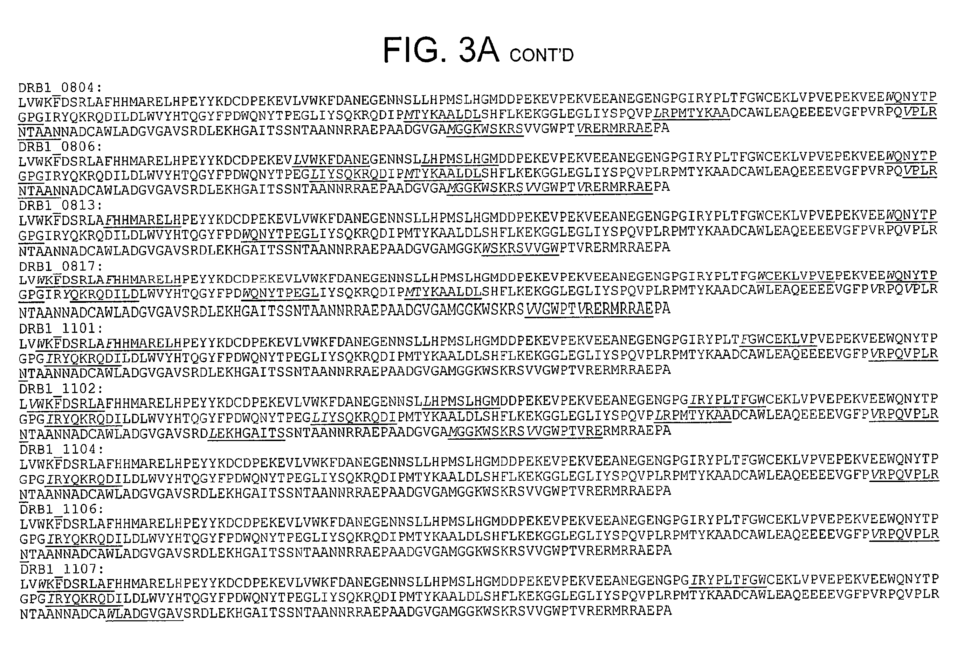

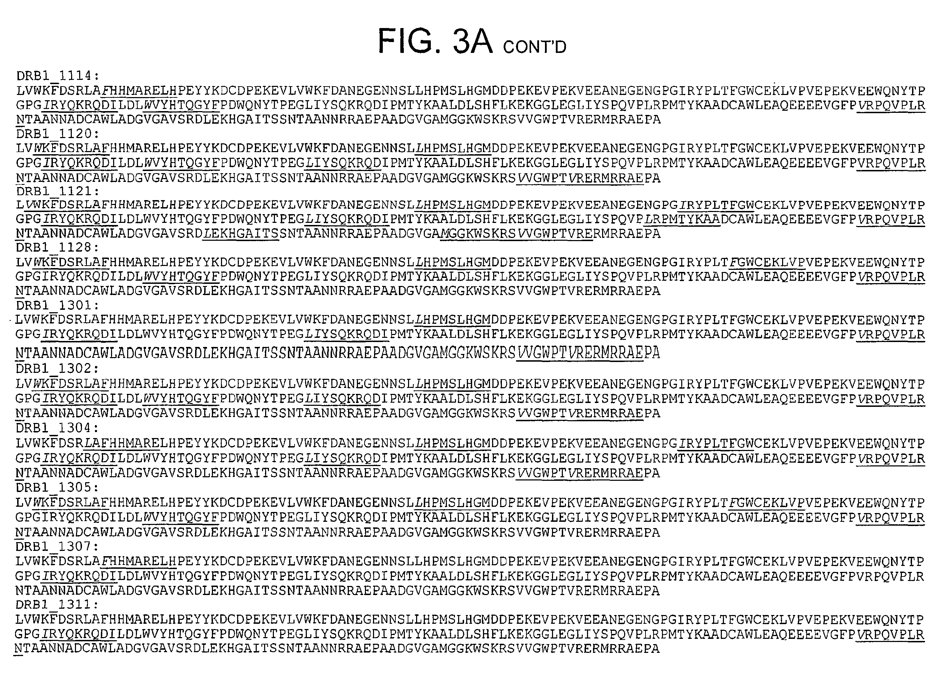

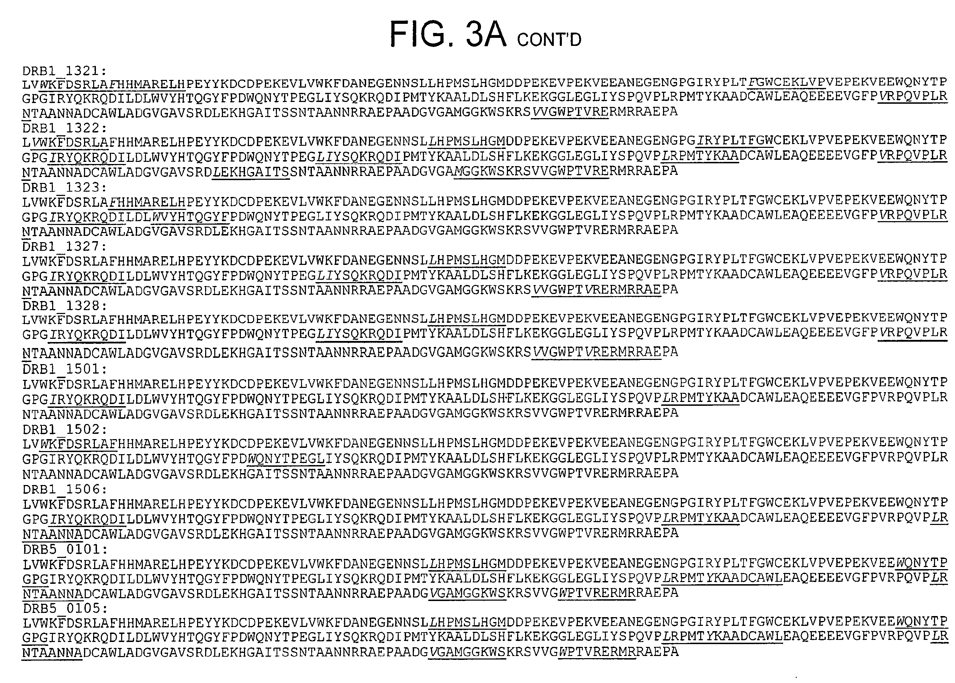

The recombinant polypeptide as described above may comprise one or more T helper epitopes which are not present in the naturally occurring nef sequence and are shown in Figure 3A.

The recombinant polypeptide as described above may further comprise substantially all of the naturally occurring nef CD8+ T cell epitopes as defined in Example 8.

The recombinant polypeptide as described above may further comprise substantially all of the naturally occurring nef T helper epitopes as defined in Example 8.

The recombinant polypeptide as described above may comprise sequence as shown in SEQ ID NO:6, or a sequence having at least 95% identity thereto.

There is also described a recombinant polypeptide as described above, said polypeptide comprising amino acid sequence derived from an HIV pol gene product, said recombinant polypeptide sequence being mutated to disrupt the reverse transcriptase activity of the pol sequence, wherein substantially all of the CD8+ T cell epitopes of the naturally occurring po1 sequence as defined in amino acids 1-440 of RT (pol) shown in Example 8 are retained in said recombinant polypeptide.

There is also described a recombinant polypeptide as described above, wherein the reverse transcriptase activity of said pol sequence is mutated by duplication of an internal sequence derived from the centre of the naturally occurring pol gene and exchange of the amino and carboxy terminal portions of said pol sequence.

There is also described a recombinant polypeptide as described above wherein said duplicated internal sequence comprises TPDKKHQKEPPF (SEQ ID NO:4).

There is also described a recombinant polypeptide as described above wherein said polypeptide comprises sequence as shown in SEQ ID NO:12 or a sequence having at least 95% identity thereto.

There is also described a recombinant polypeptide as described above, said polypeptide comprising amino acid sequence derived from an HIV gag gene product, said recombinant polypeptide sequence being mutated to disrupt processing of the gag gene product, and said gag sequence further comprising a disrupted myristoylation site, wherein substantially all of the CD8+ T cell epitopes of the naturally occurring gag sequence as defined in p17 and p24 (gag) shown in Example 8 are retained in said recombinant polypeptide.

There is also described a recombinant polypeptide as described above wherein the processing of gag is disrupted by exchanging the p17 and p24 domains and wherein the myristoylation site is disrupted by mutation of the second glycine to alanine.

There is also described a recombinant polypeptide as described above wherein said polypeptide comprises sequence as shown in SEQ ID NO:13 or a sequence having at least 95% identity thereto.

There is also described a recombinant polypeptide as described above wherein the HIV is a clade B HIV.

There is also described a recombinant nucleic acid encoding a polypeptide as described above.

In another aspect, the invention relates to a recombinant nucleic acid sequence comprising SEQ ID NO:11, or a sequence which differs only by silent mutations with respect to the genetic code, or a sequence having at least 95% identity thereto.

In another aspect, the invention relates to a viral vector encoding a polypeptide as described above.

In another aspect, the invention relates to a viral vector as described above wherein said vector is an MVA or MVA derived vector.

In another aspect, the invention relates to a viral vector as described above wherein said vector is a fowlpox or fowlpox derived vector.

In another aspect, the invention relates to a viral vector as described above wherein said vector is a FP9 fowlpox vector. Specific teachings with regard to this vector may be found in WO03/047617.

There is also described the use of a polypeptide as described above in medicine.

There is also described the use of a polypeptide as described above in the preparation of a medicament for the treatment or prevention of HIV infection.

In another aspect, the invention relates to the use of polypeptide as described above in the preparation of a medicament for immunisation against HIV infection.

There is also described the use of a nucleic acid as described above in medicine.

There is also described the use of nucleic acid as described above in the preparation of a medicament for the treatment or prevention of HIV infection.

There is also described the use of nucleic acid as described above in the preparation of a medicament for immunisation against HIV infection.

There is also described the use of a polypeptide or nucleic acid as described above as a priming agent or as a boosting agent in a prime-boost immunisation regime. Prime boost immunisation is well known in the art, and specific teachings on this subject may be taken from WO98/056919.

There is also described the use of a polypeptide or nucleic acid as described herein in the induction of an immune response. Said immune response may be, for example, a cellular immune response, such as a CD8+ or CD4+ response, or a humoral (antibody) response.

There is also described a nucleic acid vector comprising a nucleic acid sequence as described above or encoding a polypeptide as described above.

There is also described an adenovirus vector comprising a nucleic acid sequence as described above or encoding a polypeptide as described above.

There is also described a vector based on VSV (vesicular stomatitis virus), adeno-associated virus (AAV), alphavirus, Sendai virus or Herpes Simplex virus comprising a nucleic acid sequence as described above or encoding a polypeptide as described above.

There is also described a poxvirus vector comprising a nucleic acid sequence as described above or encoding a polypeptide as described above.

There is also described a plasmid selected from the group consisting of p29D.gpn, pOPK6.gpn and pSG2.gpn.

### DETAILED DESCRIPTION OF THE INVENTION

### DEFINITIONS

As used herein, the term "adenovirus" comprises the members of the Adenoviridae (adenovirus family). This family, in turn, comprises three genera: Mastadenovirus, Aviadenovirus and ATadenovirus. In particular, the invention contemplates the use of ovine adenovirus (an ATadenovirus).

A "CD8+ T cell epitope" is an amino acid sequence which is a peptide recognised by CD8+ T cells usually in conjunction with a class I major histocompatibility complex. In particular, reference to "all" CD8+ T cell epitopes, and/or "all known" CD8+ T cell epitopes, refers to currently known epitopes, as defined in Example 8 hereto, HIV Molecular Immunology 2002: Maps of CTL Epitope Locations Plotted by Protein; Theoretical Biology & Biophysics, Los Alamos National Laboratory, August 7, 2003. In accordance with the present invention, substantially all human CD8+ T cell epitopes are retained in the modified polypeptides; however, CD8+ T cell epitopes relevant in other mammalian species, such as murine CD8+ T cell epitopes, may be lost. CD8+ T cells are synonymous with CTLs (cytotoxic T-lymphocytes).

A "T helper epitope", likewise, is a peptide recognised by T helper cells usually in conjunction with a class II major histocompatibility complex; "all" and/or "all known" T helper cell epitopes are as defined in Example 8, HIV Molecular Immunology 2002: Maps of CTL Epitope Locations Plotted by Protein; Theoretical Biology & Biophysics, Los Alamos National Laboratory, August 7, 2003. In accordance with the present invention, substantially all human T helper cell epitopes are retained in the modified polypeptides; however, T helper epitopes relevant in other mammalian species, such as murine T helper epitopes, may be lost. A T helper cell is synonynous with a helper T cell.

"Substantially all" means at least 99%; preferably, it means at least 98%, 97%, 96%, 95%, 94%, 93%, 92%, 91%, 90%, 89%, 88%, 87%, 86% or 85%. In an alternative embodiment, 'substantially all' refers to all except one, all except two, all except three, all except four, all except five, all except six, all except seven, all except eight, all except nine, all except ten, all except eleven, all except twelve, all except thirteen, all except fourteen, all except fifteen.

An epitope is considered to be 'lost' or 'destroyed' or 'not retained' if the peptide sequence to which it is mapped, for example with reference to Example 8, is mutated. Preferably all epitopes are retained, with reference to those shown in Example 8.

A 'natural' sequence or 'reference' sequence as used herein refers to the source sequence from which the claimed polypeptide or nucleotide sequence(s) are designed or derived. Wherever possible this term should take its ordinary meaning ie. referring to the sequence of the corresponding gene(s) in a virus found in nature. However, it will be apparent to a person skilled in the art that there is not a single HIV virus found in nature, but rather there are many different clades and many different isolates or clones within those clades. Therefore, when a particular sequence according to the invention has been designed or derived from a particular clone or isolate of HIV, then the 'natural' or 'reference' sequence refers to that particular clone or isolate's own sequence. However, there are numerous consensus sequences known in the art which have been formed and indeed are sometimes updated by the comparison or 'pileup' of a number of different isolate or clonal sequences which may be divergent at one or more nucleotide or amino acid positions. In the situation where a construct according to the present invention has been designed or derived from such a consensus sequence, then this consensus sequence will be taken to be the natural or reference sequence.

Preferably all epitopes for the 'full length' g/p/n gene products as described herein are retained. Preferably all said epitopes are retained even when non-epitope containing regions of the gene product are truncated or deleted.

The natural or reference sequence should preferably be considered on a gene-by-gene basis. For example, a construct according to the present invention could comprise a gag gene, a nef gene and a pol gene. These genes will each have a natural or reference sequence. It may be that the natural or reference sequences for each gene will be from the same source eg. the same overall consensus sequence, or it may be that the natural or reference sequence for each will be from a different source, eg. the gag and pol reference sequences may be from the 2001 clade B consensus sequence whilst the nef reference sequence may be from the 2000 clade B consensus sequence or vice versa. Reference sequences (both consensus and clonal) may be found in the Los Alamos Database as discussed herein (HIV Molecular Immunology 2002: Maps of CTL Epitope Locations Plotted by Protein; Theoretical Biology & Biophysics, Los Alamos National Laboratory, August 7, 2003). Specifically the 2001 clade B consensus sequence may be found at http://www.hiv.lanl.gov/content/hiv-db/ALIGN 02/ALIGN-INDEX.html and the 2000 clade B consensus sequence may be found at http://www.hiv.lanl.aov/content/hiv-db/ALIGN 01/ALIGN-INDEX.html.

"HIV" is Human Immunodeficiency Virus, a virus that causes immunodeficiency by attacking CD4+ cells in the body. The term "HIV", as used herein, includes any HIV, including all groups and subtypes (clades) of HIV-1 and HIV-2, for example HIV-1 M and HIV-1 O groups; the invention embraces each of the known clades; Clade B HIV-1 is preferred. Gag, pol and nef gene products are well known in the art and are as defined, for instance, in Example 8 hereto.

Guidance on the application of the invention to different clades may also be found in HIV Sequence Compendium 2002 Kuiken C, Foley B, Freed E, HahnB, Marx P, McCutchan F, Mellors J, Wolinsky S, and Korber B,editors. Published by the Theoretical Biology and Biophysics Group, Los Alamos National Laboratory, LA-UR number 03-3564, incorporated herein by reference. In particular, consensus sequence data for HIV-1 clades A, B, C and D and a number of isolates can be found on pages 490 to 550; consensus sequence data for HIV-2 clades A, B, C and D and a number of isolates can be found one pages 554 to 578. Preferably the HIV is HIV-1 or HIV-2, most preferably HIV-1. Preferably the HIV is of clade A, B, C or D, preferably of clade B or D, preferably of clade B. An HIV sequence can be aligned to the HXB2 reference sequence (example 8), and therefore the g/p/n genes can be identified and corresponding epitopes delineated, no matter which clade is used. This can be done by eye, but is preferably done using the Los Alamos database's "HXB2 Numbering Engine" for this purpose (http://www.hiv.lanl.gov/content/hiv-db/NUM-HXB2/HXB2.MAIN.html).

A "mutation", as referred to herein, encompasses any addition, deletion or substitution of amino acids in a polypeptide or nucleic acid. Mutations, in general, alter the amino acid sequence of the polypeptide in question such that it differs from a or the naturally occurring polypeptide sequence.

In the context of the present invention, mutations are often introduced in order to abrogate or ameliorate a known biological activity of the viral protein. Thus, preferably when a sequence is said to be mutated with respect to the natural sequence, this means mutated to reduce or preferably remove one or more biological activities of the viral polypeptide. Examples of specific mutations which result in the reduction or removal of particular viral biological activities are presented herein. Further examples of motifs or domains associated with viral functions are known in the art. However, in accordance with the present invention, it is important that when the mutations are made to reduce/remove the biological functions, the naturally occurring epitopes are preserved as explained herein.

A "recombinant" polypeptide, as referred to herein, is a peptide whose sequence differs from a or the naturally occurring equivalent polypeptide and which may be produced by genetic recombination technologies, including DNA synthesis and manipulation. Recombinant peptides also includes peptides which are not produced by recombinant means, but which have designed using recombinant DNA technology or, preferably, have a sequence identical to a peptide designed by such technology.

An "immune response" as referred to herein, is either a cellular (to include, but not limited to, CD4+ and CD8+) or humoral response to an antigenic sequence or a combination of both.

The gene names used herein to define segments of nucleic acid and/or polypeptide sequence such as 'gag', 'pol', 'nef' and the like preferably have their ordinary meaning. Most often the terms are used to describe the polypeptide sequence, or the corresponding nucleotide sequence encoding said polypeptide sequence.

A "protective immune response" as referred to herein, is an antigen specific immune response that provides a prophylactic and/or therapeutic benefit.

### VIRAL ESCAPE

Mutations can occur in any epitope and lead to viral escape. By providing a greater number of epitopes it is more likely that some of the epitopes will remain unmutated. Furthermore, by advantageously providing extra T helper epitopes, the immune response can be broadened and/or strengthened according to the present invention.

Thus, in one embodiment, the present invention advantageously counters immunodominant effects which can affect conventional vaccines.

It is demonstrated that all of the identified nef human epitopes (both CD8+ T cell and T helper epitopes) in the HIV molecular immunology database are present in the GPN sequence of the present invention (SEQ ID NO: 1).

As noted above, WO02/32943 (Nabel and Huang) makes various disclosures in the field of HIV vaccines. As will be apparent from this specification, the present invention is distinct from disclosures made therein. In preferred embodiments, the present invention relates to materials comprising and/or encoding HIV derived proteins which expressly exclude any of those disclosed in WO02/32943, and preferably exclude any having 95% or greater identity to those disclosed in WO02/32943.

As noted above, WO02/022080 (Merck and Co.) makes various disclosures in the field of HIV vaccines. As will be apparent from this specification, the present invention is distinct from disclosures made therein. In preferred embodiments, the present invention relates to materials comprising and/or encoding HIV derived proteins which expressly exclude any of those disclosed in WO02/022080, and preferably exclude any having 95% or greater identity to those disclosed in WO02/02208.

As noted above, WO03/025003 makes various disclosures in the field of HIV. As will be apparent from this specification, the present invention is distinct from disclosures made therein. In preferred embodiments, the present invention relates to materials comprising and/or encoding HIV derived proteins which expressly exclude any of those disclosed in WO03/025003, and preferably exclude any having 95% or greater identity to those disclosed in WO03/025003.

### CD8+ T CELL EPITOPES

CD8+ T cell epitopes can be identified experimentally and can be predicted by analysis of the sequence of interest. Preferably these epitopes are predicted/recognised using the ProPred program (epitope prediction program, employing a matrix based prediction algorithm as disclosed in Stumiolo et al. Nat. Biotechnol. 17. 555-561(1999) and Singh and Raghava (2001) Bioinformatics,17(12), 1236-37, such as may be found at (http://www.imtech.res.in/raghava/propred/)).

It is an advantage of the present invention that naturally occurring epitopes are preserved in the polypeptide(s) of interest and in nucleic acids encoding them.

Addition or introduction of new CD8+ T cell epitopes may occur in the process of mutation and gene construction.

### T HELPER EPITOPES

T helper epitopes can be identified experimentally and can be predicted by analysis of the sequence of interest. Preferably these epitopes are predicted/recognised using the ProPred program (epitope prediction program, employing a matrix based prediction algorithm as disclosed in Sturniolo et al. Nat. Biotechnol. 17. 555-561(1999) and Singh and Raghava (2001) Bioinformatics,17(12), 1236-37, such as may be found at (http://www.imtech.res.in/raghava/propred/)). Preferably, naturally occurring T helper epitopes are preserved in the polypeptide(s) of interest and in nucleic acids encoding them.

Furthermore, there is advantageously provided novel T helper epitopes which have the beneficial effect of boosting and/or broadening the immune response to an antigen bearing said epitopes. Increased numbers of T helper epitopes are provided.

New T helper epitopes are created and/or introduced into the polypeptides of the present invention, thereby enhancing the immune response.

There is also described a recombinant strain FP9 fowlpox and/or a recombinant modified vaccinia virus Ankara, (MVA), each expressing a novel fusion protein containing antigenic peptide sequences found in the translation products of the gag, pol and nef genes of human immunodeficiency virus type I, (HIV-1), preferably clade B.

The use of such vectors in vaccination methods such as prime-boost (including single prime-multiple boost versions of prime-boost is also described). Indeed, as discussed below, the use of either or both of these two recombinant viruses to boost a DNA plasmid-mediated prime has been shown to induce a strong immune response in mammals such as rodents and finds application in primates such as humans. Therapeutic immunotherapy for people infected with HIV-1 is also described, for example using the antigenic gene(s) described herein in combination with HAART, and/or a prophylactic immunotherapy for people at risk of infection with HIV-1.

Advantageously, vectors according to the invention employ appropriate codon usage to optimise protein expression from mammalian cells. Advantageously, human codon usage is employed.

Therapeutic antigen(s) may comprise a fusion protein based on the products of the products of the HIV-1 (preferably clade-B) gag, pol and nef genes. Preferably said antigen(s) are delivered by one or both of the two recombinant pox viruses described herein.

A fusion protein containing antigenic peptide sequences derived from the translation products of the gag, pol and nef genes of human immunodeficiency virus type I, (HIV-1), preferably clade B is described.

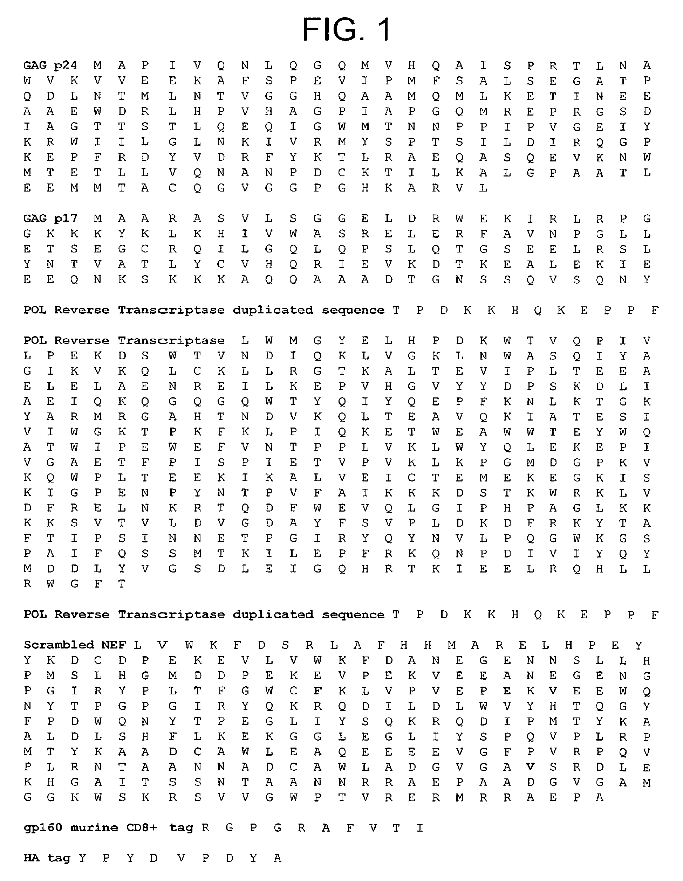

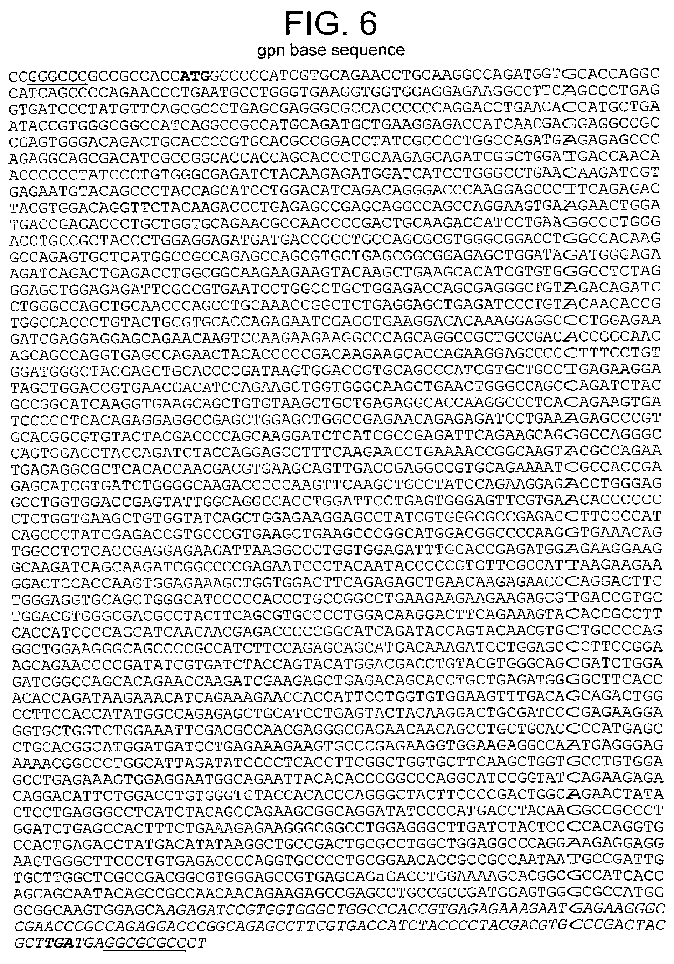

A preferred amino acid sequence of the fusion protein is shown in Fig 1. A preferred nucleotide sequence encoding this amino acid sequence is shown in Figure 6. Naturally a person skilled in the art will appreciate that due to degeneracy of the genetic code, numerous possible nucleotide sequences are possible and this is only one preferred example of same.

The gene products are preferably in the order GAG-POL-NEF in the fusion protein. This is also the way they are arranged in the HIV genome. Other orders may be equally effective. It is well within the abilities of a person skilled in the art to alter the order to meet the needs of a particular application, or even merely to facilitate an easier construction and/or handling of the reagent(s).

In a preferred embodiment, the constructs of the present invention possess advantageous effects regarding immunodominance. Immunodominance has hindered prior art vaccines, especially with respect to gag epitope(s). This is discussed above. Three epitopes in GAG in a preferred GPN construct according to the present invention are studied in the example section and each mounted an immune response. It is further disclosed in a preferred embodiment how the immune response across the entire fusion protein is monitored (see below for more details).

It will be apparent that some of the GPN protein could be deleted, for example to reduce the size of the construct. Indeed, certain individual gene(s) are presented herein for individual application. Preferably the gpn constructs disclosed are not truncated. Without wishing to be bound by theory, it is suggested that a single protein may be less immunogenic if used alone as a smaller entity rather than used as disclosed within a gene. Thus, in a preferred embodiment, gpn genes disclosed herein are used without truncation/deletion. Preferably the g/p/n genes used are each full length.

It will be apparent to the skilled reader that the immunogenic components of the present invention are generally polypeptides. However, the invention relates both to these polypeptides and to nucleic acids encoding them, whether these take the form of DNA, plasmids, viral vectors or other such entities. Indeed, the actual mode of production of the antigenic polypeptides may be varied by a person skilled in the art according to the particular application to which the invention is being put. The polypeptides may be made *in vitro* or preferably *in vivo*, whether intra- or extracellularly. Specific modes of use are described herein.

### GAG

The GAG protein has two functional domains, p17 matrix protein and p24 core capsid protein, both of which are essential and sufficient for assembly of HIV virus-like particles. In the gag gene(s) of the present invention, the p17 and p24 domains have advantageously been exchanged to disrupt processing of the GAG protein, in accordance with the International Aids Vaccination Initiative (WO01/47955). Other disruptional rearrangements are possible, such as scrambling as was applied to nef (see below), so long as the naturally occurring CD8+ T cell epitopes are preserved.

The myristoylation site of the p17 domain is advantageously mutated in order to prevent binding of the GAG protein to cellular membranes and, consequently, assembly and budding. This mutation is preferably by deletion or replacement of the second glycine residue in the domain with another amino acid, more preferably replacement with an inert non-polar amino acid, most preferably by replacement of the second glycine residue in the domain with an alanine residue. This prevents GAG from functioning in binding to cellular membranes, in virus assembly and in budding.

Advantageously the immunogenicity of gag is not adversely altered in the recombinant gene(s) of the present invention.

Preferably 'full length' gag according to the present invention means comprising the full sequence of the p17 and p24 domains. Preferably full length gag comprises only the full sequence of the p17 and p24 domains.

### POL

The pol gene encodes a number of proteins, including a protease, (p10), reverse transcriptase, (p51), RNase H, (p15) and an integrase, (p31).

The functional amino acid residues for reverse transcriptase activity centre on the YMDD motif (shown in epitope map as RT 183-186) which is an area with a number of recorded epitopes. Advantageously the reverse transcriptase is inactivated without loss of epitopes by domain swapping mutation as described in more detail below. Thus, in the present invention, preferably the reverse transcriptase activity present in the P51 subunit is disrupted. This is preferably done by rearrangement of the encoding sequence such that an altered P51 polypeptide is made with disrupted RT activity. Most preferably the sequence TPDKKHQKEPPF (which is present close to centre of the P51 polypeptide), is duplicated and the sequence encoding the amino- and carboxy-terminal parts of the P51 polypeptide exchanged about the point between the duplicated sequences.

This novel technique disrupts the active site in POL to ameliorate reverse transcriptase activity. Furthermore, this is advantageously accomplished according to the present invention while retaining the immunogenicity of the gene product. If the active site is mutated eg. by insertion, deletion or other known replacement type mutation, a T cell epitope would be destroyed. According to the present invention such epitope(s) are retained.

Preferably 'full length' pol according to the present invention means comprising all of the p51 domain of pol (ie. aa 1-440) which is often known as Reverse Transcriptase (RT). In Example 8, RT and RNase are combined in the section named RT CTL Map; appropriate care should be taken when reading this Example. Sometimes this may be referred to as '1-440 of RT'. Sometimes aa 1-440 of pol is referred to as 'truncated pol' (p51) to indicate that other parts of the full viral pol ORF are omitted such as the RNAse unit. However, references to 'pol', 'truncated pol' (p51) and 'full length pol' preferably mean pol p51 amino acids 1-440 as used herein and as will be apparent from the context.

### NEF

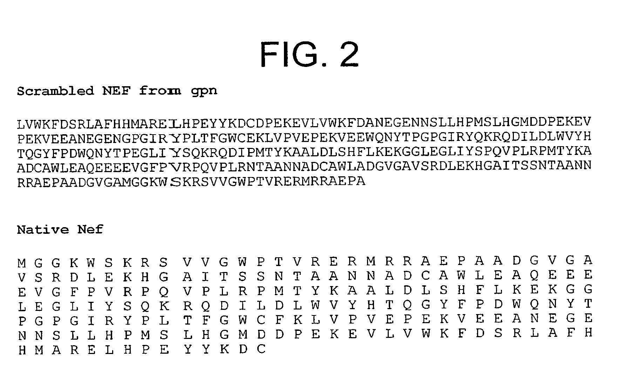

The nef gene encodes a multifunctional protein which enhances virus growth and mediates immune evasion. The NEF protein encoded by the gene in the current invention has been inactivated (ie. its function disrupted). In a preferred embodiment this inactivation is by dividing the coding region into eight subregions which were then reordered. Preferably these eight regions comprise 6 regions of 26 amino acid and two regions of 25 amino acids. A highly preferred scrambled NEF sequence is presented below (see Figure 2). Exact junctional boundaries in this sequence are easily identified by comparison to the native nef sequence (also presented below).

The objective of scrambling according to the present invention is to disrupt the functions of nef whilst preserving its T cell epitopes.

In scrambling nef according to the present invention, attention should be paid to disrupt the function of nef whilst creating a minimal number of surplus CD8+ T cell epitopes.

The preferred choice of 8 sub regions disclosed herein represents an advantageous balance between maintaining CD8+ T cell epitopes and not creating too many new junctional CD8+ T cell epitopes. In a preferred embodiment, creation of new junctional CD8+ T cell epitopes is minimised. In this context 'new' CD8+ T cell epitopes are those not occurring in the natural nef sequence. The average length of a CD8+ T cell epitope is 9 amino acids.

The preferred GPN fusion protein according to the present invention is mapped to show where epitopes have been maintained and where new junctional epitopes have been made. This is discussed further below.

When making a scrambled gene according to the present invention, it is advantageous to introduce junctional linkers in order to maintain any epitopes which span the chosen junctions. This may be easily accomplished by review of the sequence(s) using the ProPred tools and making correlating modifications as necessary.

In a preferred embodiment, twelve residue linkers, spanning these subregion junctions, are inserted between the re-ordered subregions to restore T cell epitopes located at the junctions of the eight subregions in the original NEF protein sequence.

Preferably 'full length' nef according to the present invention means comprising all of the amino acid sequence of the nef gene. Preferably this includes the N-terminal part (ie. nef aa 1-65) of the nef polypeptide which contains epitopes but has been deleted from prior art nef constructs.

### SEQUENCES

Specific sequences are presented in the sequence listing. In brief, SEQ ID NO:1 comprises a full exemplary gpn gene sequence, including tags. In a preferred embodiment, the tags are removed such as for human use, and an exemplary core GPN sequence is presented in SEQ ID NO:9.

SEQ ID NO:2 is the p24 gag fragment, SEQ ID NO:3 is the p17 gag fragment. The GAG p24 construct begins M A P I V even though the natural or reference p24 sequence does not contain M A. This is due to the preferred Kozak sequence used for translation (GCC GCC ACC ATG G ; SEQ ID NO:14). ATG is the initiation codon and translates to Methionine (M). The DNA codons for Proline (P) begin with a C so an additional amino acid having DNA codon(s) beginning with G was inserted (alanine).

In one embodiment, SEQ ID NO:2 and SEQ ID NO:3 are joined (last residue of SEQ ID NO:2 to first residue of SEQ ID NO:3) to make a preferred gag gene according to the present invention.

SEQ ID NO: 4 represents a short pol p51 sequence which is advantageously duplicated in a preferred pol construct according to the present invention as explained herein. SEQ ID NO:5 is the N and C terminal pol sequence. In one embodiment, a larger sequence is constructed in the order SEQ ID NO:4- SEQ ID NO:5- SEQ ID NO:4, resulting in duplication of SEQ ID NO:4, one repeat each side of SEQ ID NO:5 (ie. one at the N-terminus and one at the C-terminus of SEQ ID NO:5), thereby making a preferred po1 gene according to the present invention.

SEQ ID NO: 6 is a scrambled nef according to the present invention.

SEQ ID NO:7 is a tag recognised by murine CD8+ T cells, and SEQ ID NO:8 is an HA tag. Either or both of these tags may be advantageously incorporated into gene sequences of the present invention, for example to monitor expression and/or to monitor immune response(s) and/or to monitor for persistence/processing of the protein via the N- or C- termini (preferably via the C-terminus). However, for embodiments involving human subjects, preferably these tags are not incorporated into the gene sequence(s) of the present invention, or are removed from or not expressed on said gene sequence(s).

SEQ ID NO:10 is the native HIV nef sequence of the HIV clade B consensus sequence protein.

SEQ ID NO:11 is an exemplary nucleotide sequence coding for GPN. Naturally the person skilled in the art will appreciate that due to the degeneracy of the genetic code, many variant nucleotide sequences could equally code for the GPN of the present invention, especially those related to SEQ ID NO:11 and varying only by translationally silent differences in nucleotide sequence.

As used herein the term sequence 'derived from' an HIV gene product has its natural meaning in the art. Derived from simply indicates that it is based on the HIV sequence eg. as a starting point, whether this is *in silico* or actually experimental derivation eg. by cloning, PCR etc. For example the term would include predicted amino acid sequence from an HIV ORF, and is not limited to experimentally derived sequence. If a skilled person can recognise that the sequence originates from HIV, however much it has been rearranged or mutated, then it will be considered to be 'derived from' HIV. In a preferred embodiment, the sequence derived from another will possess a number of contiguous residues (amino acid or nucelotide) identical to the sequence from which is derived. Preferably there will be at least 5 such residues, preferably at least 8 such residues, preferably at least 10 such residues, preferably at least 14 such residues, preferably at least 18 such residues, preferably at least 20 such residues, preferably at least 22 such residues, preferably at least 25 such residues. For example, the gag, pol and nef sequences given herein are each derived from HIV.

The term 'mutated' has its usual meaning and includes deletion, insertion, point mutation, truncation, inversion as well as site directed mutation and scrambling as described herein.

It will be appreciated that the invention also embraces nucleic acid fragment(s) of the disclosed nucleotide sequences. Preferably nucleic acid fragments comprise at least 40 nucleotides, preferably at least 50 nucleotides, preferably at least 100 nucleotides, preferably at least 200 nucleotides, preferably at least 400 nucleotides, preferably at least 600 nucleotides, preferably at least 800 nucleotides, preferably at least 1000 nucleotides, preferably at least 1500 nucleotides, preferably at least 2000 nucleotides, preferably at least 2500 nucleotides, preferably at least 3000 nucleotides, preferably at least 3400 nucleotides, preferably at least 3410 nucleotides.

It will be appreciated that the invention also relates to nucleic acids which differ from those presented only by virtue of the degeneracy of the genetic code. The open reading frame(s) of importance when judging variation connected to degeneracy of the genetic code will be those which encode polypeptide(s) of the present invention, particularly encoding core GPN (SEQ ID NO:9 and nucleic acids encoding it).

Unnatural nucleotide residues or analogues or derivatised moieties may feature in nucleic acids according to the present invention.

Polypeptide fragment(s) of the disclosed amino acid sequences are also described. Preferably fragments are considered on a gene-by-gene basis. Preferably polypeptide fragments comprise at least 8 amino acids, preferably at least 9 amino acids, preferably at least 10 amino acids, preferably at least 12 amino acids, preferably at least 15 amino acids, preferably at least 20 amino acids, preferably at least 25 amino acids, preferably at least 26 amino acids, preferably at least 30 amino acids, preferably at least 40 amino acids, preferably at least 60 amino acids, preferably at least 80 amino acids, preferably at least 100 amino acids, preferably at least 150 amino acids, preferably at least 200 amino acids, preferably at least 300 amino acids, preferably at least 400 amino acids, preferably at least 600 amino acids, preferably at least 800 amino acids, preferably at least 1000 amino acids, preferably at least 1108 amino acids, preferably at least 1125 amino acids.

It will be appreciated that when considering fragments of the polypeptides and nucleic acids, the same importance is attached to the preservation of epitopes. That is to say, if only a part of a gene (such as amino acids 1-440 of reverse transcriptase (pol)) are present in a construct according to the present invention, then it is required that the corresponding naturally occurring epitopes for that part of the gene product are preserved. This applies equally to CD8+ T cell epitopes and to T helper epitopes as appropriate. In general, and as set out above, longer fragments of the individual genes are preferred and most preferred are fullest length polypeptides as described herein and as set forth in the sequence listing. Clearly the same principles extend to the consideration of sequence identity, that is to say that when assessing sequence identity with regard to a fragment according to the present invention, first the fragment length is chosen and then the sequence comparison is made across the corresponding fragment length section of the natural or reference sequence. The invention requires that all epitopes present on that fragment length are preserved. Preferably full length g/p/n genes are considered as described herein.

Gag/pol/nef polypeptides comprising strings of the naturally occurring epitopes, with only minimal connecting sequences are also described. Unnatural amino acids or analogues thereof or derivatised moieties may feature in the polypeptides of the present invention.

Relative sequence identity may be determined by computer programs which can calculate the percentage identity between two or more sequences using any suitable algorithm for determining identity, using for example default parameters. A typical example of such a computer program is CLUSTAL (see Thompson et al., 1994 (NAR 22:4673-80) or http://www.psc.edulgeneral/software/packages/clustal/clustal.html). Alternatively, the BLAST algorithm is employed, with parameters set to default values. The BLAST algorithm is described in detail at http://www.ncbi.nih.gov/BLAST/blast_help.html, which is incorporated herein by reference.

Other computer programs used to determine identity and/or similarity between sequences include but are not limited to the GCG program package (Devereux et al 1984 Nucleic Acids Research 12:387), FASTA (Atschul et al 1990 J Mol Biol 403-410) and the GENEWORKS suite of comparison tools.

FASTA uses the method of Pearson and Lipman (Proc. Natl. Acad. Sci. USA 85; 2444-2448 (1988)) to search for similarities between one sequence (the query) and any group of sequences. FASTA uses the following search parameters: these can be advantageously set to the defined default parameters: Matrix: as for BLAST (not used by FASTA for nucleotide comparisons). Wordsize - the number of continuous residues or bases which are considered at once in the initial comparison; default is 6 for nucleotide sequences, 2 for amino acid sequences. Gap penalty: This is the number of points deducted from a similarity score when a new gap is created; default is 16 for nucleotide sequences, 12 for amino acid sequences. Gap extension penalty: This is the number of points deducted from a similarity score when an existing gap is enlarged; default is 4 for nucleotide sequences, 2 for amino acid sequences. Expect: this restricts the number of sequences returned according to statistical significance; default is 2. List: this restricts the number of homologous sequences which are reported; default is 40. Align: this restricts the number of homologous sequences for which alignments are displayed; default is 10.

FASTA is available *via* Biology WorkBench at the University of Illinois (http://biology.ncsa.uiuc.edu/), or from the Genetics Computer Group (GCG).

BLAST (Basic Local Alignment Search Tool) is a heuristic search algorithm employed by the programs blastp, blastn, blastx, tblastn, and tblastx; these programs ascribe significance to their findings using the statistical methods of Karlin and Altschul (see http://www.ncbi.nih.gov/BLAST/blast_help.html) with a few enhancements. The BLAST programs were tailored for sequence similarity searching, for example to identify homologues to a query sequence. For a discussion of basic issues in similarity searching of sequence databases, see Altschul et al (1994:Nature Genetics 6:119-29).

BLAST uses the following search parameters: these can be advantageously set to the defined default parameters: HISTOGRAM - Displays a histogram of scores for each search; default is yes. DESCRIPTIONS - Restricts the number of descriptions of homologous sequences reported; default is 100. EXPECT - The statistical significance threshold for matches between sequences, according to the stochastic model of Karlin and Altschul (1990: PNAS 87:2264-8); default is 10. ALIGNMENTS - Restricts the number of sequences for which alignments are displayed; default is 50. MATRIX - Specifies a scoring matrix for BLASTP, BLASTX, TBLASTN and TBLASTX. The default matrix is BLOSLTM62 (Henikoff & Henikoff 1992:PNAS 89:10915-9). STRAND - Restrict a search to one or other strands of the sequence, (if a nucleotide sequence); default is both strands. FILTER - Masks off segments of the query sequence which have low complexity, as determined by the SEG program of Wootton & Federhen (1993: Computers in Chemistry 17:149-163), or segments consisting of short-periodicity internal repeats, as determined by the XNU program of Claverie & States (1993: Computers and Chemistry 17:191-201) or by the DUST program of Tatusov and Lipman (see http://www.ncbi.nlm.nih.gov); default filtering is DUST for BLASTN, SEG for other programs.

Most preferably, sequence comparisons are conducted using the FASTA alignment tool.

Although in general the sequence comparison techniques mentioned herein are well known in the art, reference may be made in particular to Sambrook et al., Molecular Cloning, A Laboratory Manual (1989) and Ausubel et al., Short Protocols in Molecular Biology (1999) 4th Ed, John Wiley & Sons, Inc.

Sequences possessing significant identity to the exemplary sequences disclosed herein are also described. Preferably a sequence has at least 40% identity to a sequence disclosed herein, preferably at least 45% identity, preferably at least 50% identity, preferably at least 55% identity, preferably at least 60% identity, preferably at least 65% identity, preferably at least 70% identity, preferably at least 75% identity, preferably at least 76% identity, preferably at least 78% identity, preferably at least 80% identity, preferably at least 85% identity, preferably at least 90% identity, preferably at least 91% identity, preferably at least 92% identity, preferably at least 93% identity, preferably at least 94% identity, preferably at least 95% identity, more preferably at least 96% identity, preferably at least 97% identity, preferably at least 98% identity, preferably at least 99% identity, or even more.

Clearly for amino acid sequences of polypeptides according to the present invention, the requirement for preservation of epitopes means that the sequence identity may be concentrated in areas of known epitopes where the sequence identity will preferably be 100% within the actual epitope sequence(s). Therefore, the regions of inter-epitope amino acid sequence may vary to a greater degree than the actual epitope regions, which preferably remain constant with respect to the reference sequence. Preferably the percentage identity figure is judged across the whole corresponding lengths of the relevant sequences being compared, including the epitope and inter-epitope regions. In another embodiment, the identity figures can relate to the variable regions only ie. the inter-epitope regions only, the epitopes being taken to be 100% conserved in this embodiment.

### OPTIMISATION OF GENE CONSTRUCTS

In a preferred embodiment, a 'Kozak' consensus sequence, GCC GCC ACC ATG G, is placed upstream of the gene construct(s).

In a preferred embodiment, the codon usage of the sequence may be modified to optimize the efficiency of translation of the gag-pol-nef transcript in human cells.

In a preferred embodiment, an Apa I and an Asc I restriction endonuclease recognition site are placed at the 5-prime and 3-prime ends of the gag-pol-nef (gpn) gene respectively. This advantageously facilitates subcloning of the gene cassette. In this embodiment, the restriction sites will be present in recombinant virus but are advantageously outside the open reading frame of the GAG-POL-NEF fusion protein. Consequently they will not have any influence on the amino acid sequence of the fusion protein nor the immune response generated to that protein. ApaI and AscI were also chosen to advantageously allow direct cloning into the pOPK6 recombinant plasmid. They have the further advantage of exerting no known influence on gene function.

In a preferred embodiment, a sequence encoding a reporter CD8+ T cell epitope, RGPGRAFVTI (SEQ ID NO:7), recognized by murine CD8-positive T cells specific for the gp160 protein, may be incorporated into a recombinant gene of the present invention. This has the advantage of allowing monitoring of the induction of CD8+ T cells following immunisation with GPN-containing vaccines. Advantageously the presence of the epitope is not known to affect the function of the fusion gene. In a highly preferred embodiment, this reporter epitope is absent from construct(s)/recombinant gene(s) for primate vaccination, such as human vaccination.

In a preferred embodiment, an additional antibody tag, YPYDVPDYA (SEQ ID NO:8), recognized by antibodies specific for this part of the influenza virus haemagglutinin protein, is added to the gene of the present invention (or to the nucleic acid encoding it). Preferably this is added to the carboxyterminus of the protein to allow the detection of expression in antibody-based immunoassays, such as 'western blot assays'. In a highly preferred embodiment, this tag is incorporated into the carboxyterminus of a recombinant gag-pol-nef gene, such as in a recombinant vector comprising a recombinant gag-pol-nef gene. In a highly preferred embodiment, this antibody tag is absent from construct(s)/recombinant gene(s) for primate vaccination, such as human vaccination.

### PHARMACEUTICAL COMPOSITION

There is also described a pharmaceutical composition comprising administering a therapeutically effective amount of an agent (such as a recombinant HIV gene such as the recombinant gpn gene as discussed herein) and a pharmaceutically acceptable carrier, diluent or excipients (including combinations thereof).

The pharmaceutical composition may comprise two components - wherein a first component comprises a nucleic acid vector and a second component which comprises a viral vector thereof. The first and second component may be delivered sequentially, simultaneously or together, and even by different administration routes.

The pharmaceutical compositions may be for human or animal usage in human and veterinary medicine and will typically comprise any one or more of a pharmaceutically acceptable diluent, carrier, or excipient. Acceptable carriers or diluents for therapeutic use are well known in the pharmaceutical art, and are described, for example, in Remington's Pharmaceutical Sciences, Mack Publishing Co. (A. R. Gennaro edit. 1985). The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s) or solubilising agent(s).

Preservatives, stabilizers, dyes and even flavouring agents may be provided in the pharmaceutical composition. Examples of preservatives include sodium benzoate, sorbic acid and esters of p-hydroxybenzoic acid. Antioxidants and suspending agents may be also used.

There may be different composition/formulation requirements dependent on the different delivery systems. By way of example, the pharmaceutical composition of the present invention may be formulated to be delivered using a mini-pump or by a mucosal route, for example, as a nasal spray or aerosol for inhalation or ingestible solution, or parenterally in which the composition is formulated by an injectable form, for delivery, by, for example, an intravenous, intramuscular or subcutaneous route. Alternatively, the formulation may be designed to be delivered by both routes.

Where the agent is to be delivered mucosally through the gastrointestinal mucosa, it should be able to remain stable during transit though the gastrointestinal tract; for example, it should be resistant to proteolytic degradation, stable at acid pH and resistant to the detergent effects of bile.

Where appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

### PHARMACEUTICAL COMBINATIONS

An agent may be administered with one or more other pharmaceutically active substances. By way of example, the simultaneous, or sequential treatments with an agent and one or more steroids, analgesics, antivirals or other pharmaceutically active substance(s) is described.

Application in a therapeutic immunotherapy for people infected with HIV such as HIV-1 is described. A combination with HAART, and/or combination with a prophylactic immunotherapy for people at risk of infection with HIV such as HIV-1 is also described.

It will be understood that these regimes include the administration of the substances sequentially, simultaneously or together.

The present invention will now be described by way of example, which is not intended to limit the scope of the appended claims, in which reference will be made to the following figures:

### Brief Description of the Figures

Figure 1 shows preferred gpn gene constructs. The sequence is continuous and has only been separated here for clarity. Bold indicates sequence variation between GPN sequence and the HIV Molecular Immunology sequence (NB. not all such sequence variations are shown).
Figure 2 shows scrambled and native nef gene sequences used for MHC Class II epitope comparisons.
Figure 3 shows T helper epitopes for scrambled nef (fig 3A) and native nef (fig 3B).

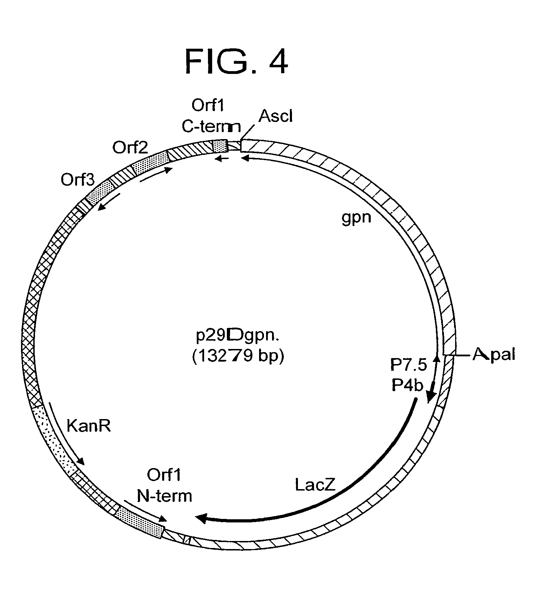

Figure 4 shows a map of recombination plasmid, p29D.gpn, for the construction of a recombinant fowlpox strain FP9 expressing gag-pol-nef.

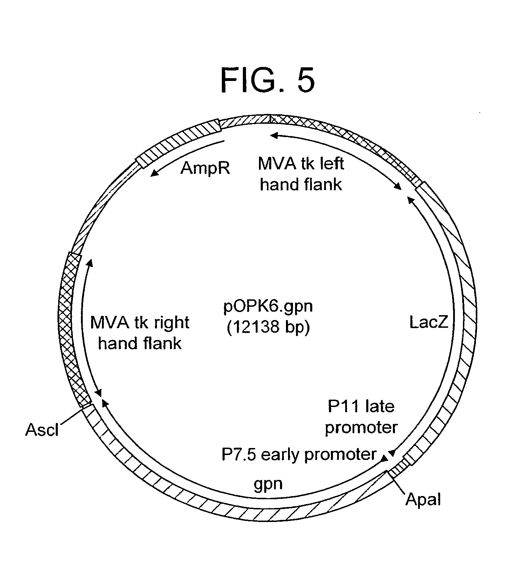

Figure 5 shows a map of recombination plasmid, pOPK6.gpn, for the construction of a recombinant MVA expressing gag-pol-nef.
Figure 6 shows the sequence of GPN. The GPN sequence is shown in normal text. The upstream region is shown in italics. 5-prime ApaI and 3-prime AscI sites are underlined. Initiating ATG and terminating TGA are shown in bold.
Figure 7 shows a bar chart of an IFN-γ **E**LISpot as described in Example 3.
Figure 8 shows a bar chart of an IFN-γ **E**LISpot as described in Example 5.
Figure 9 shows overlapping 20mer peptides used in an IFN-γ ELISpot assay. Amino acid length shown in brackets.
Figure 10 shows a bar chart of GAG-sp ecific responses in ex.4 group 4 animals.
Figure 11 shows a bar chart of POL-specific responses in ex.4 group 4 animals.
Figure 12 shows a bar chart of NEF-specific responses in ex.4 group 4 animals.
Figure 13 shows bar charts of IFN-γresponses.
Figure 14 shows a bar chart of IFN-γ responses elicited against selected peptides from gpn-sequence.

### EXAMPLES

### Example 1: Analysis of CD8+ T cell and T helper epitopes in the GPN sequence.

We used ProPred to compare T helper epitopes for native NEF versus scrambled NEF. This program predicts that there are more T helper epitopes in scrambled NEF according to the present invention. This demonstrates that the scrambled NEF sequence is at least as potent in eliciting T helper and CD8+ T cell responses as native NEF.

### CD8+ T cell Epitopes

CD8+ T cells can recognise and eliminate virally infected T cells and have been associated with control of viraemia in HIV infection in man and SIV infection in monkeys.

Comparison of the GPN sequence (Figure 1) with the HXB2 reference sequence published in the HIV molecular immunology database ("HIV Molecular Immunology 2002: Maps of CD8+ T cell Epitope Locations Plotted by Protein" Theoretical Biology and Biophysics, Los Alamos National Laboratory. August 7, 203; http://hiv-web.lanl.gov/content/immunology/maps/ctl/ctl.pdf) is performed.

This comparison reveals that all of the known murine and human CD8+ T cell epitope sequences identified for native gag (p17, p24), truncated-pol (p51) and nef are present in the GPN polypeptide sequence. There are single amino acid differences between the GPN sequence and the HXB2 reference sequence. Epitope sequences are indicated on the protein sequences of HXB2 and provide a relative location of the defined epitopes although they may vary relative to the protein sequence from which they were defined (refer to HIV Molecular Immunology Database 2002: Section II-A-2 - HIV Protein Epitope Maps, p56; http://hiv-web.lanl.gov/content/immunology/pdf/2002/ immuno2002.pdf).

Therefore, shuffling and rearrangements within the GPN sequence have not altered *per se* the capacity of this molecule to induce CD8+ T cell responses in mice, monkeys or man against the native gag (p17, p24), truncated-pol (p51) and nef sequences present in HIV.

Scrambling and rearrangement of the native polypeptides constituting the GPN protein may have created new CD8+ T cell epitopes unique to this molecule. Since these epitopes are unlikely to be present in native gag, pol or nef sequences within the HIV virus, they are therefore unlikely to be of biological relevance for a novel antigen for inclusion in a vaccine against HIV. Consequently, these epitopes have not been investigated further in this example.

### T helper epitopes

T helper immune responses enhance the CD8+ T cell effector response in terms of magnitude and breadth and therefore may enhance the efficacy of the CD8+ T cell response against HIV infection.

Comparison of the GPN sequence with the HXB2 reference sequence published in the HIV molecular database ("HIV Molecular Immunology 2002: Maps of T helper Epitope Locations Plotted by Protein" Theoretical Biology and Biophysics, Los Alamos National Laboratory. August 7, 203; http://hiv-web.lan.gov/content/immunology/maps/helper/helper.pdf) reveals that all of the human T helper epitope sequences identified for native gag (p17, p24), truncated-pol (p51) and nef are present in the GPN polypeptide sequence. Therefore, shuffling and rearrangements within the GPN sequence have not reduced the capacity *per se* of this molecule to induce T helper responses against the native gag (p17, p24), truncated-pol (p51) and nef sequences present in the clade B virus in humans.

Since T helper epitopes are usually longer than 15 amino acids, fusion of the gag, truncated-pol and nef sequences in GPN is likely to have advantageously created novel epitopes bridging the points at which these genes or parts of genes have been fused. Moreover, shuffling of the nef gene is likely to have created a number of novel epitopes bridging the shuffled sections of resulting polypeptide. Such novel T helper epitopes advantageously may elicit immune responses when GPN is administered as a vaccine, thereby enhancing the breadth and strength of the biologically-relevant CD8+ T cell responses elicited by the polypeptide(s) of the present invention relative to the native GAG, truncated-POL and NEF proteins.

To demonstrate that shuffling had created potential new T helper epitopes, the native NEF and scrambled NEF polypeptide sequences (Figure 2) are compared using the Propred MHC Class II epitope prediction program, employing a matrix based prediction algorithm as disclosed in Stumiolo et al. Nat. Biotechnol. 17. 555-561(1999) and Singh and Raghava (2001) Bioinformatics,17(12), 1236-37, such as may be found at (http://www.imtech.res.in/raghava/propred/).

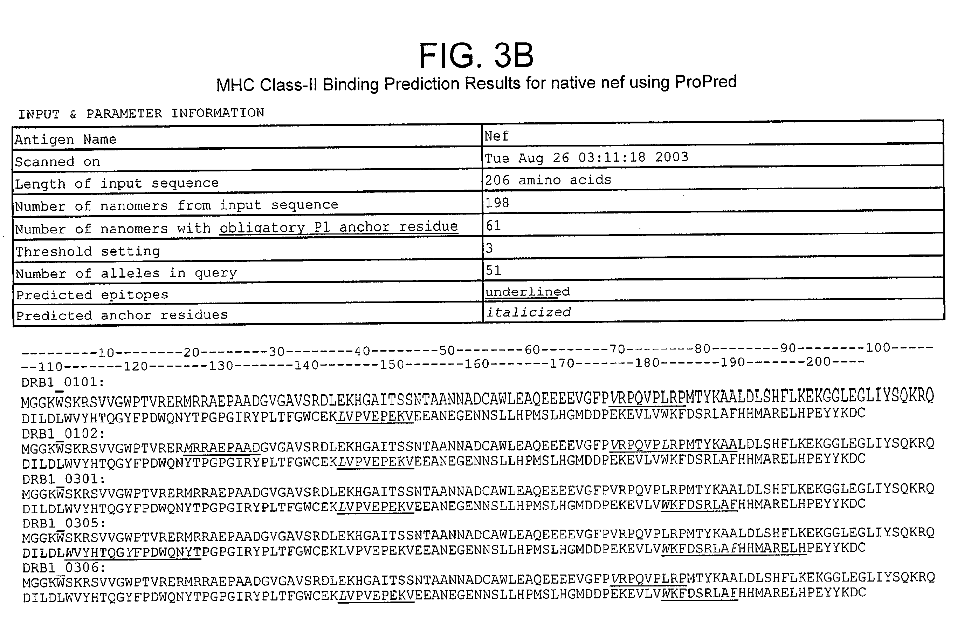

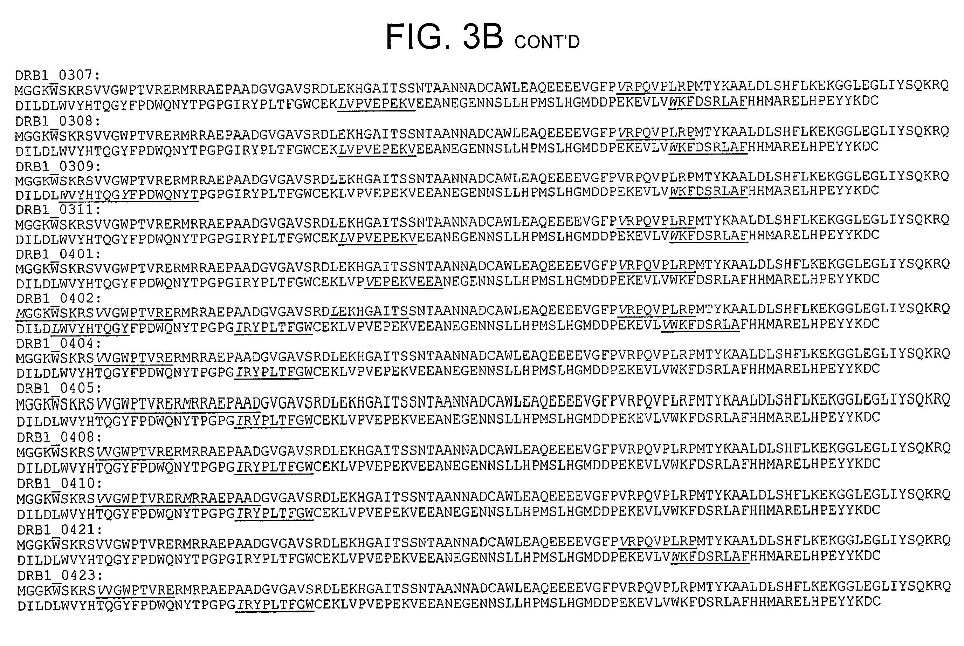

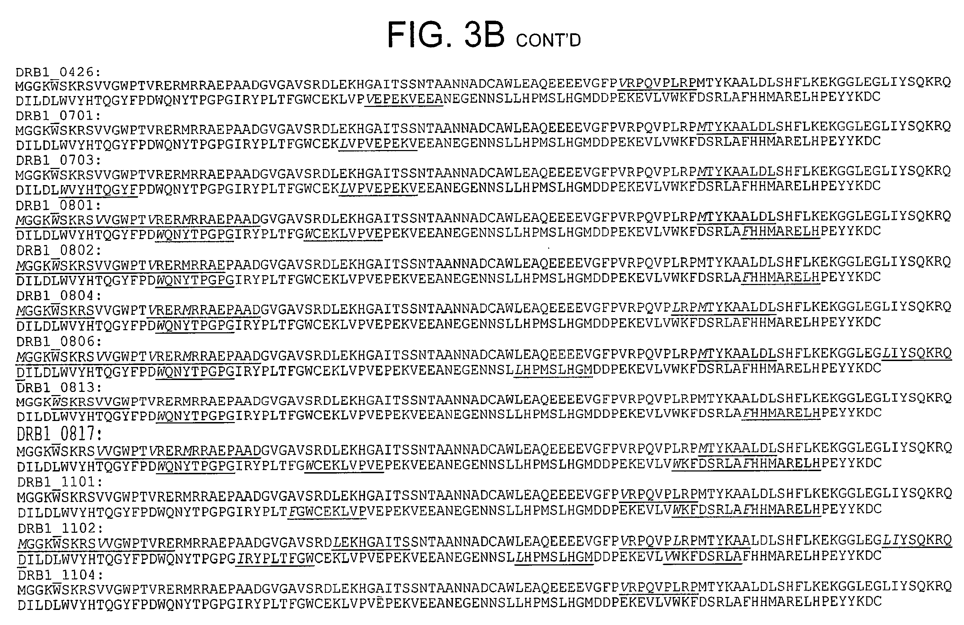

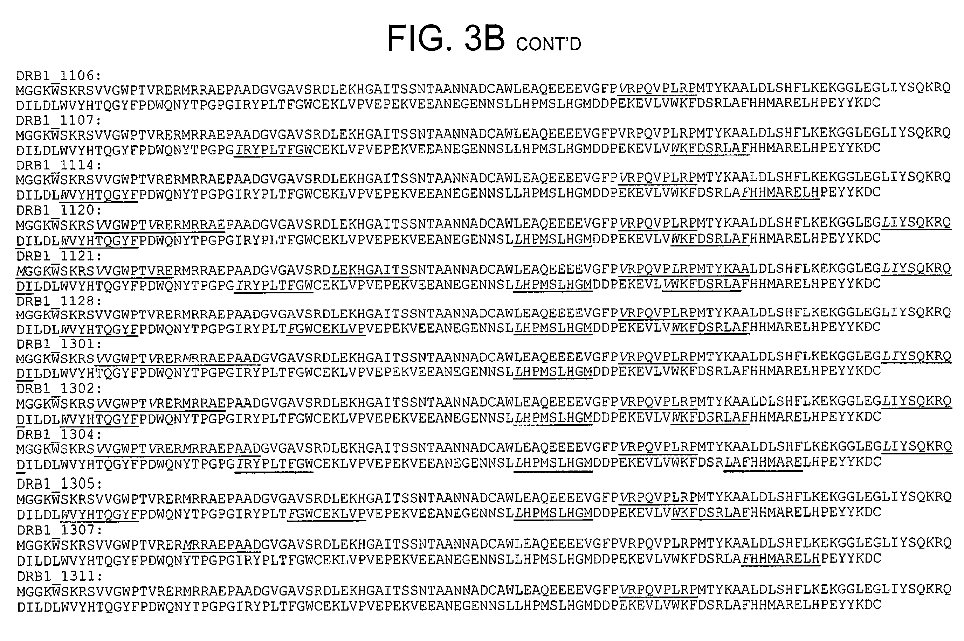

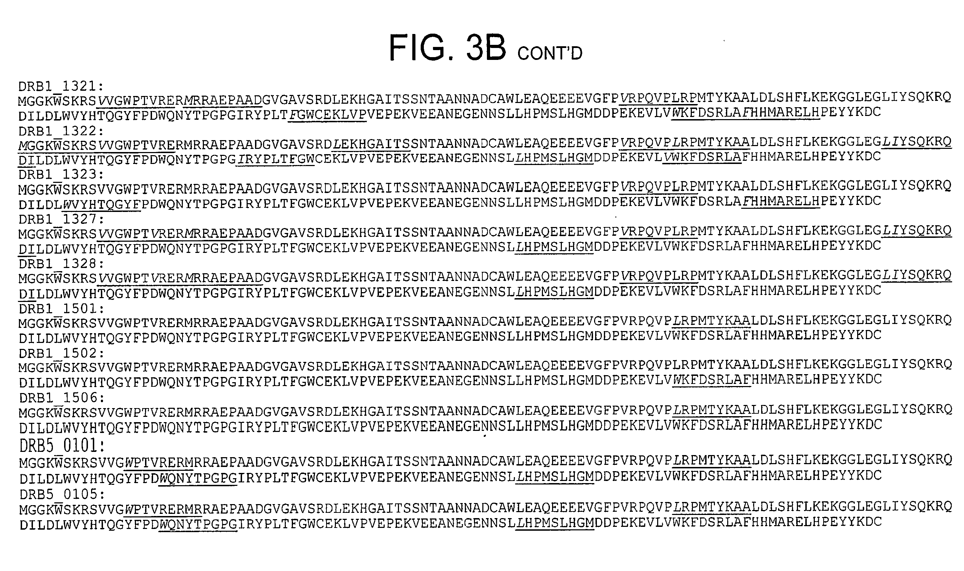

Comparison of these molecules reveals substantial changes in the predicted human MHC class **II** restricted T helper epitopes, with a greater number of predicted epitopes in the scrambled NEF sequence than the native NEF sequence (Figures 3A and 3B).

Thus, scrambling of the nef sequence has resulted in the creation of additional T helper epitopes for enhancement of the CD8+ T cell response against this polypeptide according to the present invention. These advantageous novel T helper epitopes comprise those epitopes shown in Figure 3A and which are absent from Figure 3B.

Similar novel T helper epitopes will have been generated across the fusion points between the proteins comprising the GPN polypeptide. Comparison as described above may be used to define/investigate these further.

### Example 2: Recombinant GPN gene in DNA, MVA and Fowlpox

A recombinant gag-pol-nef (gpn) gene is constructed for expression of a GPN fusion protein. This gene construct is used in nucleic acid carrier such as DNA carrier (eg. plasmid carrier), as well as in MVA and fowlpox vectors and materials for construction of same.

The use of recombinant virus vectors such as MVA and/or fowlpox vectors to boost a DNA plasmid-mediated prime has been shown to induce a strong immune response in rodents.

Essentially the same constructs also find application in primates such as humans.

In this example, the therapeutic antigen delivered by the recombinant pox viruses comprises a fusion protein based on the products of the products of the HIV-1 clade-B gag, pol and nef genes.

The recombinant gag-pol-nef gene was synthesized as a series of overlapping oligonucleotides, amplified by use of polymerase chain reaction cloned into the commercially available plasmid, pUC19, to make pUC19.gpn, (or 02-213), and the nucleotide sequence determined.

The gag-pol-nef gene is subcloned into three plasmid vectors to make
i) a plasmid expression plasmid, pSG2.gpn for use as a priming agent for *in vivo* use such as preclinical testing of gpn-containing recombinant poxvirus vectors;
ii) a recombination plasmid, pOPK6.gpn, for the construction of a recombinant MVA expressing gag-pol-nef;
iii) a recombination plasmid, p29D.gpn, for the construction of a recombinant fowlpox strain FP9 expressing gag-pol-nef.

The expression plasmid, pSG2.Me13, (Palmowski, MJ. et al 2002 J Immunol 168 p4391-4398) contains an expression cassette based on the human cytomegalovirus immediate-early promoter and intron combined with the bovine growth hormone gene polyadenylation signal to express a synthetic epitope string containing melanoma antigens. The expression plasmid pSG2.gpn is made by digesting pUC19.gpn with ApaI and AscI, blunt-ending the insert, and subcloning into blunted PstI-digested pSG2MeI3.

The MVA recombination plasmid, pOPK6 is made as follows. The plasmid is based on the commercially available cloning plasmid, pSP72, (Promega Inc.); the standard multiple cloning site being replaced with a synthetic linker containing unique restriction sites and the vaccinia virus P7.5 late-early promoter and late P11 promoter arranged in a head-to-head orientation. The linker also has the first twenty nucleotides of the *Escherichia coli* LacZ, (beta-galactosidase), gene as found in the vaccinia virus recombination plasmid pSC11, (Chakrabarti et al 1985 Mol Cell Biol 5 p3403-3409). The synthetic linker is synthesized as a series of overlapping oligonucleotides, amplified by PCR, and cloned into the commercial cloning plasmid, pcDNA3.1, to make pLink, (or 02-363). The sequence of the 300 base pair linker is given below:

agatctttaattaatgcctaggcaattgagacggcgcgcccgggcactagtaagggcccgtgcaataaattagaatatattttc
tacttttaccagaaattaattgtacaatttattatttatgggtgaaaaacttactataaaaagcgggtgggtttggaattagtggtac
catgcatcttagaatatatgtatgtaaaaatatagtagaatttcattttgtttttttctatgctataaatgaattcctcaagggatccgt
ctcctgcaggcatgctaagctagcggccggccctcgag

Part of the LacZ gene from pSC11 is subcloned into pLINK to make pLinkLacZ. The linker and LacZ gene were then subcloned into pSP72 as a XhoI-BgIII fragment such that a complete beta-galactosidase-encoding open reading frame is formed 3-prime to the P11 late promoter to make pSP72LinkLacZ.

MVA flanking regions representing 1500 base pairs 5-prime and 3-prime to the EcoRI site in the MVA thymidine kinase (tk) gene were isolated from MVA by PCR using the following primer-pairs:
Left-hand, (5-prime) tk flank
   Forward primer CAATTACAGATTTCTCCGTGATAGGT
   Reverse primer CGTGCATGCGGCCGCAACAATGTCTGGAAAGAACTG
Right-hand, (3-prime) tk flank
   Forward primer CAGGAATTCGCGGCCGCTGTGAGCGTATGGCAAACG
   Reverse primer TCATTTGCACTTTCTGGTTCGTA

The 1500bp PCR products are cloned into the commercial cloning vector pTOPO-TA, (Invitrogen) and sequenced.

The right-hand flank is subcloned into pSP72LiucLacZ as an EcoRI-MfeI fragment to make p72LinkLacZR. The left-hand flank insert is then subcloned into p72LinkLacZR as an NheI-SphI fragment to make pOPK6.

Subsequent sequence analysis of this plasmid identified an additional 174 base pairs of sequence in the pOPK6 backbone plasmid introduced from the pTOPO-TA vector with the left-hand flank due to non-specific cleavage by the NheI enzyme.

The gag-pol-nef open reading frame was subcloned from pUCl9.gpn into pOPK6 as an ApaI-AscI fragment to make pOPK6.gpn and the gpn insert sequenced to confirm no alterations had occurred during the subcloning steps.

This plasmid functions as a vehicle for introducing the gpn open reading frame, under control of the P7.5 late-early promoter, into the tk locus of MVA.

### FP9 vector

The FP9 strain fowlpox recombination plasmid, peeL29, was obtained from Dr Mike Skinner at the Institute for Animal Health, Compton, UK as published by Qingzhong, Y. et al 1994, Vaccine 12(6) p569-573.

The gpn open reading frame was subcloned from pUC19.gpn as a blunted KpnI-SacI fragment into the SmaI site of pEFL29 to make pEFL29.gpn. Sequencing of this plasmid showed that LacY and part of LacA had been introduced with the LacZ marker gene during the construction of pEFL29. Consequently, a linker was synthesized that allowed the deletion of all of the LacY and most of the remaining LacA open reading frames by means of a BsrGI-BIpI digest of pEFL29 to make p29Delta. The sequence of the linker is given below.

### TGAGCGCCGGTAGATACCATTATCAGCTGGTGTGGTGTCAGAAGTAATGTA C

The gpn expression cassette was then subcloned from pEFL29.gpn as an AatII-SphI fragment into AatII-SphI cut p29Delta to make p29D.gpn. The gpn insert was then sequenced to confirm no alterations had occurred during the subcloning steps.

The recombination plasmids, pOPK6.gpn and p29D.gpn are used to introduce the P7.5-gpn expression cassette into MVA and FP9, respectively.

The recombinant poxviruses were made by homologous recombination of the virus genome with the relevant recombination plasmid in chicken embryo fibroblast, (CEF), cultures using standard molecular biology techniques as described in Current Protocols in Molecular Biology, Ed. F.M. Ausubel, John Wiley & Sons; or according to suppliers' or manufacturers' instructions.

MVA.gpn is made by infecting confluent cultures of CEF cells with a non-recombinant MVA, (stock 575FHE-K), derived from MVA passage number 575, (Mayr et al., (1978) Zentralbl Bakteriol [b]. 167(5-6):375-90) at a multiplicity of infection of 0.1 for one hour. The infected cells are then transfected with between 0.5 and 2.0µg pOPK6.gpn plasmid DNA mixed with lipofectin (Invitrogen), according to the manufacturer's protocol. The cultures are incubated for three days before harvesting. The harvested cells are freeze-thawed three times and titrated as ten-fold dilutions and cultured under CMC-containing medium for three days. The medium is removed and replaced with CMC-medium containing X-Gal and the cultures incubated in this medium for 20 hours.

Recombinant viruses are identified by a blue plaque phenotype and picked with a pipette into cryotubes containing 100µl medium. The picked viruses are plaque purified five more times to ensure purity. Purity from non-recombinant parental virus was confirmed by the absence of white plaque phenotype and the absence of a WT PCR product when the virus DNA is screened using appropriate primers.

Purity PCR screening was performed on virus DNA purified from 2ml cultures infected with fifth or sixth round clones of MVA.gpn.

The virus DNA is purified using a Qiagen Blood and Cell Culture DNA min-kit (Qiagen GmbH, Max Volmar str. 4, 40724 Hilden, Germany) according to the manufacturer's protocol. A PCR reaction was performed using Klentaq proof reading polymerase (Sigma) using appropriate incubation conditions.

Parental virus was screened for using the following primers to give a band of 471 base pairs in the wild-type, but nothing in MVA:
TKU CAATTACAGATTTCTCCGTGATAGGT
TKL TCATTTGCACTTTCTGGTTCGTA

MVA.gpn was screened for using primer TKL and the following primer to give a band of approximately 2000 base pairs from the MVA.gpn construct:
HIV-U ATACCCCCGTGTTCGCCATTAAGA

Expression of the GPN protein from MVA.gpn is confirmed by immunoblotting as described below.

CEF cells are infected with MVA.gpn or MVA.LacZ control virus at a multiplicity of infection of 1. The culture is incubated for 3 days before harvesting. The cells are heated to 100 degrees C in SDS sample buffer and electrophoresed through a 4-20% acrylamide gel and electroblotted onto a nitrocellulose membrane. The transferred protein is then probed with either an antibody specific for the haemagglutinin tag (Abcam), or one specific for HIV. GAG P24, (Dako) and visualized using a suitable peroxidase-labelled secondary antibody and a chromogen. The blot assay indicates that the cells infected with MVA.gpn contain a protein of approximately 134kDa containing P24 and HA epitopes that was absent in the cells infected with MVA.LacZ. The predicted molecular weight of the GPN protein is 128.5 kDa, which is in close agreement with the specific band produced in the MVA.gpn-infected cells.

Expression of the GPN protein from FP9.gpn is confirmed by immunoblotting. CEF cells are infected with FP9.gpn or FP9.LacZ, ('FP9.29D'), control virus at a multiplicity of infection of 1. The culture is incubated for 5 days before harvesting. The cells are heated to 100 degrees C in SDS sample buffer and electrophoresed through a 4-20% acrylamide gel and electroblotted onto a nitrocellulose membrane. The transferred protein is then probed with either an antibody specific for the haemagglutinin tag (Abcam), or one specific for HIV GAG P24, (Dako) and visualized using a suitable alkaline phosphatase-labelled secondary antibody and a chromogen. The blot assay indicated that the cells infected with FP9.gpn contained a protein of approximately 134kDa containing P24 and HA epitopes that is absent in the cells infected with FP9.29D. The predicted molecular weight of the GPN protein is 128.5 kDa, which is in close agreement with the specific band produced in the FP9.gpn-infected cells.

The immunogenicity of the MVA.gpn and FP9.gpn viruses was tested in BALB/c mice using a 'prime-boost' protocol. The mice were divided into groups of four animals. The following test and control groups were established:
pSG2.gpn (50µ) prime followed 14 days later by a boost 1x10⁶ p.f.u. of MVA.gpn.
pSG2.gpn (50µ) prime followed 14 days later by a boost 1x10⁶ p.f.u. of FP9.gpn.
1x10⁶ p.f.u. of MVA.gpn alone.
1x10⁶ p.f.u. of FP9.gpn alone.

7 days after virus infection the peripheral blood lymphocytes were harvested and the number of GPN-specific interferon gamma-secreting lymphocytes determined in an ELISpot assay using the HIV following T cell epitopes (see also example 3):

| | |
|---|---|
| H2-D CD8 epitopes: | AMQMLKETI |
| | TTSTLQEQ |
| Gp160 H2-D CD8 epitope: | RGPGRAFVTI |
| H2-D CD4 epitope: | NPPIPVGEIYKRWIILGLNK |

Both MVA.gpn and FP9.gpn are shown to induce strong CD8+ T cell responses in the mice.

The prime-boost protocol is shown to give greater response than that seen in the groups receiving a single injection with virus.

### Example 3: Immunisation with FP9.gpn and MVA.gpn elicits antigen specific CD8+ T cell responses

In this example it is demonstrated that FP9.gpn and MVA.gpn elicit enhanced antigen-specific CD8+ T cell responses when administered alone or in a prime-boost immunisation regime with pSG2.gpn. In this example, the demonstration is presented in mice.

Female BALB/c mice (6 - 8 weeks old) are immunised with 50µg of pSG2.gpn by intramuscular (im.) injection and boosted with 1 X 10⁶ PFU FP9.gpn or 1 X 10⁵ MVA.gpn by intravenous injection (iv.) two weeks later. A further group of age matched naïve female BALB/c mice are immunised iv. with 1 X 10⁶ PFU FP9.gpn or 1 X 10⁵ MVA.gpn at the same time as the booster immunisation.

Fourteen days after the booster immunisation, all mice are sacrificed by cervical dislocation and the T cell response elicited against three H-2^{d} restricted CD8+ epitopes from the GPN polypeptide (Table A: AMQ, TTS, RGP) is determined by IFN-γ ELISpot assay as described below.

### Murine IFNγ ELISpot protocol:

### Materials:

### IFN-γ ELISpot ALP Kit Mabtech 3321-2A

600µg anti-IFN-γ purified Mab AN18
50µg anti-IFN-γ biotinylated Mab R46A2
50µl Streptavidin-Alkaline Phosphatase

### Complete α-mem medium

500ml MEM α-modification Sigma M-4526
50ml FCS [10%] Sigma F-2442
Sml pen/strep [100U penicillin 100µg strep] Sigma P-0781
10ml L-glutamine [4mM] Sigma G-7513
500µl 12-Mercaptoethanol [50µm] Gibco BRL 31350-010

### ACK buffer

8.29g NH₄Cl [0.15M] (Sigma A-4514)
1g KHCO₃[1mM] (Sigma P-9144)
37.2mg Na₂EDTA (Sigma ED2SS)
800ml milli-Q water
Adjust pH to 7.2-7.4 with HCl (Sigma S-7653)
Make up to 1000 ml with water and autoclave

### Colour Development Buffer:

### BioRad AP Conjugate Substrate kit (170-6432 ).

For one plate:
5ml deionised water
200µl of 25x buffer
50µl reagent A
50µl reagent B
Mix well and use immediately

### Protocol

### 1. Preparation of Plates:

1.1. **Coating plates:** coat MAIP multiscreen plates (Millipore Maps4510) with rat anti-mouse IFNγ (Mab AN18) antibody. Dilute to 10µg/ml in Phosphate Buffered Saline (PBS; Sigma P-3813) and add 50µl per well to MAIP plates. Incubate overnight at 4°C in a humidified chamber
1.2.**Blocking plates:** Flick off coating antibody and wash plates once with 150 ul of sterile PBS (Sigma P-3813) per well using a multi-channel pipette. Flick off the PBS, add 100u1 complete α-MEM medium per well, and incubate at room temperature for 1+ hour. It is important to keep the plates sterile at this stage.

### 2. Splenocyte preparation:

2.1.Crush individual spleens in 2 ml of PBS with the plunger of a 10 ml syringe in a 70 µm cell strainer (Falcon 352350) contained in a petri dish, add 5 ml of PBS, suspend splenocytes by pipetting, and transfer into a 50 ml tub e. Rinse cell strainer and dish with a further 10 ml of PBS and add to the 50 ml tube. Centrifuge at 1500 rpm for 5 min.
2.2.Remove supernatant, re-suspend cells by tapping tube and add 5ml ACK buffer and mix by inversion. Incubate at room temperature for no longer than 5 minutes. Add 25 ml PBS, mix by inversion and centrifuge at 400 X g for 5 min.
2.3.Remove supernatant re-suspend pellet by tapping the tube, add 10ml PBS and vortex. Count using an improved Neubauer haemacytometer by diluting 1:10 in 0.4% trypan blue solution (Sigma T-8154). Aliquot amount needed for the Elispot and centrifuge at 1500 rpm for 5 min, resuspend by vortexing in an appropriate volume of complete Alpha MEM medium to give a concentration of 10 million cells/ml.

### 3. Plate setup:

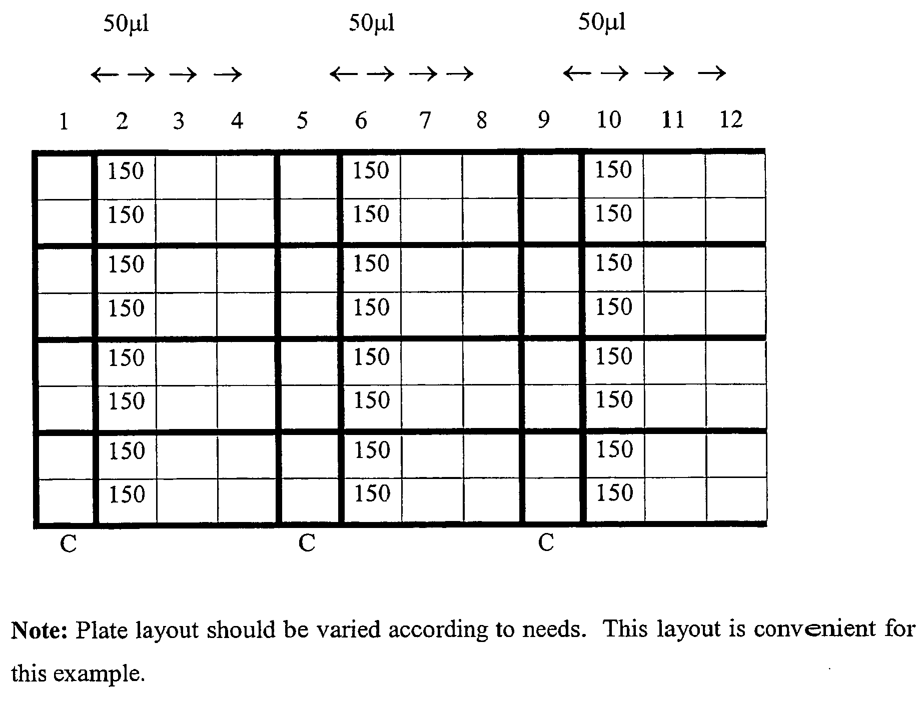

3.1.Flick blocking media from plate and add 50µl of complete alpha MEM medium to columns 3, 4, 7, 8, 11&12.
3.2.Add 150µl of splenocytes to columns 2, 6 and 10 in duplicate. (Up to 12 samples per plate)
3.3.Take 50µl of splenocytes from columns 2, 6 and 10 and transfer to columns 1, 5 and 9 respectively: these are the negative control wells.
3.4.Serially dilute each sample by taking 50µl from columns 2, 6 and 10, to columns 3, 7 and 11, mix well and transfer 50µl to 4, 9 and 12. Discard 50µl after mixing final columns in dilution.
3.5.Add test peptide and control peptide to twice the desired final concentration to naïve splenocytes at 10 million/ml in complete α-MEM medium. Add 50µl of control peptide and targeT cells to columns 1, 5 and 9.Add 50µl test peptide and targeT cells to remaining columns.
3.6.Incubate plates at 37°C for 18-20 hours.

### 4. Developing the Assay

4.1.Wash plates twice with PBS containing 0.05% Tween 20 (Sigma P1379), once with distilled water and twice with PBST.
4.2.Add 50µl/well of biotinylated rat anti-mouse interferon-gamma diluted to 1µg/ml in PBS. Incubate for 2 hours at room temperature.
4.3.Wash plates four times with PBST, then add 50 µl Streptavidin Alkaline Phosphatase (Mabtech) diluted to 1µg/ml in PBS. Incubate at room temperature for 1 hour.
4.4.Wash plates four times with PBST, add 50µl/well of colour development buffer Incubate at room temperature until spots develop (approx. 10 min). Wash plates well with tap water, peel off plastic bottom and leave to dry overnight on paper towels.

### Calculation

Results are calculated as the number of epitope-specific IFNγ spot forming cells/million splenocytes (sfc/million). Differences between groups are determined by one-way ANOVA and a post hoc Tukey-Karamer multiple comparison test on log₁₀ transformed data using GraphPad Instat version 3.05.

Results

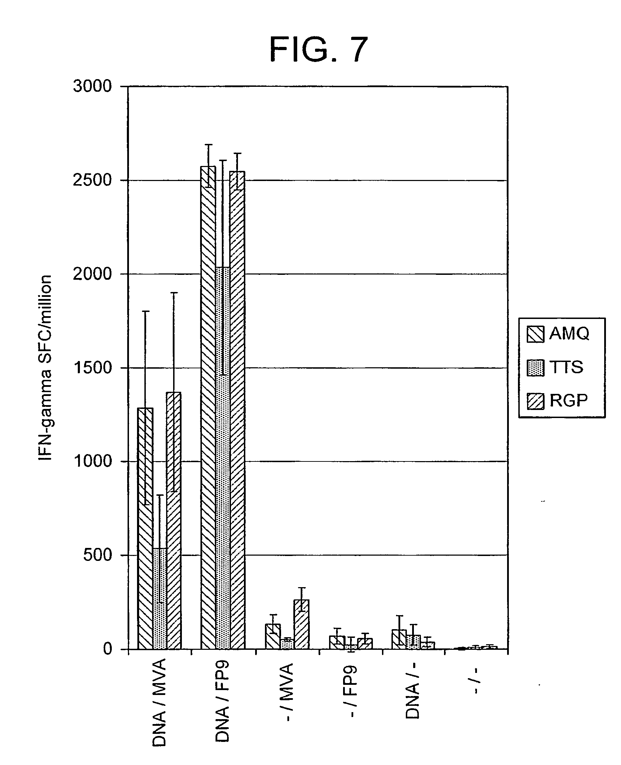

The demonstration that immunisation with FP9.gpn and MVA.gpn alone or in prime-boost regimes with pSG2.gpn elicits antigen specific CD8+ T cell responses in mice is illustrated in figure 7. Female BALB/c mice were immunised im. with pSG2.gpn (DNA) or sham immunised with PBS ( - ) and boosted 14 days later with FP9.gpn (FP9) or MVA.gpn (MVA) as described above. The CD8+ T cell response was determined in splenocytes 14 days after the booster immunisation using the IFN-γ ELISpot assay. Columns represent the mean IFN-γ spot forming cells/million splenocytes ± standard deviation for 4 mice per group elicited by CD8+ reactive epitopes AMQ, TTS and RGP (see Table A).

**Table A: GPN epitopes used in this study**

| (¹ND = not determined) | | | | |
|---|---|---|---|---|
| **Antigen** | **Abbr.** | **Epitope** | **CD4/CD8 reactive** | **MHC restriction** |
| HIV-1 env | RGP | RGPGRAFVTI | CD8 | D^{d} |
| HIV-1 gag | AMQ | AMQMLKETI | CD8 | K^{d} |
| HIV-1 gag | TTS | TTSTLQEQ | CD8 | H-2^{d} |
| HIV-1 gag | NPP | NPPIPVGEIYKRWIILG LNK | CD4 | H-2^{d} |

Single or prime-boost immunisation with either FP9.gpn or MVA.gpn elicited a significantly (*P*<*0.01*) enhanced antigen-specific T cell response against each of the CD8+ T cell epitopes when compared to sham- immunised controls (Figure 7).

In addition, priming with pSG2.gpn and boosting with FP9.gpn or MVA.gpn elicited significantly (*P*<*0.01*) enhanced responses against each CD8+ T cell epitope when compared to single immunisations with each virus (Figure 7).

It should be noted that the virus titres were not identical in this experiment. Thus, the relative immune potency of the viruses has not been compared. Naturally it is straightforward to assess this by performing the example with identical viral titres.

Thus it is demonstrated that specific CD8+ T cell responses are elicited against the recombinant HIV genes according to the present invention such as the gpn gene by immunisation with either FP9.gpn or MVA.gpn, indicating that these constructs are both potent in eliciting an immune response against the GPN polypeptide.

Moreover, potent immune responses are directed at epitopes lying in the N- and C-terminal regions of the GPN polypeptide, indicating that the whole polypeptide is expressed, processed and presented after delivery with either FP9.gpn or MVA.gpn.

Both viruses elicit significantly higher immune responses against each epitope when administered to animals that have been primed with pSG2.gpn, indicating that both can act as boosting agents in prime boost immunisation regimes.

### Example 4: Immunogenicity of FP9.gpn and MVA.gpn in primates

The immunogenicity of the gpn immunogens is demonstrated *in vivo* in primates as follows.

The immunogenicity of the GPN polypeptide consisting of the gag, pol and nef proteins of clade B HIV-1 is tested in non-human primates (Macaca mulatta).

The polyprotein (human codon usage) is expressed in recombinant MVA, fowlpoxvirus FP9, adeno virus and a DNA vaccine vector. The polyprotein expressing constructs are administered in a heterologous immunisation regimen in order to induce high levels of antigen-specific CD8+ and CD4+ T cells.

Table B shows the study design of the **immunogenicity studies.**

**Table B**

| Group No n=5 | **Prime 1** | **Prime 2** | **Boost 1** | **Boost 2** |
|---|---|---|---|---|
| | **Day 0** | **Day 28** | **Day 56** | **Day 84** |
| **1** | FP9.gpn | FP9.gpn | MVA.gpn | MVA.gpn |
| **2** | MVA.gpn | MVA.gpn | FP9.gpn | FP9.gpn |
| **3** | - | FP9.gpn | MVA.gpn | - |
| **4** | pDNA+IL2-Ig/fc | MVA.gpn | FP9.gpn | Adeno.gpn |

The design of the macaque study of this example is shown in Table B.

Blood samples are taken pre-immunisation and 7 days after each immunisation and four weeks after the last immunisation. PBMCs were cryopreserved and tested in ELISpot and intracellular cytokine assays using pools of overlapping peptides.

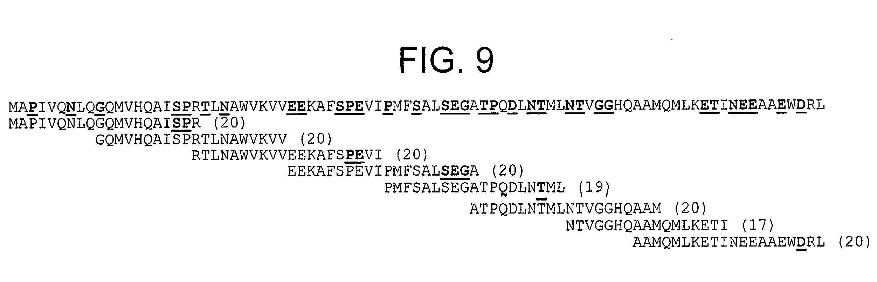

Cellular immune responses are tested during and at the end of the study. The frequency of IFN-γ-secreting T cells is tested by IFN-γ ELISpot assays (see Figure 9 for overlapping peptides used).

In an additional group, for comparitive purposes, DNA vaccines adjuvanted with IL-2/Ig fusion proteins (Barouch, D. H., A. Craiu, et al. (2000) Proc Natl Acad Sci U S A 97(8): 4192-7) and recombinant adenovirus are tested. For study design see Table B.

There are several parameters that are presented in this study:
(a) Order of FP9 and MVA. The most effective combinations of poxvirus vector primes and boosts are demonstrated. This particularly focuses on groups 1 and 2 of the study design (see Table B).
(b) Frequency of primes and boosts. The effectiveness (in terms of immunogenicity) of priming with a single poxvirus immunisation and boosting with a single heterologous poxvirus vector boost is demonstrated. This particularly focuses on animals in group 3 which will be immunised as outlined.

Read-out: The key objective of this vaccination strategy is to induce cellular immune responses. Therefore the following assays are used to monitor cellular immune responses: IFN-γ ELISpot using overlapping peptides (20mers overlapping by 10 amino acids as shown in Fig 9), intracellular cytokine staining for CD8+ and CD4+ T cells. In ELISpot assays CD4/8 depletion experiments will confirm IFN-γ secretion in response to peptides by CD4+ and/or CD8+ T cells.

Data for group 4 is presented below. The data shows immunogenicity of HIV. immunotherapeutics in rhesus macaques using novel prime-boost immunisation regimens.

**Table 1 Immunisation and sampling schedule**

| **Group** | **d -28** | **d 0** | **d 2** | **d 7** | **d 28** | **d 35** | **d 56** | **d 63** |
|---|---|---|---|---|---|---|---|---|
| **4** | Bleed | Bleed; i.m. inj. of 5 mg pfu pSG2. gpn | i.m. inj. of 5 mg pfu pS-IL-2fc | Bleed | Bleed; i.d. inj. of 5x10⁸ pfu of FP9. gpn | Bleed | Bleed; i.d. inj. of 5x10⁸ pfu of MVA. gpn | Bleed |

### Results

The GPN insert is immunogenic and induces interferon-gamma secreting T cells following viral vector immunisation in macaque monkeys.

In group 4 gpn-specific T cell responses are detected in 4/5 animals seven days after the FP9.boost. The responses are maintained following MVA boost.

This data shows that the gpn fusion protein is immunogenic in primates when it is delivered using recombinant vectors.

GAG, POL and NEF of gpn are recognised in immunised macaque monkeys.

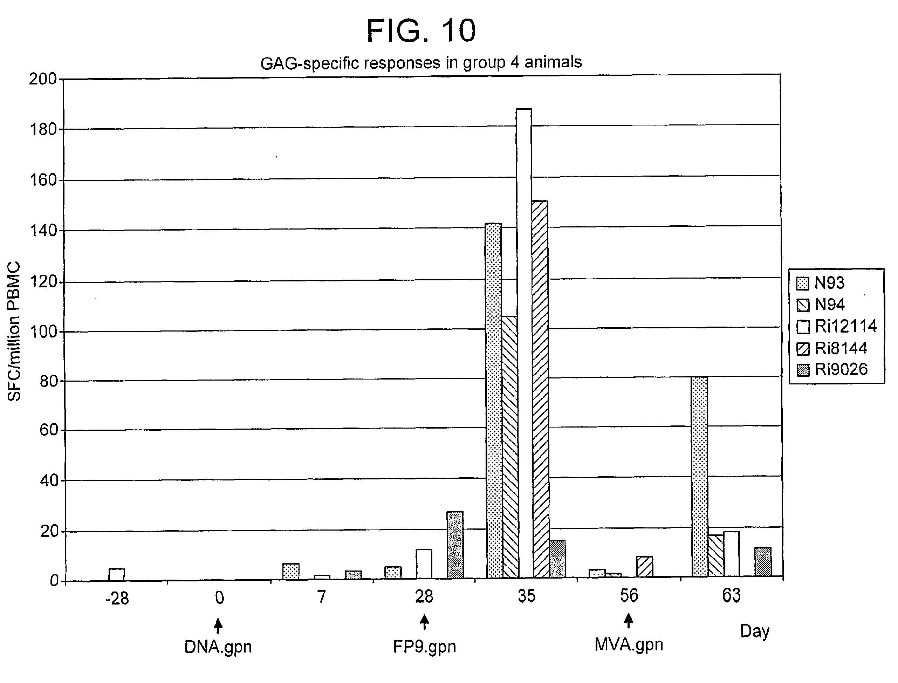

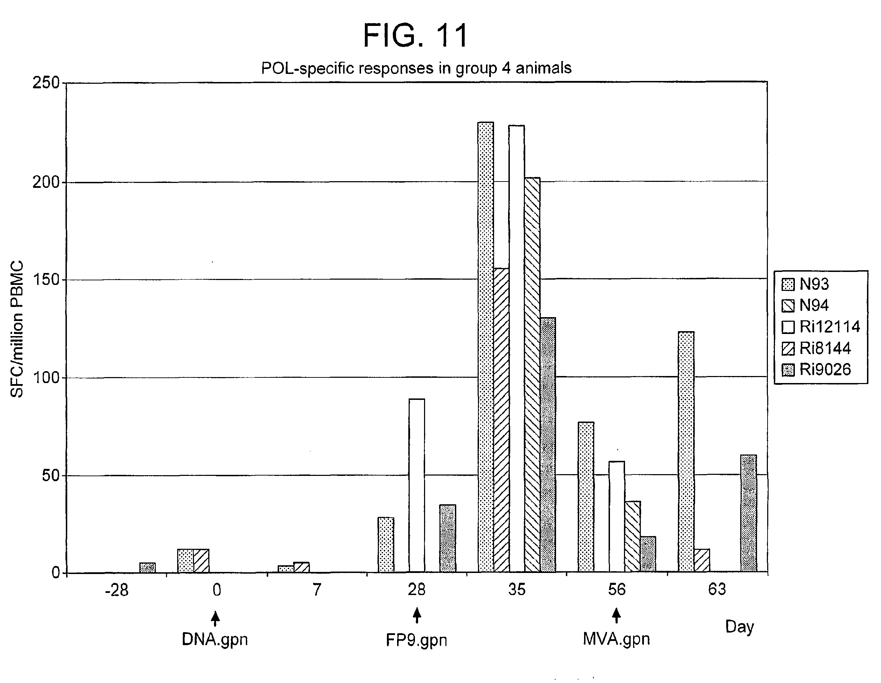

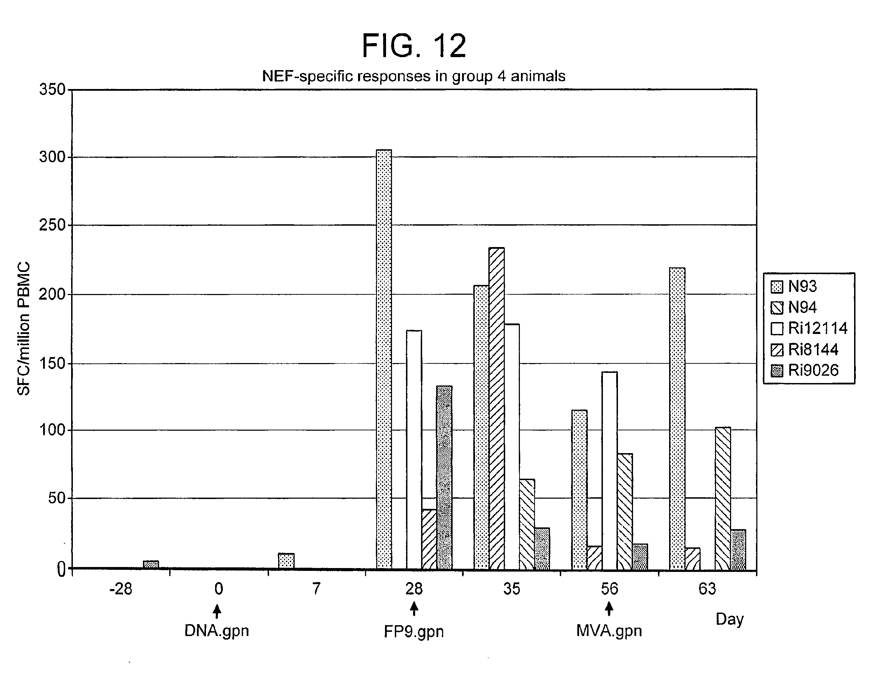

Figures 10, 11 and 12 show responses of individual animals to different parts of the gpn protein indicating that T cell epitopes from the proteins gag, pol and nef are recognised in the same responding animal. For example animal N93 in Group 4 shows T cell responses recognising the GAG, POL and NEF portion of the GPN protein indicating that cell responses can be induced using GPN. Furthermore, without wishing to be bound by theory, the breadth of the immune response to the diverse elements of the triple GPN polypeptide indicates an advantageous feature of the invention in avoiding immunodominance effects where a single epitope can dominate the immune response to a polypeptide at the cost of responses to other epitopes present on the polypeptide. By contrast, the polypeptide according to the present invention of this example advantageously demonstrates a broad and balanced response.

### Primate Immunogenicity Single Administration Experiments

The immunogenicity of the GPN polypeptide (SEQ ID No. 1) was tested in 10 rhesus macaques (Macaca mulatta), labelled A-J, in single administration ("single shot") experiments. In this example the polypeptide was delivered via either the MVA or FP9 vector.

ELISpot assays were carried out as described above. Responses to 119 overlapping ∼20mer peptides (as described in Example 7) were measured and then summed to give a total response.

Responses were measured prior to injection and 28 days post injection. Responses are shown below. GPN elicited an immune response in all animals studied.

| | | | |
|---|---|---|---|
| | | **Day 0 Bleed then i.d. 5x10⁸ pfu MVA.gpn** | **Day 28 Bleed** |
| | | Summed ELISpot response SFC/million PBMC | |
| **Animal** | A | 13.35 | 28.33 |
| | B | 6.70 | 203.33 |
| | C | 23.40 | 133.33 |
| | D | 6.65 | 20.00 |
| | E | 0.00 | 320.00 |
| | | | |
| | | **Day 0 Bleed then i.d. 5x10⁸ pfu FP9.gpn** | **Day 28 Bleed** |
| | | Summed ELISpot response SFC/million PBMC | |
| **Animal** | F | 0 | 9.90 |
| | G | 28.35 | 48.33 |
| | H | 3.35 | 31.67 |
| | I | 0 | 98.33 |
| | J | 0 | 345.00 |

The results indicate that the GPN polypeptide is immunogenic and induces interferon-gamma secreting T cells following viral vector immunisation in primates such as macaque monkeys.

Thus the effectiveness of the recombinant genes of the present invention is demonstrated in vivo in primates.

### Example 5: Heterologous prime-boost immunisation regimens with FP9.gpn and MVA.gpn elicit enhanced T cell responses.

In this example it is demonstrated that FP9.gpn and MVA.gpn elicit enhanced antigen-specific CD4+ and CD8+ T cell responses when administered to subjects in heterologous or homologous prime-boost immunisation regimens. In this example, the effect is demonstrated in mice.

Female BALB/c mice (6 - 8 weeks old) are immunised with either 1 X 10⁵ PFU FP9.gpn or the 1 X 10⁶ of MVA.gpn by iv. injection. Animals were boosted two weeks later with either virus in an identical manner to the initial immunisation.

Fourteen days after the booster immunisation, all mice were sacrificed by cervical dislocation and the T cell responses elicited against three CD8+ epitopes (Table A: AMQ, TTS, RGP) and a CD4+ epitope (Table A: NPP) from GPN were determined by IFN-γ ELISpot assay as described in the above examples.

Results were calculated as the sum of the number of epitope-specific IFNγ spot forming cells/million splenocytes (sfc/million). Differences between groups were determined by one-way ANOVA and a post hoc Tukey-Karamer multiple comparison test on log₁₀ transformed data using GraphPad Instat version 3.05.

### Results

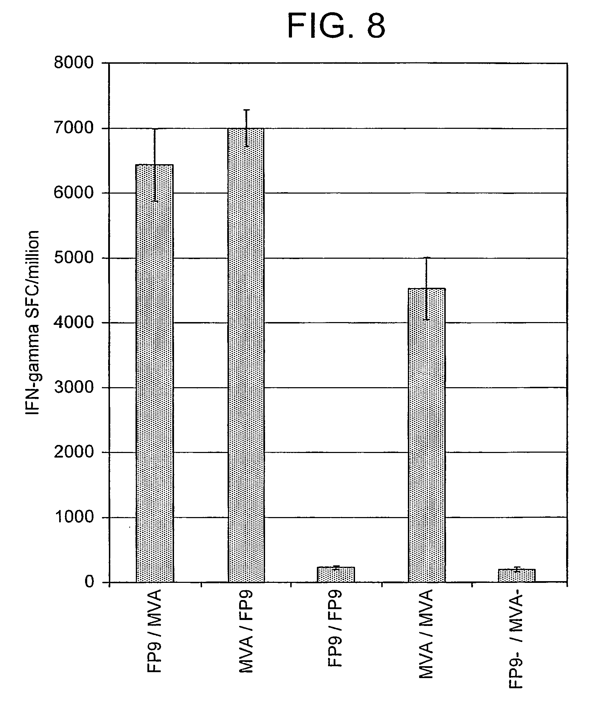

The demonstration that immunisation with FP9.gpn and MVA.gpn in prime-boost regimes elicits enhanced T cell responses against GPN in mice is illustrated in figure 8. Female BALB/c mice were immunised iv. with FP9.gpn (FP9) or MVA.gpn (MVA)sham immunised with PBS ( - ) and boosted 14 days later with either virus as described above. The CD8+ T cell response was determined in splenocytes 14 days after the booster immunisation using the IFN-γ ELISpot assay- Columns represent the sum of the mean IFN-γ spot forming cells/million splenocytes ± standard deviation for 4 mice per group elicited by AMQ, TTS, RGP and NPP (see Table A).

Heterologous prime-boost immunisation with FP9.gpn and MVA.gpn in either order elicited a significantly (*P*<*0.001*) enhanced overall T cell response against epitopes from GPN when compared to subjects given homologous immunisations with either virus (Figure 8).

Heterologous immunisation with FP9.gpn/MVA.gpn or MVA.gpn/FP9.gpn elicits significantly higher T cell responses against GPN than homologous immunisation with FP9.gpn/FP9.gpn or MVA.gpn/MVA.gpn.

Moreover, these viruses can advantageously be used interchangeably as priming and boosting agents with each other.

Thus the efficacy of recombinant HIV genes such as the gpn gene is demonstrated, in particular when delivered by viral vector such as fowlpox or MVA based vectors.

### Example 6: Heterologous prime-boost immunisation with FP9.gpn and MVA.gpn elicits enhanced CD8+ and CD4+ T cell responses against the individual component proteins of gpn

It is demonstrated that heterologous prime-boost immunisation with FP9.gpn and MVA.gpn or FP9.gpn and MVA.gpn elicits enhanced antigen-specific CD4+ and CD8+ T cell responses against the component parts of the GPN polypeptide. In this example, the demonstration is presented in mice.

Female BALB/c and C57/BL6 mice (6 - 8 weeks old) are immunised with either 1 X 10⁶ PFU FP9.gpn or 1 X 10⁶ PFU MVA.gpn by iv. injection. Animals are boosted two weeks later with either virus in an identical manner to the initial immunisation.

Fourteen days after the booster immunisation, all mice are sacrificed by cervical dislocation and the T cell responses elicited against a library of peptides (20 amino acids overlapping by 10 amino acids) derived from the GPN sequence determined using the IFN-γ ELISpot assay as described in the above example.

Results were calculated as the sum of the number of epitope-specific IFNγ spot forming cells/million splenocytes (sfc/million) for each peptide.

### Results

Heterologous prime-boost immunisation with FP9.gpn and MVA.gpn (administered in either order) elicits enhanced T cell responses across the GPN sequence when compared to homologous immunisation regimes with the same vectors.

Moreover, these T cell responses include CD8+ and CD4+ responses, as well as being directed at epitopes derived from several regions of the GPN polypeptide.

Thus it is demonstrated that heterologous prime-boost is a highly effective application of the recombinant gene(s) of the present invention.

### Example 7: Epitope Mapping of GPN in Mice

The gene encoding a gag, pol and nef polyprotein (gpn; figure 1; SEQ ID NO: 11) of the Human Immunodeficiency Virus (HIV) was inserted into pox virus vectors: modified vaccinia virus Ankara (MVA.gpn) and attenuated fowlpox virus (FP9.gpn). The immune response elicited in BALB/c mice against gpn by these vectors in prime-boost immunization regimens was evaluated. These studies showed that T cell responses can be detected in the gag, pol and nef regions of this polypeptide using pools of overlapping peptides. One aim of this study was to identify individual peptides within responding peptide pools, thereby defining the location of the responding epitopes within the gag, pol and nef regions of the polypeptide.

In this example, immunogenic epitopes within each responding peptide pool are identified, the T cell subset responsible for eliciting the identified peptide-specific IFN-γ responses is delineated and sequences corresponding to the identified epitopes are compared to other studies.

### Methods

### Mice and Immunisations

Female BALB/c mice (H2d; 6 - 8 weeks) were used in all experiments and kept in individually ventilated cages in accordance with the Animals (Scientific Procedure) Act 1986 of the U.K. 1 x 106 pfu of recombinant virus was administered intravenously (i.v.; 100µ into the tail vein).

### Immunological Assays

ELISPOT assays were performed as described previously. A cut-off value of 3 times standard deviation of the negative control (responses >60 SFCs per million) was applied to detect positive responses.

### Depletion of CD4+and CD8+ cells

A single cell suspension was prepared in PBS containing 1%FCS and incubated for 15 minutes at 4°C with either anti-CD4 or anti-CD8 MACS beads (Miltenyi BiotechC®, Germany), according to the manufacturer's instructions. On completion of the incubation, cells were washed once (PBS, 1%FCS) and loaded onto a MACS column which was placed on a MACS magnet. CD4 and CD8 cell content before and after cell subset depletion was determined by two-colour flow cytometry. CD4+-deplete splenocytes contained <1% CD4+ cells whereas >6% of CD8+ cells were still preser in the splenocyte preparation following CD8+ depletion.

### Peptides

A total of 119 overlapping -20-mer peptides spanning the entire gpn-sequence were synthesized by Natural and Medical Sciences Institute (NMI; Reutlingen, Germany) (Table 1). Peptides were arranged into 6 peptide pools.

**Table 1: Peptides used in this study**

| **Peptide #** | **Amino Acid Sequence** | **Peptide Length** | **gag pool 1 = 20 peptides** |
|---|---|---|---|
| 1 | MAPIVQNLQGQMVHQAISPR | 20 | |
| 2 | GQMVHQAISPRTLNAWVKVV | 20 | |
| 3 | RTLNAWVKVVEEKAFSPEVI | 20 | |
| 4 | EEKAFSPEVIPMFSALSEGA | 20 | |
| 5 | PMFSALSEGATPQDLNTML | 19 | |
| 6 | ATPQDLNTMLNTVGGHQAAM | 20 | |
| 7 | NTVGGHQAAMQMLKETI | 17 | |
| 8 | AAMQMLQKETINEEAAEWDRL | 20 | |
| 9 | NEEAAEWDRLHPVHAGPIA | 19 | |
| 10 | LHPVHAGPIAPGQMREPR | 18 | |
| 11 | IAPGQMREPRGSDIAGTTSTL | 21 | |
| 12 | SDIAGTTSTLQEQIGWM | 17 | |
| 13 | STLQEQIGWMTNNPPIPV | 18 | |
| 14 | WMTNNPPIPVGEIYKRWIIL | 20 | |
| 15 | GEIYKRWIILGLNKIVRMY | 19 | |
| 16 | LGLNKIVRMYSPTSILDIRQ | 20 | |
| 17 | SPTSILDIRQGPKEPFRDYV | 20 | |
| 18 | GPKEPFRDYVDRFYKTLRA | 19 | |
| 19 | VDRFYKTLRAEQASQEVKNW | 20 | |
| 20 | EQASQEVKNWMTETLLVQNA | 20 | |
| | | | |
| 21 | MTETLLVQNANPDCKTILKA | 20 | **gag pool 2 = 20 peptides** |
| 22 | NPDCKTILKALGPAATLEEM | 20 | |
| 23 | LGPAATLEEMMTACQGV | 17 | |
| 24 | EEMMTACQGVGGPGHKARVL | 20 | |
| 25 | GGPGHKARVLMAARASVL | 18 | |
| 26 | VLMAARASVLSGGELDRWEK | 20 | |
| 27 | SGGELDRWEKIRLRPGGKKK | 20 | |
| 28 | IRLRPGGKKKYKLKHIVWA | 19 | |
| 29 | KYKLKHIVWASRELERFAV | 19 | |
| 30 | ASRELERFAVNPGLLETSEGCR | 22 | |
| 31 | GLLETSEGCRQILGQLQPSL | 20 | |
| 32 | RQILGQLQPSLQTGSEELR | 19 | |
| 33 | SLQTGSEELRSLYNTVATLY | 20 | |
| 34 | SLYNTVATLYCVHQRIEVK | 19 | |
| 35 | YCVHQRIEVKDTKEALEKI | 19 | |
| 36 | KDTKEALEKIEEEQNKSKKK | 20 | |
| 37 | EEEQNKSKKKAQQAAADTGN | 20 | |
| 38 | AQQAAADTGNSSQVSQNY | 18 | |
| 39 | GNSSQVSQNYTPDKKHQK | 18 | |
| 40 | NYTPDKKHQKEPPFLWMGY | 19 | |
| | | | |
| 41 | KEPPFLWMGYELHPDKWTVQ | 20 | **pol pool 1= 23 peptides** |
| 42 | ELHPDKWTVQPIVLPEKDSW | 20 | |
| 43 | PIVLPEKDSWTVNDIQKLV | 19 | |
| 44 | WTVNDIQKLVGKLNWASQIY | 20 | |
| 45 | GKLNWASQIYAGIKVKQLCK | 20 | |
| 46 | AGIKVKQLCKLLRGTKAL | 18 | |
| 47 | CKLLRGTKALTEVIPLTEEA | 20 | |
| 48 | TEVIPLTEEAELELAENREI | 20 | |
| 49 | ELELAENREILKEPVHGVYY | 20 | |
| 50 | LKEPVHGVYYDPSKDLIAEI | 20 | |
| 51 | DPSKDLIAEIQKQGQGQWTY | 20 | |
| 52 | IQKQGQGQWTYQIYQEPFK | 19 | |
| 53 | TYQIYQEPFKNLKTGKYARM | 20 | |
| 54 | NLKTGKYARMRGAHTNDVKQ | 20 | |
| 55 | RGAHTNDVKQLTEAVQKIA | 19 | |
| 56 | KQLTEAVQKIATESIVIWGK | 20 | |
| 57 | ATESIVIWGKTPKFKLPIQK | 20 | |
| 58 | TPKFKLPIQKETWEAWWTEY | 20 | |
| 59 | ETWEAWWTEYWQATWIPEW | 19 | |
| 60 | YWQATWIPEWEFVNTPPLVK | 20 | |
| 61 | EFVNTPPLVKLWYQLEKEPI | 20 | |
| 62 | LWYQLEKEPIVGAETFPI | 18 | |
| 63 | PIVGAETFPISPIETVPVKL | 20 | |
| | | | |
| 64 | SPIETVPVKLKPGMDGPKVK | 20 | **pol pool 2 = 23 peptides** |
| 65 | KPGMDGPKVKQWPLTEEKIK | 20 | |
| 66 | KQWPLTEEKIKALVEICTEM | 20 | |
| 67 | KALVEICTEMEKEGKISKI | 19 | |
| 68 | MEKEGKISKIGPENPYNTPV | 20 | |
| 69 | GPENPYNTPVFAIKKKDSTK | 20 | |
| 70 | FAIKKKDSTKWRKLVDFREL | 20 | |
| 71 | WRKLVDFRELNKRTQDFWEV | 20 | |
| 72 | NKRTQDFWEVQLGIPHPAGL | 20 | |
| 73 | VQLGIPHPAGLKKKSVTVL | 20 | |
| 74 | LKKKKSVTVLDVGDAYFSV | 19 | |
| 75 | LDVGDAYFSVPLDKDFRKY | 19 | |
| 76 | VPLDKDFRKYTAFTIPSI | 18 | |
| 77 | KYTAFTIPSINNETPGIRYQ | 20 | |
| 78 | NNETPGIRYQYNVLPQGWK | 19 | |
| 79 | YQYNVLPQGWKGSPAIFQ | 18 | |
| 80 | GWKGSPAIFQSSMTKILEPF | 20 | |
| 81 | SSMTKILEPFRKQNPDIVIY | 20 | |
| 82 | RKQNPDIVIYQYMDDLYV | 18 | |
| 83 | IYQYMDDLYVGSDLEIGQHR | 20 | |
| 84 | GSDLEIGQHRTKIEELRQHL | 20 | |
| 85 | TKIEELRQHLLRWGFTTPDK | 20 | |
| 86 | LRWGFTTPDKKHQKEPPFLV | 20 | |
| | | | |
| 87 | KHQKEPPFLVWKFDSRLAFH | 20 | **nef pool 1 = 16 peptides** |
| 88 | WKFDSRLAFHHMARELHPEY | 20 | |
| 89 | HMARELHPEYYKDCDPEKEV | 20 | |
| 90 | YKDCDPEKEVLVWKFDA | 17 | |
| 91 | KEVLVWKFDANEGENNSLLH | 20 | |
| 92 | NEGENNSLLHPMSLHGM | 17 | |
| 93 | LLHPMSLHGMDDPEKEVPEK | 20 | |
| 94 | DDPEKEVPEKVEEANEGENG | 20 | |
| 95 | VEEANEGENGPGIRYPLTF | 19 | |
| 96 | GPGIRYPLTFGWCFKLVPV | 19 | |
| 97 | FGWCFKLVPVEPEKVEEWQ | 19 | |
| 98 | VEPEKVEEWQNYTPGPGIRY | 20 | |
| 99 | NYTPGPGIRYQKRQDILDLW | 20 | |
| 100 | YQKRQDILDLWVYHTQGYF | 19 | |
| 101 | LWVYHTQGYFPDWQNYTPEGL | 21 | |
| 102 | DWQNYTPEGLIYSQKRQDI | 19 | |
| | | | |
| 103 | LIYSQKRQDIPMTYKAALDL | 20 | **nef pool 2 = 17peptides** |
| 104 | PMTYKAALDLSHFLKEKGGL | 20 | |
| 105 | SHFLKEKGGLEGLIYSPQV | 19 | |
| 106 | LEGLIYSPQVPLRPMTYKAA | 20 | |
| 107 | PLRPMTYKAADCAWLEAQ | 18 | |
| 108 | AADCAWLEAQEEEEVGFPVR | 20 | |
| 109 | EEEEVGFPVRPQVPLRNTAA | 20 | |
| 110 | PQVPLRNTAANNADCAWLA | 19 | |
| 111 | ANNADCAWLADGVGAVSRDL | 20 | |
| 112 | DGVGAVSRDLEKHGAITSSNTA | 22 | |
| 113 | HGAITSSNTAANNRRAEPAA | 20 | |
| 114 | ANNRRAEPAADGVGAMGGKW | 20 | |
| 115 | DGVGAMGGKWSKRSVVGW | 18 | |
| 116 | KWSKRSVVGWPTVRERMRRA | 20 | |
| 117 | PTVRERMRRAEPARGPGRAF | 20 | |
| 118 | EPARGPGRAFVTIYPYDV | 18 | |
| 119 | AFVTIYPYDVPDYA | 14 | |
| | | | |
| AMQ | AMQMLKETI | 9 | Immunodomi nant control |
| RGP | RGPGRAFVTI | 10 | Reporter epitope control |

### Results

### Identification of immunogenic epitopes within each responding peptide pool

Individual epitope mapping of responding peptide pools was conducted by IFN-γ.

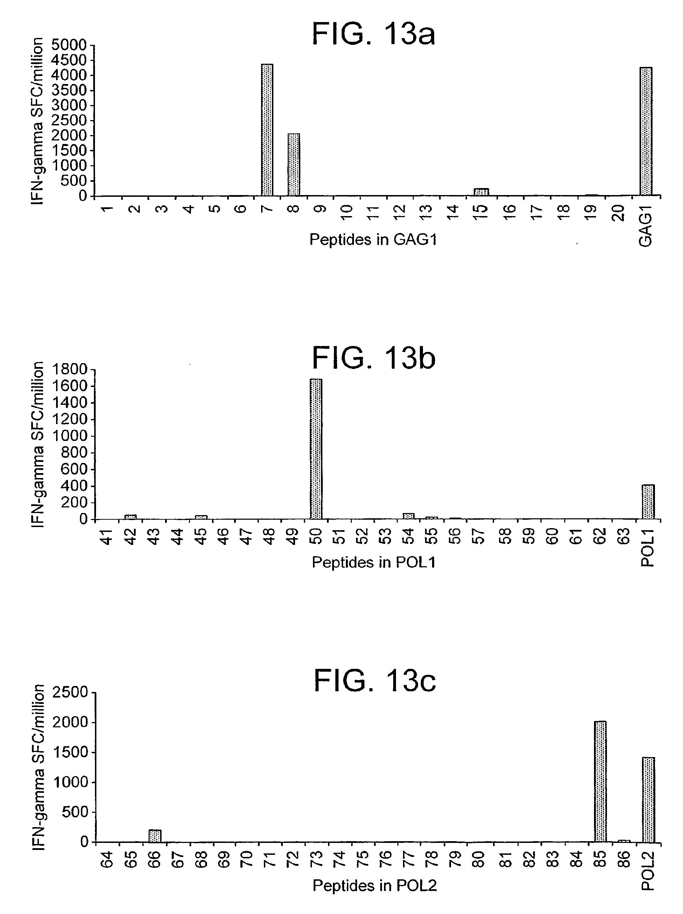

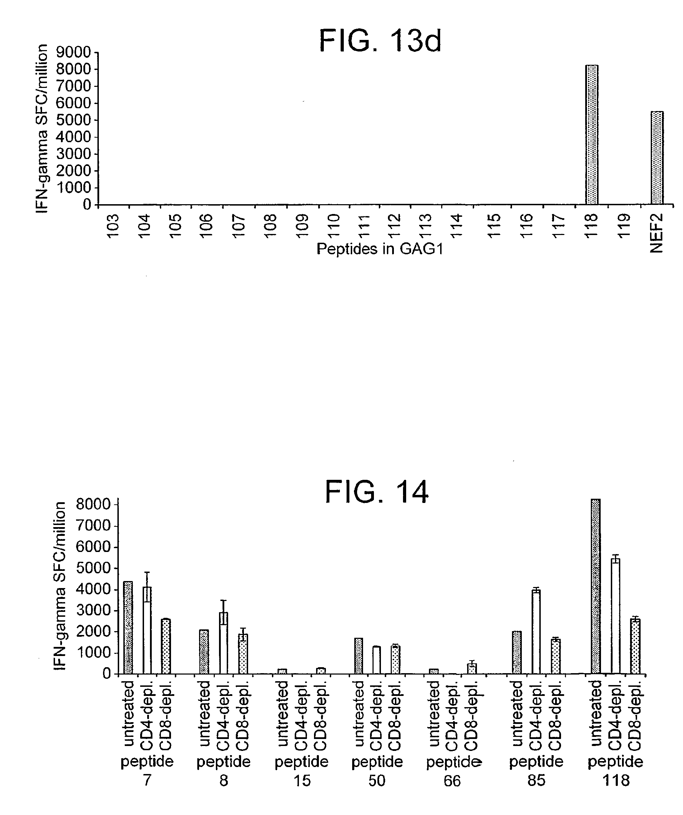

ELISPOT assay was performed using overlapping peptides from each pool (see Figure 13). Figure 13 shows IFN-γ responses elicited against peptide pools and overlapping peptide fragments covering the entire gpn-sequence (with markers). Groups of female BALB/c mice (H2^{d}; n=4 mice) were immunised intravenously (i.v.) with 1 x 10⁶ plaque forming units (pfu) of MVA.gpn. Fourteen days later, mice were boosted by i.v. administration of 1 x 10⁶ pfu of FP9.gpn. Fourteen days after boosting, spleens were removed, pooled (n=4 spleens) and the number of IFN-γ spot forming cells (SFC) per million splenocytes was determined by IFN-γ ELISPOT. Columns represent the number of SFC/million.

In summary these results indicate that the following individual peptides in responding peptide pools were identified:
- In peptide pool gag1:: peptide 7: NTVGGHQAAMQMLKETI
peptide 8: AAMQMLKETINEEAAEWDRL
peptide 15: GEIYKRWIILGLNKIVRMY
- In peptide pool poll:: peptide 50: LKEPVHGVYYDPSKDLIAEI
- In peptide pool po12:: peptide 66: KQWPLTEEKIALVEICTEM
peptide 85: TKIEELRQHLLRWGFTTPDK
- In peptide pool nef2:: peptide 118: EPARGPGRAFVTIYPYDV

The underlined sequence in nef2 corresponds to the reporter epitope that was added to provide a measure of the induction of IFN-γ responses. This response in the penultimate peptide, 118, indicates that the entire GPN polyprotein is being expressed. However the IFN-γ response detected in peptide pool nef2 was not due to epitope sequences derived from native nef but due to the inserted reporter epitope. Example 8 contains very few CD8+ T cell epitopes in nef for BALB/c mice (H-2d) so the lack of response in nef is not surprising.

### Determination of the T cell subset responsible for eliciting the identified peptide-specific IFN-γ responses

Figure 14 shows IFN-γ responses elicited against selected peptides from gpn-sequence. Groups of female BALB/c mice (H2^{d}; n=4 mice) were immunised intravenously (i.v.) with 1 x 10⁶ plaque forming units (pfu) of MVA.gpn. Fourteen days later, mice were boosted by i.v. administration of 1 x 10⁶ pfu of FP9.gpn. Fourteen days after boosting, spleens were removed, pooled (n=4 spleens) and either left untreated, depleted of CD4⁺ cells or depleted of CD8⁺ cells as indicated. The number of IFN-γ spot forming cells (SFC) per million splenocytes was determined by IFN-γ ELISPOT. Columns represent the number of SFC/million.

CD4+ cell depletion studies demonstrated a dramatic reduction in IFN-γ secretion following *ex vivo* stimulation with peptides 15 and 66, whereas a moderate reduction was observed after stimulation with peptide 118. These findings suggest that CD4⁺ T cells elicit IFN-γ responses against peptides 15, 66 and 118 (see figure 14).

A partial reduction in the IFN-γ response against peptides 7, 50, 85 and 118 was observed following depletion of CD8⁺ T cells. The partial reduction in IFN-γ responses observed against these peptides indicates that they are mediated by CD8⁺ T cells (see figure 14).

### Comparison of sequences corresponding to the identified epitopes with other described epitopes

To determine whether the peptide sequences identified in this study have been defined previously , they were compared with those in the Los Alamos HIV sequence data base (mouse; see example 8 (BALB/c; H2^{d}))

Epitopes within the sequences corresponding to peptides 7,8,15 and 118 have been previously identified in BALB/c mice according to Example 8, whereas epitopes within sequences corresponding to peptides 50, 66 and peptide 85 have not been previously described.

Epitopes within the sequences corresponding to all seven peptides described in this report have been identified as immunogenic in HIV infected humans as shown in Table 2.

**Table 2: Results from Los Alamos HIV data base search.**

| Peptide # | Mouse (BALB/c; H2^{d}) | Human |
|---|---|---|
| 7 | MHC class I K^{d}-restricted | MHC class I-restricted |
| 8 | MHC class I K^{d}-restricted | MHC class I-restricted |
| 15 | MAC class II-restricted | MHC class II-restricted |
| 50 | this study | MAC class I-restricted |
| 66 | this study | MHC class I-restricted |
| 85 | this study | MHC class I-restricted |
| 118 | MHC class I Dd-restricted MHC class II IA^{d}-restricted | MHC class I-restricted MHC class II-restricted |

These studies indicate that heterologous prime-boost immunisation regimens using MVA.gpn and FP9.gpn elicit potent CD4⁺ and GD8⁺ IFN-γ secreting T cell responses against the gag and pol regions of the gpn-molecule in BALB/c mice. In common with previous studies conducted with BALB/c mice, no immunogenic epitopes were identified in the nef molecule.

### Example 8: Maps of CTL epitope locations plotted by protein

This example presents preferred CTL epitopes following HIV Molecular Immunology 2002: Maps of CTL Epitope Locations Plotted by Protein; Theoretical Biology & Biophysics, Los Alamos National Laboratory, August 7, 2003. Linear CTL epitopes less than 22 amino acids long are shown. Also shown are T-Helper epitope maps of the preferred T-Helper epitopes.

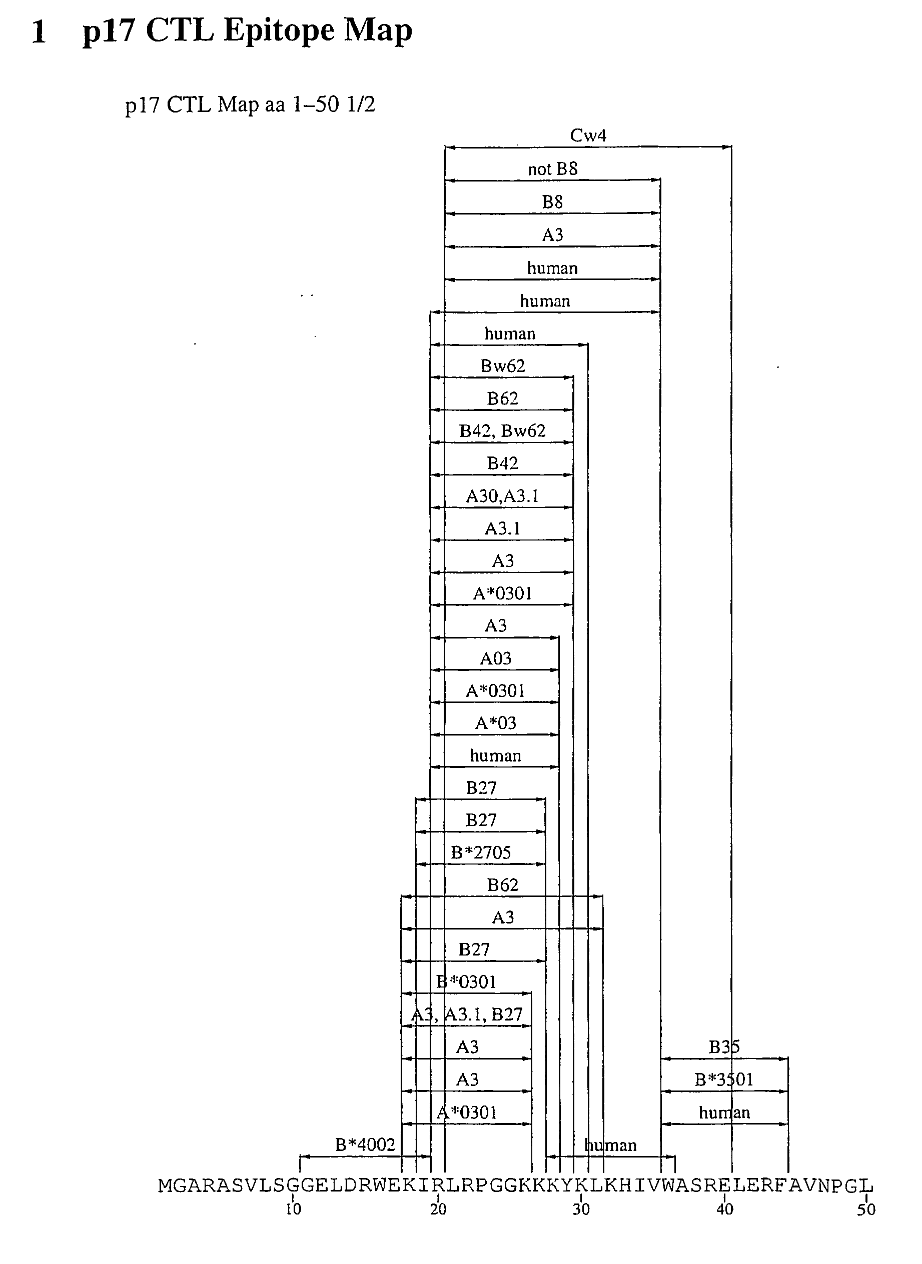
1 p17 CTL Epitope Map

p17 CTL Map aa 1–50 1/2

Cw4
not B8
B8
A3
human
human
human
Bw62
B62
B42, Bw62
B42
A30,A3.1
A3.1
A3
A*0301
A3
A03
A*0301
A*03
human
B27
B27
B*2705
B62
A3
B27
B*0301
A3, A3.1, B27
A3
B35
A3
B*3501
A*0301
human
B*4002
human
MGARASVLSGGELDRWEKIRLRPGGKKKYKLKHIVWASRELERFAVNPGL
10 20 30 40 50


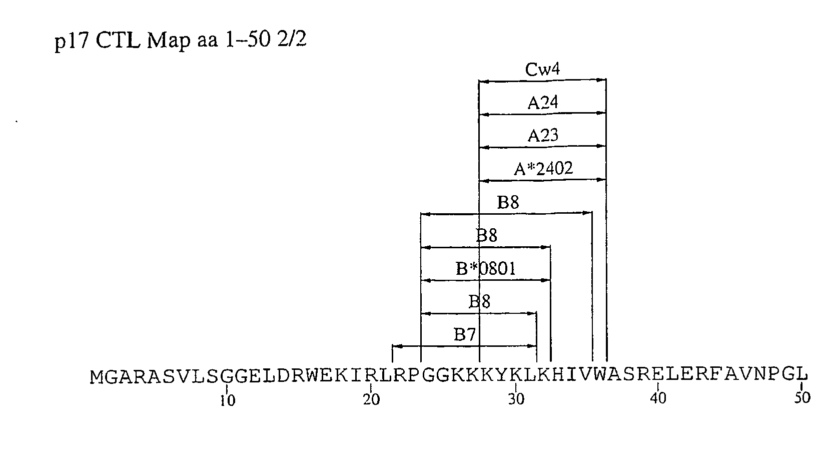
p17 CTL Map aa 1–50 2/2


Cw4

A24

A23

A*2402

B8

B8

B*0801

B8

B7

MGARASVLSGGELDRWEKIRLRPGGKKKYKLKHIVWASRELERFAVNPGL

10 20 30 40 50

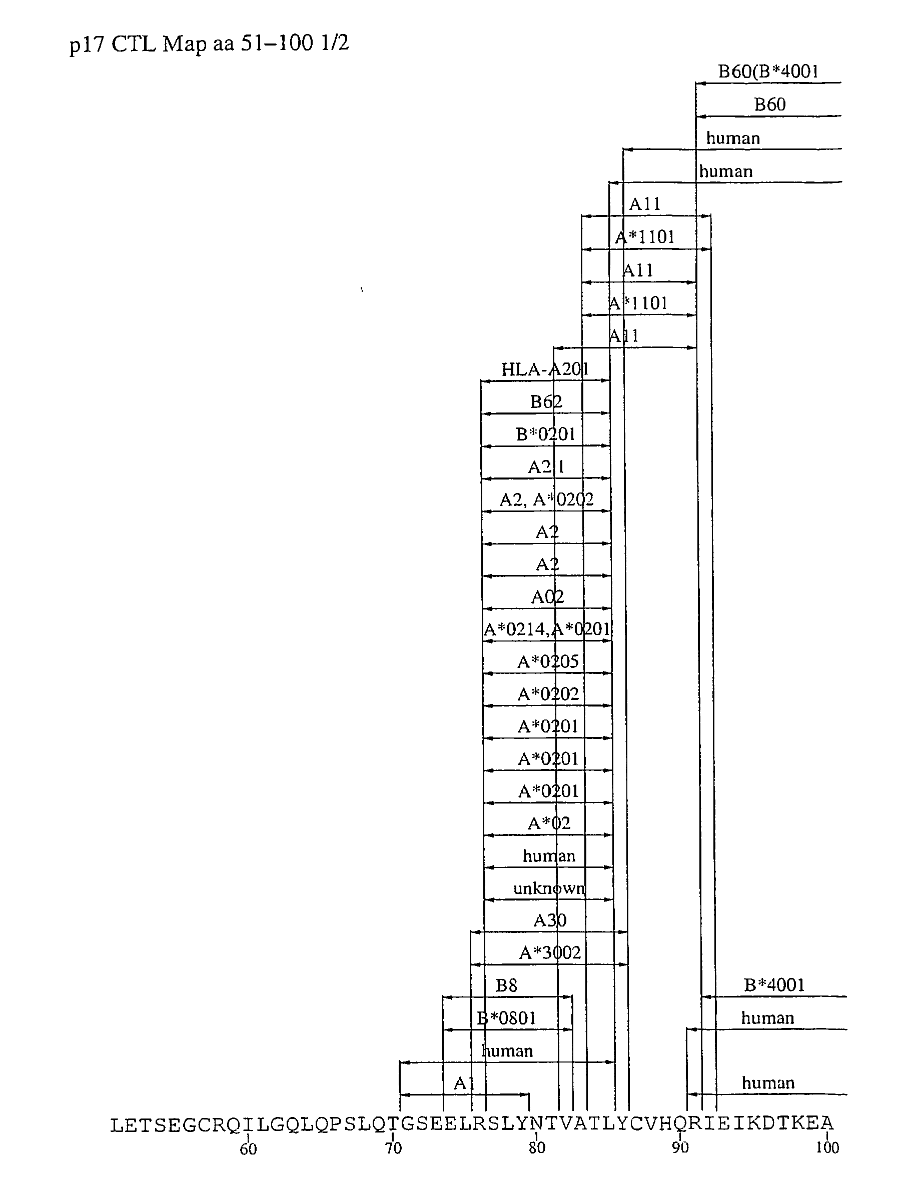
p17 CTL Map aa 51–100 1/2

B60(B*4001
B60
human
human
A11
A*1101
A11
A*1101
A11
HLA-A201
B62
B*0201
A2.1
A2, A*0202
A2
A2
A02
A*0214,A*0201
A*0205
A*0202
A*0201
A*0201
A*0201
A*02
human
unknown
A30
A*3002
B8
B*4001
B*0801
human
human
A1
human

LETSEGCRQILGQLQPSLQTGSEELRSLYNTVATLYCVHQRIEIKDTKEA
60 70 80 90 100


p17 CTL Map aa 51–100 2/2

B8,B60
B8
human
B60/B61

LETSEGCRQILGQLQPSLQTGSEELRSLYNTVATLYCVHQRIEIKDTKEA
60 70 80 90 100

Figure 5: p17 CTL Map aa 101–132

B8,B60
B8
human
B60/B61
B60(B*4001
B60
B*4001
human
human
human
human
B35
B*3501
A33

LDKIEEEQNKSKKKAQQAAADTGHSNQVSQNY
110 120 130

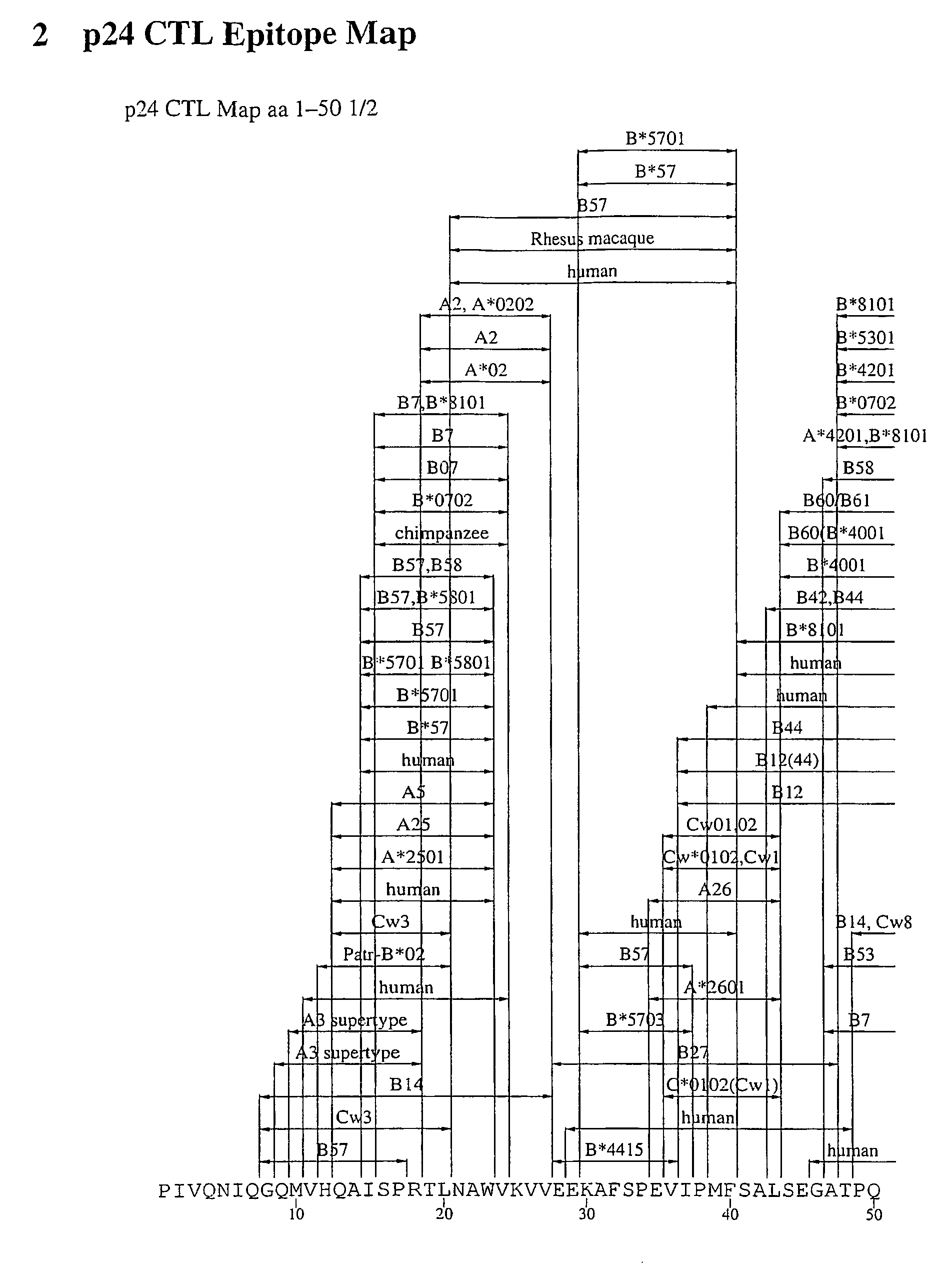
2 p24 CTL Epitope Map

p24 CTL Map aa 1–50 1/2

B*5701
B*57
B57
Rhesus macaque
human
A2, A*0202
A2
A*02
B7,B*8101
B7
B07
B*0702
chimpanzee
B57,B58
B57,B*5801
B57
B*5701 B*5801
B*5701
B*57
human
A5
A25
A*2501
human
Cw3
Patr-B*02
human
A3 supertype
A3 supertype
B14
Cw3
B57
B*8101
B*5301
B*4201
B*0702
A*4201,B*8101
B58
B60/B61
B60(B*4001
B*4001
B42,B44
B*8101
human
human
B44
B12(44)
B12
Cw01,02
Cw*0102,Cw1
A26
human
B14, Cw8
B57
B53
A*2601
B*5703
B7
B27
C*0102(Cw1)
human
B*4415
human
PIVQNIQGQMVHQAISPRTLNAWVKVVEEKAFSPEVIPMFSALSEGATPQ
10 20 30 40 50

p24 CTL Map aa 1–50 2/2

B7
C*0802(Cw8)
B7
B53
B42
B*8101, B*5301, B07
human
B58
B57/B58
B57,B58
B57
B*5703
B*5701, B*5703
PIVQNIQGQMVHQAISPRTLNAWVKVVEEKAFSPEVIPMFSALSEGATPQ
10 20 30 40 50

p24 CTL Map aa 51–100 1/2
Cw8, B*1402
Cw8
C*0802
B14, Cw8
B14
chimpanzee
B14, Cw8
B7
C*0802(Cw8)
B7
B53
B42
B*8101, B*5301, B07
B*8101
B*5301
B*4201
B*0702
A*4201,B*8101
B58
B53
B7
human
B60/B61
B60(B*4001
B*4001
B42,B44
B*8101
human
human
B44
B12(44)
B12
human
B53
B*5301
H-2Kd
A25
H-2kd
H-2d
B52
human
A*2501
B*4002
human
A2
B39
B*3901
B*4002
B7
B55
A2
human
human
DLNTMLNTVGGHQAAMQMLKETINEEAAEWDRVHPVHAGPIAPGQMREPR
60 70 80 90 100

Figure 9: p24 CTL Map aa 51–100 2/2
human
DLNTMLNTVGGHQAAMQMLKETINEEAAEWDRVHPVHAGPIAPGQMREPR
60 70 80 90 100

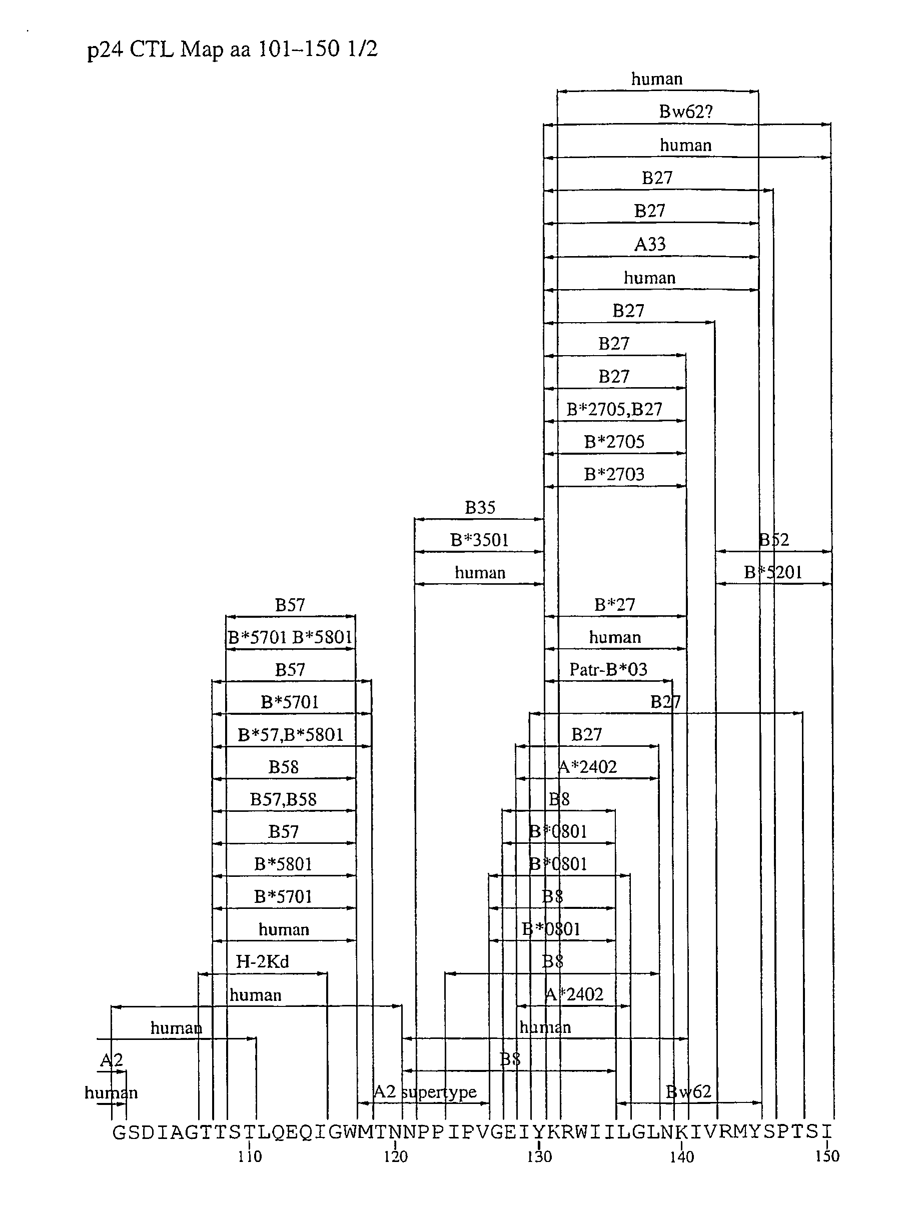
p24 CTL Map aa 101–150 1/2

human
Bw62?
human
B27
B27
A33
human
B27
B27
B27
B*2705,B27
B*2705
B*2703
B35
B*3501
B52
human
B*5201
B57
B*27
B*5701 B*5801
human
B57
Patr-B*03
B*5701
B27
B*57,B*5801
B27
B58
A*2402
B57,B58
B8
B57
B*0801
B*5801
B*0801
B*5701
B8
human
B*0801
H-2Kd
B8
human
A*2402
human
human
A2
B8
human
A2 supertype
Bw62
GSDIAGTTSTLQEQIGWMTNNPPIPVGEIYKRWIILGLNKIVRMYSPTSI
110 120 130 140 150


p24 CTL Map aa 101-150 2/2

B62
B*1501
human
B62
A33
B27

GSDIAGTTSTLQEQIGWMTNNPPIPVGEIYKRWIILGLNKIVRMYSPTSI
110 120 130 140 150

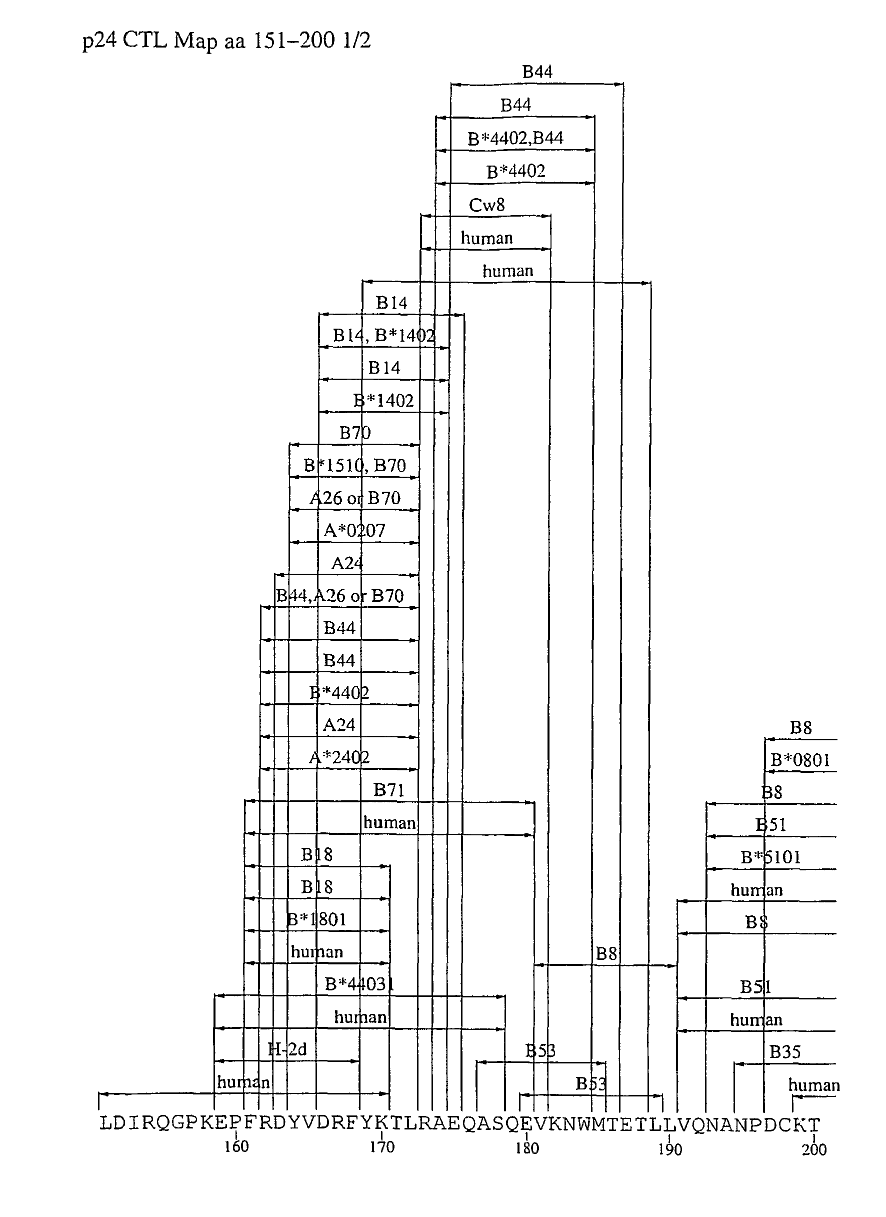
p24 CTL Map aa 151–200 1/2

B44
B44
B*4402,B44
B*4402
Cw8
human
human
B14
B14, B*1402
B14
B*1402
B70
B*1510, B70
A26 or B70
A*0207
A24
B44,A26 or B70
B44
B44
B*4402
A24
A*2402
B8
B*0801
B71
B8
human
B51
B18
B*5101
B18
human
B*1801
B8
human
B8
B*44031
B51
human
human
H-2d
B53
B35
human
B53
human
LDIRQGPKEPFRDYVDRFYKTLRAEQASQEVKNWMTETLLVQNANPDCKT
160 170 180 190 200


p24 CTL Map aa 151–200 2/2

B57
Cw4
B57
B53
B*5701
B*5301, B57
B*5301

LDIRQGPKEPFRDYVDRFYKTLRAEQASQEVKNWMTETLLVQNANPDCKT
160 170 180 190 200

Figure 14: p24 CTL Map aa 201–231

human
B8
B*0801
B7
B35
B7
B8
human
B51
B7
B*5101
human
human
B*0702
B8
B7
B51
A11
human
A*1101

ILKALGPAATLEEMMTACQGVGGPGHKARVL
210 220 230

5 RT CTL Epitope Map

RT CTL Map aa 1–50

B51
B*5101
H-2Kk
C3H/HeJ mice
A3
A*0301
B7,B8
broad
B8
H2k
B*0801
H-2d
B7
A2
A2, B61
A*0201
PISPIETVPVKLKPGMDGPKVKQWPLTEEKIKALVEICTEMEKEGKISKI
10 20 30 40 50

Figure 21: RT CTL Map aa 51–100

Cw4
human
A3 supertype
A11
broad
A3
H2k
A3
H-2d
A3
GPENPYNTPVFAIKKKDSTKWRKLVDFRELNKRTQDFWEVQLGIPHPAGL
60 70 80 90 100

RT CTL Map aa 101-150
B35
B*3501,B35
B*3501
Cw4
A2, A*0201
A2
A*0201
B51
B35
B*5101
B*3501
A*0217, B*5101
Cw4
A2 supertype
Cw4
A2
human
A2
A11
A11
A3
human
A3
B*5101, B24
KKKKSVTVLDVGDAYFSVPLDEDFRKYTAFTIPSINNETPGIRYQYNVLP
110
120
130
140
150

RT CTL Map aa 101-150
B35
B*3501,B35
B*3501
Cw4
A2, A*0201
A2
A*0201
B51
B35
B*5101
B*3501
A*0217, B*5101
Cw4
A2 supertype
A2
Cw4
human
A2
A11
A11
human
A3
A3
B*5101, B24
KKKKSVTVLDVGDAYFSVPLDEDFRKYTAFTIPSINNETPGIRYQYNVLP
110
120
130
140
150

RT CTL Map aa 251–300
B42
Bw62
B62
B*1501
A2 supertype
B57
B*4201
B57
A3
B51
B57
A3
B35, B51
B*5801
A*1101
B35
B*5701, B*5801
human
B*3501, B*5301, B*5101, B*0702
B*5701
human
B57
A30
B*3501 AND B*5101
B*5701
A*3002
B*3501
SWTVNDIQKLVGKLNWASQIYPGIKVRQLCKLLRGTKALTEVIPLTEEAE
260
270
280
290
300

RT CTL Map aa 301-350
Bw62
B62
B*1501
A2.1
A2.1
A2, A2 transgenic
A2, A*0202
A2, A*0201
A2 supertype
A2
A2
A2
A02
A*0201,A*0205
A*0201
A*0201
A*0201
A*0201
A*0201
A*02
human
A*0201
B51
B35, B51
B35
3*3501, B*5301, B*5101, B*0702
B*3501 AND B*5101
B*3501
A11
A*1101
A11
A11
human
LELAENREILKEPVHGVYYDPSKDLIAEIQKQGQGQWTYQIYQEPFKNLK
310 320 330 340 350

RT CTL Map aa 351–400

B57
human
B70
A3
B*5701 B*5801
B44
A*3002
B*5701
A32
A11
A28, A*6802
A*3201
TGKYARMRGAHTNDVKQLTEAVQKITTESIVIWGKTPKFKLPIQKETWET
360
370
380
390
400

Figure 28: RT CTL Map aa 401–450

A29
A28
B*4501
B45
B35
B35
B*3501
B35, B51
B35
B44
B*3501
A*2601
A32
A2
A26
A*3201
A11
A*6802
WWTEYWQATWIPEWEFVNTPPLVKLWYQLEKEPIVGAETFYVDGAANRET
410
420
430
440
450

Figure 29: RT CTL Map aa 451–500

Cw8
B14, B*1402
B*1503
B14
A2.1
A*2601
A2
A29
human
KLGKAGYVTNRGRQKVVTLTDTTNQKTELQAIYLALQDSGLEVNIVTDSQ
460
470
480
490
500

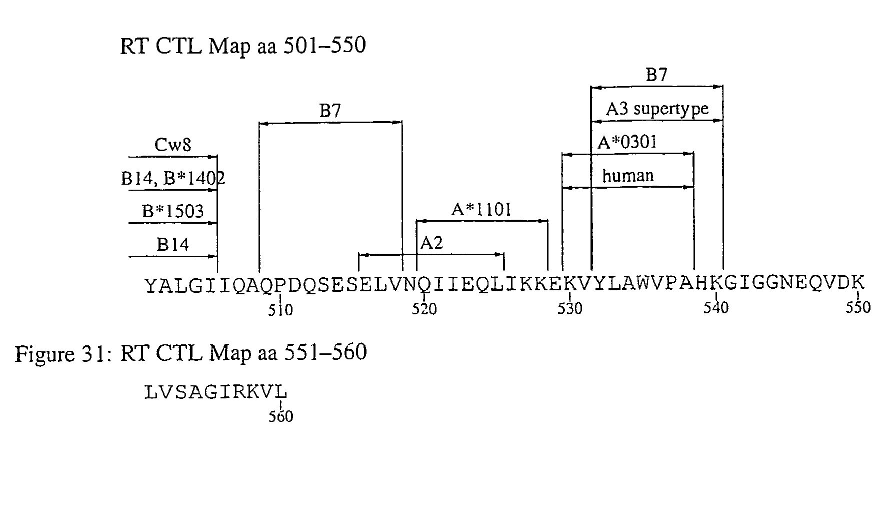
RT CTL Map aa 501–550

B7

A3 supertype

B7

A*0301

Cw8

human

B14, B*1402

A*1101

B*1503

A2

B14


YALGIIQAQPDQSESELVNQIIEQLIKKEKVYLAWVPAHKGIGGNEQVDK
510 520 530 540 550

Figure 31: RT CTL Map aa 551–560

LVSAGIRKVL
560

13 Nef CTL Epitope Map

Nef CTL Map aa 1–50

B8
B*0801
B*0801
H-2b
HLA-A201 transgenic
human
MGGKWSKSSVIGWPTVRERMRRAEPAADRVGAASRDLEKHGAITSSNTAA
10 20 30 40 50

Nef CTL Map aa 51–100 1/3

B27
B*0301
A3.1
A3, A11, B35
A3 supertype
A3
A2, A3, A11, B35
A11
A11
A03
A*0301, A11
A*0301
human
human
human
B7
B51
B35
B*3501
B7
B35
B*0702
human
human
B7
B*0702
B7
B35
B*3501
human
human
A1, B8
H-2b

human
B60/B61
B60(B*4001
human
B60
B*4002
B*4001
A2
B8
B8
human
A1, B8
B*0801
B35
A2
A3.1
A3
A*0301
B*08
A3
human

TNAACAWLEAQEEEEVGFPVTPQVPLRPMTYKAAVDLSHFLKEKGGLEGL
60 70 80 90 100

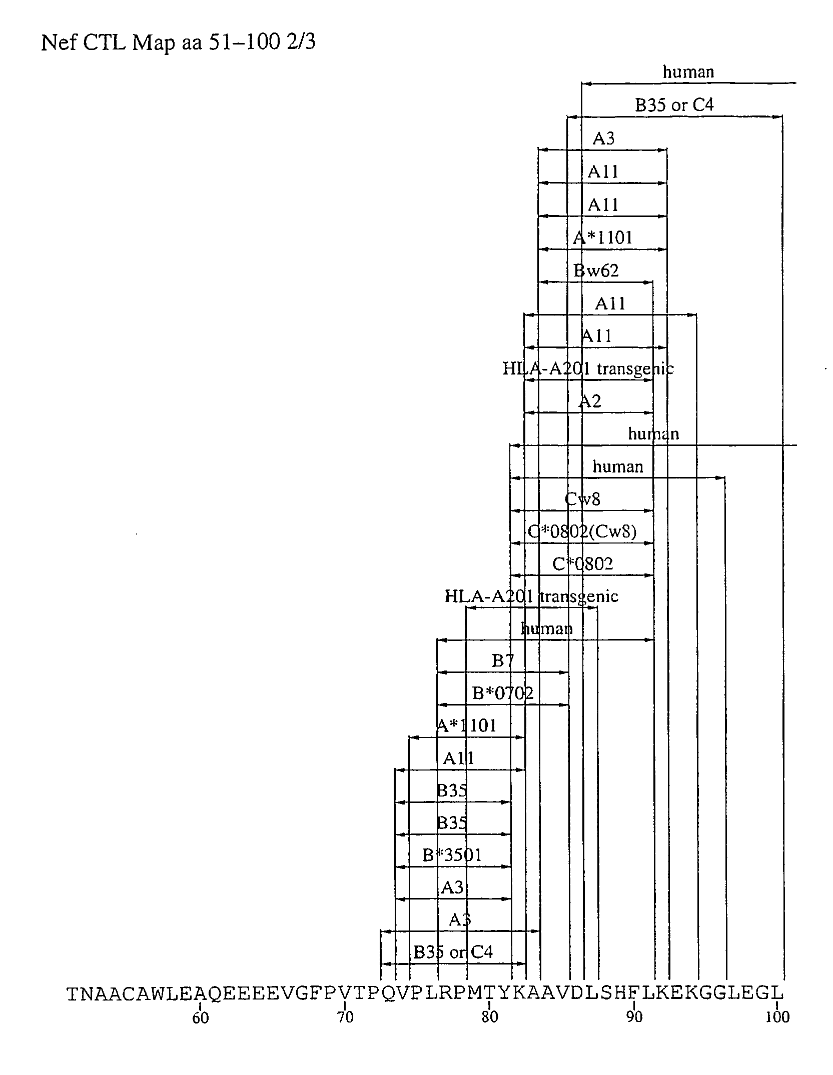
Nef CTL Map aa 51-100 2/3


human
B35 or C4
A3
A11
A11
A*1101
Bw62
A11
A11
HLA-A201 transgenic
A2
human
human
Cw8
C*0802(Cw8)
C*0802
HLA-A201 transgenic
human
B7
B*0702
A*1101
A11
B35
B35
B*3501
A3
A3
B35 or C4

TNAACAWLEAQEEEEVGFPVTPQVPLRPMTYKAAVDLSHFLKEKGGLEGL
60 70 80 90 100

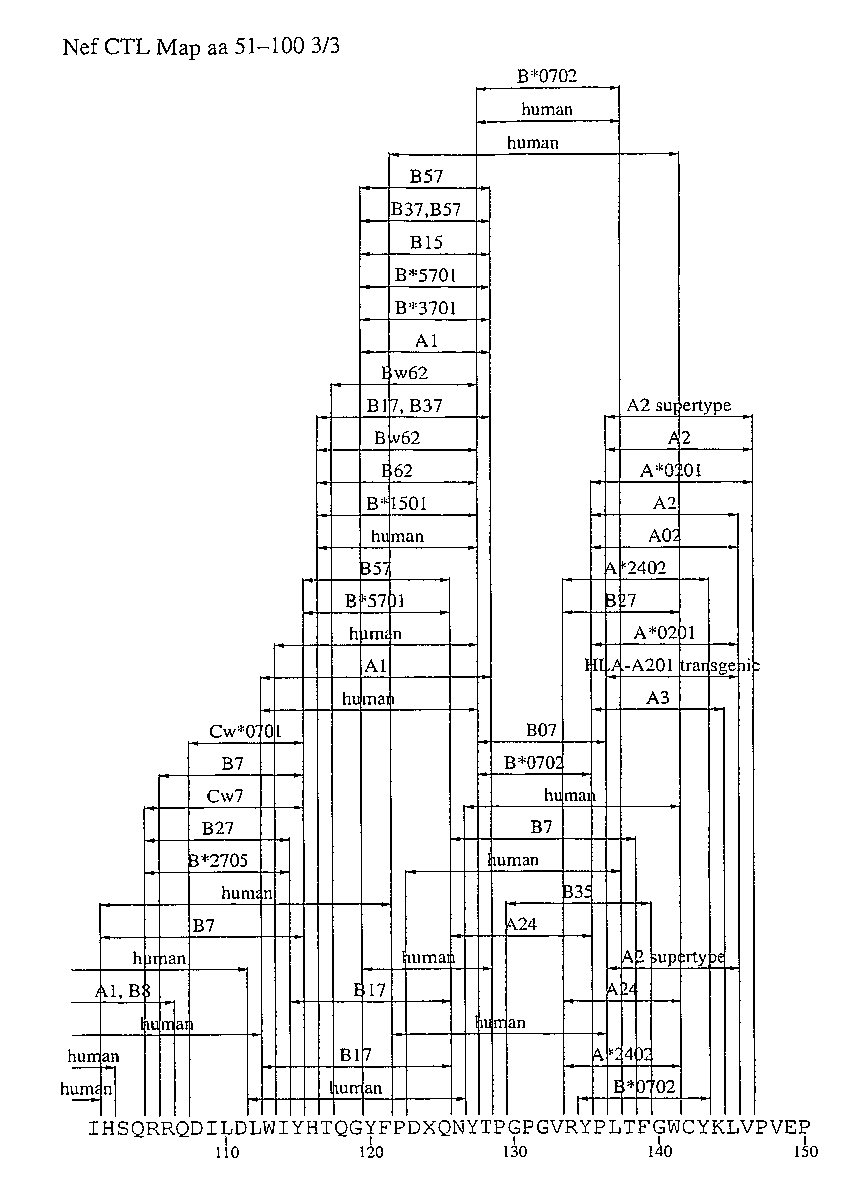
Nef CTL Map aa 51–100 3/3

B*0702
human
human
B57
B37,B57
B15
B*5701
B*3701
A1
Bw62
B17, B37
A2 supertype
Bw62
A2
B62
A*0201
B*1501
A2
human
A02
B57
A*2402
B*5701
B27
human
A*0201
A1
HLA-A201 transgenic
human
A3
Cw*0701
B07
B7
B*0702
Cw7
human
B27
B7
B*2705
human
human
B35
B7
A24
human
human
A2 supertype
A1, B8
B17
A24
human
human
human
B17
A*2402
human
human
B*0702


IHSQRRQDILDLWIYHTQGYFPDXQNYTPGPGVRYPLTFGWCYKLVPVEP
110 120 130 140 150

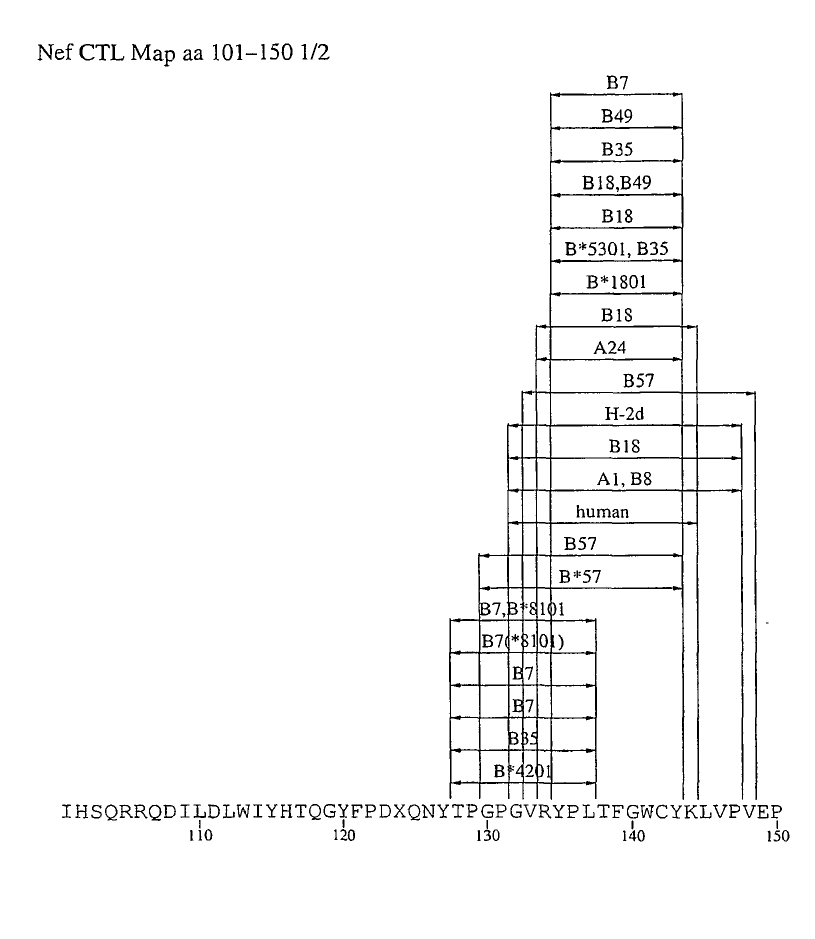
Nef CTL Map aa 101-150 1/2

B7
B49
B35
B18,B49
B18
B*5301, B35
B*1801
B18
A24
B57
H-2d
B18
A1, B8
human
B57
B*57
B7,B*8101
B7(*8101)
B7
B7
B35
B*4201


IHSQRRQDILDLWIYHTQGYFPDXQNYTPGPGVRYPLTFGWCYKLVPVEP
110 120 130 140 150

Nef CTL Map aa 101–150 2/2

A1
A3
A2, A*0202, A*0201
A2
A*0201
A*0201
B35 or C4
B52
B51
A24
B*1503
human
H-2d
B35
A25
A2, A25(10)
A1, B8
A2
A*0201
A*0201
A*0201
B7
human
B35
B35
human
human
DKIEEANKGENTSLLHPVSLHGMDDPEREVLEWRFDSRLAFHHVARELHP
160 170 180 190 200

Figure 78: Nef CTL Map aa 151–200

A1
B35 or C4
human
EYFKNC
206

. Nef CTL Map aa 201–206
A1
B35 or C4
human
EYFKNC
206

1 p17 T-Helper Epitope Map

p17 T-Helper Map aa 1-50

human
human
DR13.02
MGARASVLSGGELDRWEKIRLRPGGKKKYKLKHIVWASRELERFAVNPGL
10 20 30 40 50

Figure 2: p17 T-Helper Map aa 51-100

human
LETSEGCRQILGQLQPSLQTGSEELRSLYNTVATLYCVHQRIEIKDTKEA
60 70 80 90 100

Figure 3: p17 T-Helper Map aa 101-132

human human
LDKIEEEQNKSKKKAQQAAADTGHSNQVSQNY
110 120 130

2 p24 T-Helper Epitope Map

p24 T-Helper Map aa 1–50

H-2d, H-2b
human
I-Ab and HLA-DR
DR supermotif
human
human
A*0201
human
DR1
human
PIVQNIQGQMVHQAISPRTLNAWVKVVEEKAFSPEVIPMFSALSEGATPQ
10 20 30 40 50

Figure 5: p24 T-Helper Map aa 51–100

H-2b
DQ7
macaque
human
human
human
human
human
human
human
human
DR
DR4
human
DLNTMLNTVGGHQAAMQMLKETINEEAAEWDRVHPVHAGPIAPGQMREPR
60 70 80 90 100

Figure 6: p24 T-Helper Map aa 101–150

human
human
DR supermotif
macaque
human
DR4
human
human
DRB1*1301, DRB1*1302
macaque
DR4
DR supermotif
human
H-2d
human
human
DR4
human
human
H-2b
DRB1*1301
human
GSDIAGTTSTLQEQIGWMTNNPPIPVGEIYKRWIILGLNKIVRMYSPTSI
110 120 130 140 150

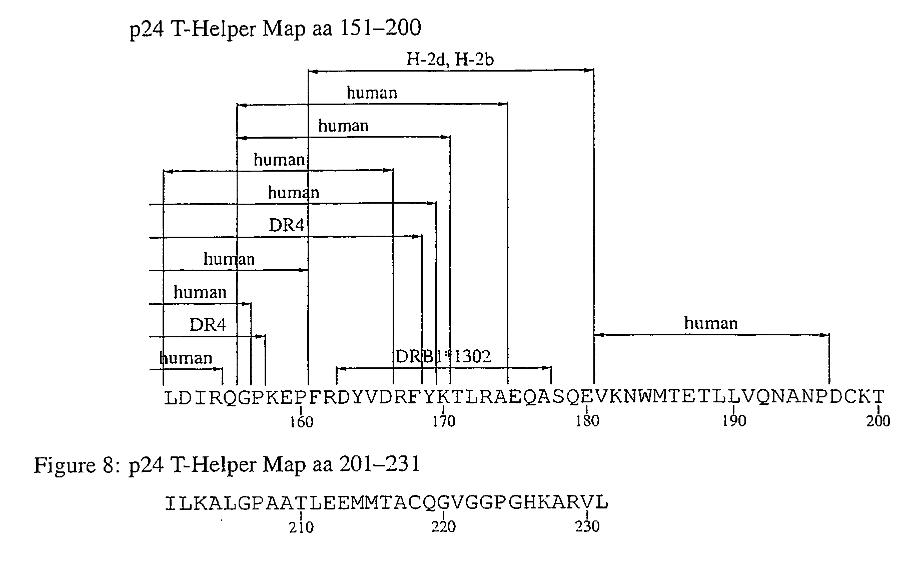
p24 T-Helper Map aa 151–200
H-2d, H-2b
human
human
human
human
DR4
human
human
DR4
human
DRB1*1302
human
LDIRQGPKEPFRDYVDRFYKTLRAEQASQEVKNWMTETLLVQNANPDCKT
160 170 180 190 200
Figure 8: p24 T-Helper Map aa 201–231
ILKALGPAATLEEMMTACQGVGGPGHKARVL
210 220 230

5 RT T-Helper Epitope Map

RT T-Helper Map aa 1–50

H-2k
human
H-2k
human

PISPIETVPVKLKPGMDGPKVKQWPLTEEKIKALVEICTEMEKEGKISKI
10 20 30 40 50

Figure 15: RT T-Helper Map aa 51–100

H-2k
human
H-2k
human
H-2k
H-2t4

GPENPYNTPVFAIKKKDSTKWRKLVDFRELNKRTQDFWEVQLGIPHPAGL
60 70 80 90 100

Figure 16: RT T-Helper Map aa 101–150

H-2t4
H-2k,i5
I-Ab and HLA-DR
DR supermotif
H-2t4

KKKKSVTVLDVGDAYFSVPLDEDFRKYTAFTIPSINNETPGIRYQYNVLP
110 120 130 140 150

Figure 17: RT T-Helper Map aa 151–200

DR1, DR2, DR3, DR4, DR7
I-Ab and HLA-DR
human
DR supermotif
human
H-2t4
DR1, 2 or 3, 4 and 7

QGWKGSPAIFQSSMTKILEPFRKQNPDIVIYQYMDDLYVGSDLEIGQHRT
160 170 180 190 200

Figure 18: RT T-Helper Map aa 201–250

DRS
human
DR5
human
human

KIEELRQHLLRWGLTTPDKKHQKEPPFLWMGYELHPDKWTVQPIVLPEKD
210 220 230 240 250

RT T-Helper Map aa 251–300
DR1, DR2, DR3, DR5, DR7
human
DR5(11.01)
human
DRS
human
DR5
human
human
human
SWTVNDIQKLVGKLNWASQIYPGIKVRQLCKLLRGTKALTEVIPLTEEAE
260 270 280 290 300
Figure 20: RT T-Helper Map aa 301–350
human
human
LELAENREILKEPVHGVYYDPSKDLIAEIQKQGQGQWTYQIYQEPFKNLK
310 320 330 340 350
Figure 21: RT T-Helper Map aa 351–400
human
TGKYARMRGAHTNDVKQLTEAVQKITTESIVIWGKTPKFKLPIQKETWET
360 370 380 390 400
Figure 22: RT T-Helper Map aa 401–450
DR1, DR2, DR3, DR4
human
DR supermotif
WWTEYWQATWIPEWEFVNTPPLVKLWYQLEKEPIVGAETFYVDGAANRET
410 420 430 440 450
Figure 23: RT T-Helper Map aa 451–500
KLGKAGYVTNRGRQKVVTLTDTTNQKTELQAIYLALQDSGLEVNIVTDSQ
460 470 480 490 500
Figure 24: RT T-Helper Map aa 501–550
DR supermotif
I-Ab and HLA-DR
DR supermotif
H-2f,k,d
Ad and Dd
Ad or Dd
YALGIIQAQPDQSESELVNQIIEQLIKKEKVYLAWVPAHKGIGGNEQVDK
510 520 530 540 550

RT T-Helper Map aa 551–560
LVSAGIRKVL
560

13 Nef T-Helper Epitope Map

Nef T-Helper Map aa 1–50

H-2d
H-2d
H-2d
H-2d
H-2d
H-2d
MGGKWSKSSVIGWPTVRERMRRAEPAADRVGAASRDLEKHGAITSSNTAA
10 20 30 40 50

Figure 65: Nef T-Helper Map aa 51–100

H-2b
chimpanzee
DQ2, DQ3, DQ5, DQ6, DQ7, DQ8,
H-2b
H-2d
H-2b
TNAACAWLEAQEEEEVGFPVTPQVPLRPMTYKAAVDLSHFLKEKGGLEGL
60 70 80 90 100

Figure 66: Nef T-Helper Map aa 101–150

H-2b
H-2b
chimpanzee
H-2d
H-2b
H-2b
IHSQRRQDILDLWIYHTQGYFPDXQNYTPGPGVRYPLTFGWCYKLVPVEP
110 120 130 140 150

Figure 67: Nef T-Helper Map aa 151–200

chimpanzee
human
H-2b,d
H-2d
H-2b
H-2d
H-2b
DKIEEANKGENTSLLHPVSLHGMDDPEREVLEWRFDSRLAFHHVARELHP
160 170 180 190 200

Figure 68: Nef T-Helper Map aa 201–206

chimpanzee
EYFKNC
206

### Sequence Listing

SEQ ID NO:1 Artificial sequence – GPN

M A P I V Q N L Q G Q M V H Q A I S P R T L N A W V K V V E E K A F S
P E V I P M F S A L S E G A T P Q D L N T M L N T V G G H Q A A M Q M
L K E T I N E E A A E W D R L H P V H A G P I A P G Q M R E P R G S D
I A G T T S T L Q E Q I G W M T N N P P I P V G E I Y K R W I I L G L
N K I V R M Y S P T S I L D I R Q G P K E P F R D Y V D R F Y K T L R
A E Q A S Q E V K N W M T E T L L V Q N A N P D C K T I L K A L G P A
A T L E E M M T A C Q G V G G P G H K A R V L M A A R A S V L S G G E
L D R W E K I R L R P G G K K K Y K L K H I V W A S R E L E R F A V N
P G L L E T S E G C R Q I L G Q L Q P S L Q T G S E E L R S L Y N T V
A T L Y C V H Q R I E V K D T K E A L E K I E E E Q N K S K K K A Q Q
A A A D T G N S S Q V S Q N Y T P D K K H Q K E P P F L W M G Y E L H
P D K W T V Q P I V L P E K D S W T V N D I Q K L V G K L N W A S Q I
Y A G I K V K Q L C K L L R G T K A L T E V I P L T E E A E L E L A E
N R E I L K E P V H G V Y Y D P S K D L I A E I Q K Q G Q G Q W T Y Q
I Y Q E P F K N L K T G K Y A R M R G A H T N D V K Q L T E A V Q K I
A T E S I V I W G K T P K F K L P I Q K E T W E A W W T E Y W Q A T W
I P E W E F V N T P P L V K L W Y Q L E K E P I V G A E T F P I S P I
E T V P V K L K P G M D G P K V K Q W P L T E E K I K A L V E I C T E
M E K E G K I S K I G P E N P Y N T P V F A I K K K D S T K W R K L V
D F R E L N K R T Q D F W E V Q L G I P H P A G L K K K K S V T V L D
V G D A Y F S V P L D K D F R K Y T A F T I P S I N N E T P G I R Y Q
Y N V L P Q G W K G S P A I F Q S S M T K I L E P F R K Q N P D I V I
Y Q Y M D D L Y V G S D L E I G Q H R T K I E E L R Q H L L R W G F T
T P D K K H Q K E P P F L V W K F D S R L A F H H M A R E L H P E Y Y
K D C D P E K E V L V W K F D A N E G E N N S L L H P M S L H G M D D
P E K E V P E K V E E A N E G E N G P G I R Y P L T F G W C F K L V P
V E P E K V E E W Q N Y T P G P G I R Y Q K R Q D I L D L W V Y H T Q
G Y F P D W Q N Y T P E G L I Y S Q K R Q D I P M T Y K A A L D L S H
F L K E K G G L E G L I Y S P Q V P L R P M T Y K A A D C A W L E A Q
E E E E V G F P V R P Q V P L R N T A A N N A D C A W L A D G V G A V
S R D L E K H G A I T S S N T A A N N R R A E P A A D G V G A M G G K
W S K R S V V G W P T V R E R M R R A E P A R G P G R A F V T I Y P Y
D V P D Y A

SEQ ID NO:2 Artificial sequence – GAG P24

M A P I V Q N L Q G Q M V H Q A I S P R T L N A W V K V V E E K A F S
P E V I P M F S A L S E G A T P Q D L N T M L N T V G G H Q A A M Q M
L K E T I N E E A A E W D R L H P V H A G P I A P G Q M R E P R G S D
I A G T T S T L Q E Q I G W M T N N P P I P V G E I Y K R W I I L G L
N K I V R M Y S P T S I L D I R Q G P K E P F R D Y V D R F Y K T L R
A E Q A S Q E V K N W M T E T L L V Q N A N P D C K T I L K A L G P A
A T L E E M M T A C Q G V G G P G H K A R V L

SEQ ID NO:3 Artificial sequence – GAG P17

M A A R A S V L S G G E L D R W E K I R L R P G G K K K Y K L K H I V
W A S R E L E R F A V N P G L L E T S E G C R Q I L G Q L Q P S L Q T
G S E E L R S L Y N T V A T L Y C V H Q R I E V K D T K E A L E K I E
E E Q N K S K K K A Q Q A A A D T G N S S Q V S Q N Y

SEQ ID NO:4 Artificial sequence – POL P51 duplication

T P D K K H Q K E P P F

SEQ ID NO:5 Artificial sequence – POL P51 core

L W M G Y E L H P D K W T V Q P I V L P E K D S W T V N D I Q K L V G
K L N W A S Q I Y A G I K V K Q L C K L L R G T K A L T E V I P L T E
E A E L E L A E N R E I L K E P V H G V Y Y D P S K D L I A E I Q K Q
G Q G Q W T Y Q I Y Q E P F K N L K T G K Y A R M R G A H T N D V K Q
L T E A V Q K I A T E S I V I W G K T P K F K L P I Q K E T W E A W W
T E Y W Q A T W I P E W E F V N T P P L V K L W Y Q L E K E P I V G A
E T F P I S P I E T V P V K L K P G M D G P K V K Q W P L T E E K I K
A L V E I C T E M E K E G K I S K I G P E N P Y N T P V F A I K K K D
S T K W R K L V D F R E L N K R T Q D F W E V Q L G I P H P A G L K K
K K S V T V L D V G D A Y F S V P L D K D F R K Y T A F T I P S I N N
E T P G I R Y Q Y N V L P Q G W K G S P A I F Q S S M T K I L E P F R
K Q N P D I V I Y Q Y M D D L Y V G S D L E I G Q H R T K I E E L R Q
H L L R W G F T

SEQ ID NO:6 Artificial sequence – scrambled NEF

L V W K F D S R L A F H H M A R E L H P E Y Y K D C D P E K E V L V W
K F D A N E G E N N S L L H P M S L H G M D D P E K E V P E K V E E A
N E G E N G P G I R Y P L T F G W C F K L V P V E P E K V E E W Q N Y
T P G P G I R Y Q K R Q D I L D L W V Y H T Q G Y F P D W Q N Y T P E
G L I Y S Q K R Q D I P M T Y K A A L D L S H F L K E K G G L E G L I
Y S P Q V P L R P M T Y K A A D C A W L E A Q E E E V G F P V R P Q
V P L R N T A A N N A D C A W L A D G V G A V S R D L E K H G A I T S
S N T A A N N R R A E P A A D G V G A M G G K W S K R S V V G W P T V
R E R M R R A E P A

SEQ ID NO:7 Artificial sequence – gp160 murine CD8 tag

R G P G R A F V T I

SEQ ID NO:8 Artificial sequence – HA-tag

Y P Y D V P D Y A

SEQ ID NO: 9 Artificial sequence - core GPN

M A P I V Q N L Q G Q M V H Q A I S P R T L N A W V K V V E E K A F S
P E V I P M F S A L S E G A T P Q D L N T M L N T V G G H Q A A M Q M
L K E T I N E E A A E W D R L H P V H A G P I A P G Q M R E P R G S D
I A G T T S T L Q E Q I G W M T N N P P I P V G E I Y K R W I I L G L
N K I V R M Y S P T S I L D I R Q G P K E P F R D Y V D R F Y K T L R
A E Q A S Q E V K N W M T E T L L V Q N A N P D C K T I L K A L G P A
A T L E E M M T A C Q G V G G P G H K A R V L M A A R A S V L S G G E
L D R W E K I R L R P G G K K K Y K L K H I V W A S R E L E R F A V N
P G L L E T S E G C R Q I L G Q L Q P S L Q T G S E E L R S L Y N T V
A T L Y C V H Q R I E V K D T K E A L E K I E E E Q N K S K K K A Q Q
A A A D T G N S S Q V S Q N Y T P D K K H Q K E P P F L W M G Y E L H
P D K W T V Q P I V L P E K D S W T V N D I Q K L V G K L N W A S Q I
Y A G I K V K Q L C K L L R G T K A L T E V I P L T E E A E L E L A E
N R E I L K E P V H G V Y Y D P S K D L I A E I Q K Q G Q G Q W T Y Q

I Y Q E P F K N L K T G K Y A R M R G A H T N D V K Q L T E A V Q K I
A T E S I V I W G K T P K F K L P I Q K E T W E A W W T E Y W Q A T W
I P E W E F V N T P P L V K L W Y Q L E K E P I V G A E T F P I S P I
E T V P V K L K P G M D G P K V K Q W P L T E E K I K A L V E I C T E
M E K E G K I S K I G P E N P Y N T P V F A I K K K D S T K W R K L V
D F R E L N K R T Q D F W E V Q L G I P H P A G L K K K K S V T V L D
V G D A Y F S V P L D K D F R K Y T A F T I P S I N N E T P G I R Y Q
Y N V L P Q G W K G S P A I F Q S S M T K I L E P F R K Q N P D I V I
Y Q Y M D D L Y V G S D L E I G Q H R T K I E E L R Q H L L R W G F T
T P D K K H Q K E P P F L V W K F D S R L A F H H M A R E L H P E Y Y
K D C D P E K E V L V W K F D A N E G E N N S L L H P M S L H G M D D
P E K E V P E K V E E A N E G E N G P G I R Y P L T F G W C F K L V P
V E P E K V E E W Q N Y T P G P G I R Y Q K R Q D I L D L W V Y H T Q
G Y F P D W Q N Y T P E G L I Y S Q K R Q D I P M T Y K A A L D L S H
F L K E K G G L E G L I Y S P Q V P L R P M T Y K A A D C A W L E A Q
E E E E V G F P V R P Q V P L R N T A A N N A D C A W L A D G V G A V
S R D L E K H G A I T S S N T A A N N R R A E P A A D G V G A M G G K
W S K R S V V G W P T V R E R M R R A E P A

SEQ ID NO:10 Human immunodeficiency virus - Native NEF

M G G K W S K R S V V G W P T V R E R M R R A E P A A D G V G A V S R D L E K H G A I
T S S N T A A N N A D C A W L E A Q E E E E V G F P V R P Q V P L R P M T Y K A A L D L
S H F L K E K G G L E G L I Y S Q K R Q D I L D L W V Y H T Q G Y F P D W Q N Y T P G P
G I R Y P L T F G W C F K L V P V E P E K V E E A N E G E N N S L L H P M S L H G M D D
P E K E V L V W K F D S R L A F H H M A R E L H P E Y Y K D C

SEQ ID NO:11 Artificial sequence - gpn nucleotide sequence

C C G G G C C C G C C G C C A C C A T G G C C C C C A T C G T G C A G
A A C C T G C A A G G C C A G A T G G T G C A C C A G G C C A T C A G
C C C C A G A A C C C T G A A T G C C T G G G T G A A G G T G G T G G
A G G A G A A G G C C T T C A G C C C T G A G G T G A T C C C T A T G
T T C A G C G C C C T G A G C G A G G G C G C C A C C C C C C A G G A
C C T G A A C A C C A T G C T G A A T A C C G T G G G C G G C C A T C
A G G C C G C C A T G C A G A T G C T G A A G G A G A C C A T C A A C
G A G G A G G C C G C C G A G T G G G A C A G A C T G C A C C C C G T
G C A C G C C G G A C C T A T C G C C C C T G G C C A G A T G A G A G
A G C C C A G A G G C A G C G A C A T C G C C G G C A C C A C C A G C
A C C C T G C A A G A G C A G A T C G G C T G G A T G A C C A A C A A
C C C C C C T A T C C C T G T G G G C G A G A T C T A C A A G A G A T
G G A T C A T C C T G G G C C T G A A C A A G A T C G T G A G A A T G
T A C A G C C C T A C C A G C A T C C T G G A C A T C A G A C A G G G
A C C C A A G G A G C C C T T C A G A G A C T A C G T G G A C A G G T
T C T A C A A G A C C C T G A G A G C C G A G C A G G C C A G C C A G
G A A G T G A A G A A C T G G A T G A C C G A G A C C C T G C T G G T
G C A G A A C G C C A A C C C C G A C T G C A A G A C C A T C C T G A
A G G C C C T G G G A C C T G C C G C T A C C C T G G A G G A G A T G
A T G A C C G C C T G C C A G G G C G T G G G C G G A C C T G G C C A
C A A G G C C A G A G T G C T C A T G G C C G C C A G A G C C A G C G
T G C T G A G C G G C G G A G A G C T G G A T A G A T G G G A G A A G
A T C A G A C T G A G A C C T G G C G G C A A G A A G A A G T A C A A
G C T G A A G C A C A T C G T G T G G G C C T C T A G G G A G C T G G
A G A G A T T C G C C G T G A A T C C T G G C C T G C T G G A G A C C
A G C G A G G G C T G T A G A C A G A T C C T G G G C C A G C T G C A
A C C C A G C C T G C A A A C C G G C T C T G A G G A G C T G A G A T
C C C T G T A C A A C A C C G T G G C C A C C C T G T A C T G C G T G
C A C C A G A G A A T C G A G G T G A A G G A C A C A A A G G A G G C
C C T G G A G A A G A T C G A G G A G G A G C A G A A C A A G T C C A
A G A A G A A G G C C C A G C A G G C C G C T G C C G A C A C C G G C
A A C A G C A G C C A G G T G A G C C A G A A C T A C A C C C C C G A
C A A G A A G C A C C A G A A G G A G C C C C C T T T C C T G T G G A
T G G G C T A C G A G C T G C A C C C C G A T A A G T G G A C C G T G
C A G C C C A T C G T G C T G C C T G A G A A G G A T A G C T G G A C
C G T G A A C G A C A T C C A G A A G C T G G T G G G C A A G C T G A

A C T G G G C C A G C C A G A T C T A C G C C G G C A T C A A G G T G
A A G C A G C T G T G T A A G C T G C T G A G A G G C A C C A A G G C
C C T C A C A G A A G T G A T C C C C C T C A C A G A G G A G G C C G
A G C T G G A G C T G G C C G A G A A C A G A G A G A T C C T G A A A
G A G C C C G T G C A C G G C G T G T A C T A C G A C C C C A G C A A
G G A T C T C A T C G C C G A G A T T C A G A A G C A G G G C C A G G
G C C A G T G G A C C T A C C A G A T C T A C C A G G A G C C T T T C
A A G A A C C T G A A A A C C G G C A A G T A C G C C A G A A T G A G
A G G C G C T C A C A C C A A C G A C G T G A A G C A G T T G A C C G
A G G C C G T G C A G A A A A T C G C C A C C G A G A G C A T C G T G
A T C T G G G G C A A G A C C C C C A A G T T C A A G C T G C C T A T
C C A G A A G G A G A C C T G G G A G G C C T G G T G G A C C G A G T
A T T G G C A G G C C A C C T G G A T T C C T G A G T G G G A G T T C
G T G A A C A C C C C C C C T C T G G T G A A G C T G T G G T A T C A
G C T G G A G A A G G A G C C T A T C G T G G G C G C C G A G A C C T
T C C C C A T C A G C C C T A T C G A G A C C G T G C C C G T G A A G
C T G A A G C C C G G C A T G G A C G G C C C C A A G G T G A A A C A
G T G G C C T C T C A C C G A G G A G A A G A T T A A G G C C C T G G
T G G A G A T T T G C A C C G A G A T G G A G A A G G A A G G C A A G
A T C A G C A A G A T C G G C C C C G A G A A T C C C T A C A A T A C
C C C C G T G T T C G C C A T T A A G A A G A A G G A C T C C A C C A
A G T G G A G A A A G C T G G T G G A C T T C A G A G A G C T G A A C
A A G A G A A C C C A G G A C T T C T G G G A G G T G C A G C T G G G
C A T C C C C C A C C C T G C C G G C C T G A A G A A G A A G A A G A
G C G T G A C C G T G C T G G A C G T G G G C G A C G C C T A C T T C
A G C G T G C C C C T G G A C A A G G A C T T C A G A A A G T A C A C
C G C C T T C A C C A T C C C C A G C A T C A A C A A C G A G A C C C
C C G G C A T C A G A T A C C A G T A C A A C G T G C T G C C C C A G
G G C T G G A A G G G C A G C C C C G C C A T C T T C C A G A G C A G
C A T G A C A A A G A T C C T G G A G C C C T T C C G G A A G C A G A
A C C C C G A T A T C G T G A T C T A C C A G T A C A T G G A C G A C
C T G T A C G T G G G C A G C G A T C T G G A G A T C G G C C A G C A
C A G A A C C A A G A T C G A A G A G C T G A G A C A G C A C C T G C
T G A G A T G G G G C T T C A C C A C A C C A G A T A A G A A A C A T
C A G A A A G A A C C A C C A T T C C T G G T G T G G A A G T T T G A
C A G C A G A C T G G C C T T C C A C C A T A T G G C C A G A G A G C
T G C A T C C T G A G T A C T A C A A G G A C T G C G A T C C C G A G
A A G G A G G T G C T G G T C T G G A A A T T C G A C G C C A A C G A
G G G C G A G A A C A A C A G C C T G C T G C A C C C C A T G A G C C

T G C A C G G C A T G G A T G A T C C T G A G A A A G A A G T G C C C
G A G A A G G T G G A A G A G G C C A A T G A G G G A G A A A A C G G
C C C T G G C A T T A G A T A T C C C C T C A C C T T C G G C T G G T
G C T T C A A G C T G G T G C C T G T G G A G C C T G A G A A A G T G
G A G G A A T G G C A G A A T T A C A C A C C C G G C C C A G G C A T
C C G G T A T C A G A A G A G A C A G G A C A T T C T G G A C C T G T
G G G T G T A C C A C A C C C A G G G C T A C T T C C C C G A C T G G
C A G A A C T A T A C T C C T G A G G G C C T C A T C T A C A G C C A
G A A G C G G C A G G A T A T C C C C A T G A C C T A C A A G G C C G
C C C T G G A T C T G A G C C A C T T T C T G A A A G A G A A G G G C
G G C C T G G A G G G C T T G A T C T A C T C C C C A C A G G T G C C
A C T G A G A C C T A T G A C A T A T A A G G C T G C C G A C T G C G
C C T G G C T G G A G G C C C A G G A A G A G G A G G A A G T G G G C
T T C C C T G T G A G A C C C C A G G T G C C C C T G C G G A A C A C
C G C C G C C A A T A A T G C C G A T T G T G C T T G G C T C G C C G
A C G G C G T G G G A G C C G T G A G C A G a G A C C T G G A A A A G
C A C G G C G C C A T C A C C A G C A G C A A T A C A G C C G C C A A
C A A C A G A A G A G C C G A G C C T G C C G C C G A T G G A G T G G
G C G C C A T G G G C G G C A A G T G G A G C A A G A G A T C C G T G
G T G G G C T G G C C C A C C G T G A G A G A A A G A A T G A G A A G
G G C C G A A C C C G C C A G A G G A C C C G G C A G A G C C T T C G
T G A C C A T C T A C C C C T A C G A C G T G C C C G A C T A C G C T
T G A T G A G G C G C G C C C T

5-prime ApaI and 3-prime AscI sites are underlined. Initiating ATG and terminating TGA are in bold.

SEQ ID NO:12 Artificial sequence – disrupted pol

T P D K K H Q K E P P F L W M G Y E L H P D K W T V Q P I V L P E K D
S W T V N D I Q K L V G K L N W A S Q I Y A G I K V K Q L C K L L R G
T K A L T E V I P L T E E A E L E L A E N R E I L K E P V H G V Y Y D
P S K D L I A E I Q K Q G Q G Q W T Y Q I Y Q E P F K N L K T G K Y A
R M R G A H T N D V K Q L T E A V Q K I A T E S I V I W G K T P K F K
L P I Q K E T W E A W W T E Y W Q A T W I P E W E F V N T P P L V K L
W Y Q L E K E P I V G A E T F P I S P I E T V P V K L K P G M D G P K
V K Q W P L T E E K I K A L V E I C T E M E K E G K I S K I G P E N P
Y N T P V F A I K K K D S T K W R K L V D F R E L N K R T Q D F W E V
Q L G I P H P A G L K K K K S V T V L D V G D A Y F S V P L D K D F R
K Y T A F T I P S I N N E T P G I R Y Q Y N V L P Q G W K G S P A I F
Q S S M T K I L E P F R K Q N P D I V I Y Q Y M D D L Y V G S D L E I
G Q H R T K I E E L R Q H L L R W G F T T P D K K H Q K E P P F

SEQ ID NO:13 Artificial sequence – disrupted gag

M A P I V Q N L Q G Q M V H Q A I S P R T L N A W V K V V E E K A F S
P E V I P M F S A L S E G A T P Q D L N T M L N T V G G H Q A A M Q M
L K E T I N E E A A E W D R L H P V H A G P I A P G Q M R E P R G S D
I A G T T S T L Q E Q I G W M T N N P P I P V G E I Y K R W I I L G L
N K I V R M Y S P T S I L D I R Q G P K E P F R D Y V D R F Y K T L R
A E Q A S Q E V K N W M T E T L L V Q N A N P D C K T I L K A L G P A
A T L E E M M T A C Q G V G G P G H K A R V L M A A R A S V L S G G E
L D R W E K I R L R P G G K K K Y K L K H I V W A S R E L E R F A V N
P G L L E T S E G C R Q I L G Q L Q P S L Q T G S E E L R S L Y N T V
A T L Y C V H Q R I E V K D T K E A L E K I E E E Q N K S K K K A Q Q
A A A D T G N S S Q V S Q N Y

SEQ ID NO:14 Kozak sequence for Gag p24
GCC GCC ACC ATG G

## Claims

1. A recombinant polypeptide comprising amino acid sequence derived from
(i) an HIV gag gene product comprising the sequence set forth in SEQ ID NO. 2 & SEQ ID NO. 3;
(ii) an HIV pol gene product comprising the sequence set forth in SEQ ID NO. 5; and
(iii) an HIV nef gene product comprising the sequence set forth in SEQ ID NO. 10,
said sequence being mutated with respect to the natural sequence of said gene products, and said sequence maintaining substantially all of the naturally occurring CD8+ T cell epitopes of said gene products, said polypeptide comprising an amino acid sequence having at least 75% identity to the full length of SEQ ID NO:9.

2. A recombinant polypeptide according to claim 1 wherein the naturally occurring CD8+ T cell epitopes of said gene products are as defined in p17 and p24 (gag), amino acids 1-440 ofRT (pol) and nef shown in Example 8.

3. A recombinant polypeptide according to claim 1 wherein said polypeptide comprises an amino acid sequence having at least 95% identity to the full length of SEQ ID NO:9.

4. A recombinant polypeptide according to any preceding claim wherein the epitopes correspond with 100% sequence identity to the epitopes in SEQ ID No. 9 and the remaining non-epitope regions of the seqeucne have at least 75% identity to the corresponding regions of SEQ ID NO. 9.

5. A recombinant polypeptide according to claim 4 wherein said polypeptide comprises SEQ ID NO:9.

6. A recombinant polypeptide according to any preceding claim wherein substantially all of the naturally occurring T helper epitopes of said gene products are maintained.

7. A recombinant polypeptide according to claim 6 where the naturally occurring T helper epitopes of said gene products are as defined in p17 and p24 (gag), amino acids 1-440 of RT (pol) and nef shown in Example 8

8. A recombinant polypeptide according to any preceding claim, said polypeptide comprising amino acid sequence derived from an HIV nef gene product, said recombinant polypeptide sequence being mutated to disrupt the function of said nef sequence, said nef sequence further comprising one or more T helper epitopes which are not present in the naturally occurring nef gene and wherein the one or more T helper epitope(s) which are not present in the naturally occurring nef sequence are shown in Figure 3A and are absent from Figure 3B.

9. A recombinant polypeptide according to claim 8 further comprising all naturally occuring CD8+ T cell epitopes of the nef gene product.

10. A recombinant polypeptide according to claim 9 wherein the naturally occurring CD8+ T cell epitopes of the nef gene product are as defined in Example 8.

11. A recombinant polypeptide according to any of claims 8 to 10 further comprising all naturally occurring nef human T helper epitopes.

12. A recombinant polypeptide according to claim 11 wherein the naturally occurring nef human T helper epitopes are as defined in Example 8.

13. A recombinant polypeptide according to any of claims 8 to 12 wherein said polypeptide comprises an amino acid sequence as shown in SEQ ID NO:6, or a sequence having at least 95% identity thereto.

14. A recombinant polypeptide according to any preceding claim, said polypeptide comprising amino acid sequence derived from an HIV pol gene product, said recombinant polypeptide sequence being mutated to disrupt the reverse transcriptase activity of the pol sequence, wherein substantially all of the CD8+ T cell epitopes of the naturally occurring pol sequence are retained in said recombinant polypeptide.

15. A recombinant polypeptide according to claim 14 wherein the CD8+ T cell epitopes of the naturally occurring pol sequence are as defined in amino acids 1-440 of RT (pol) shown in Example 8.

16. A recombinant polypeptide according to claim 14 or 15, wherein the reverse transcriptase activity of said pol sequence is mutated by duplication of an internal sequence derived from the centre of the naturally occurring pol gene and exchange of the amino and carboxy terminal portions of said pol sequence.

17. A recombinant polypeptide according to claim 16 wherein said duplicated internal sequence comprises TPDKKHQKEPPF (SEQ LID NO:4).

18. A recombinant polypeptide according to claim 16 or claim 17 wherein said polypeptide comprises an amino acid sequence as shown in SEQ ID NO:12 or a sequence having at least 95% identity thereto.

19. A recombinant polypeptide according to any preceding claim, said polypeptide comprising amino acid sequence derived from an HIV gag gene product, said recombinant polypeptide sequence being mutated to disrupt processing of the gag gene product, and said gag sequence further comprising a disrupted myristoylation site, wherein substantially all of the CD8+ T cell epitopes of the naturally occurring gag sequence are retained in said recombinant polypeptide.

20. A recombinant polypeptide according to claim 19 wherein the CD8+ T cell epitopes of the naturally occurring gag sequence are as defined in p 17 and p24 (gag) shown in Example 8.

21. A recombinant polypeptide according to claim 19 or 20 wherein the processing of gag is disrupted by exchanging the p17 and p24 domains and wherein the myristoylation site is disrupted by mutation of the second glycine to alanine.

22. A recombinant polypeptide according to any one of claims 19 to 21 wherein said polypeptide comprises an amino acid sequence as shown in SEQ ID NO:13 or a sequence having at least 95% identity thereto.

23. A recombinant polypeptide according to any preceding claim further comprising an antibody recognition tag wherein said tag is an HA tag comprising the sequence as shown in SEQ ID NO:8.

24. A recombinant polypeptide according to any preceding claim further comprising a CD8+ T cell epitope tag wherein said tag is a gp160 derived tag comprising the sequence as shown in SEQ ID NO: 7.

25. A recombinant polypeptide according to any preceding claim, said polypeptide comprising the sequence as shown in SEQ ID NO: 1.

26. A recombinant polypeptide according to any preceding claim wherein the HIV is a clade B HIV.

27. A recombinant nucleic acid encoding a polypeptide according to any preceding claim.

28. A recombinant nucleic acid sequence comprising SEQ ID NO:11, or a sequence which differs only by silent mutations with respect to the genetic code, or a sequence having at least 95% identity thereto.

29. A viral vector encoding a polypeptide according to any one of claims 1 to 26, said viral vector being selected from the group consisting of poxviruses, adenoviruses, AAV, alphavirus, VSV, HSV and Sendai virus.

30. A viral vector according to claim 29 wherein said vector is an MVA or MVA derived vector.

31. A viral vector according to claim 29 wherein said vector is a fowlpox or fowlpox derived vector.

32. A viral vector according to claim 31 wherein said vector is an FP9 fowlpox vector.

33. A nucleic acid vector comprising a nucleic acid sequence according to claim 27 or claim 28 or encoding a polypeptide according to any one of claims 1 to 26.

34. An adenovirus vector comprising a nucleic acid sequence according to claim 27 or claim 28 or encoding a polypeptide according to any one of claims 1 to 26.

35. A poxvirus vector comprising a nucleic acid sequence according to claim 27 or claim 28 or encoding a polypeptide according to any one of claims 1 to 26.

36. Use of polypeptide according to any one of claims 1 to 26 in the preparation of a medicament for immunisation against HIV infection.

37. A Herpes Simplex Virus (HSV) vector comprising a nucleic acid sequence according to claim 27 or claim 28 or encoding a polypeptide according to any one of claims 1 to 26.

## Patentansprüche

1. Rekombinantes Polypeptid, welches Aminosäuresequenz umfaßt, die abgeleitet ist von
(i) einem HIV-gag-Genprodukt, welches die in SEQ ID NO: 2 und SEQ ID NO: 3 aufgeführte Sequenz umfaßt,
(ii) einem HIV-pol-Genprodukt, welches die in SEQ ID NO: 5 aufgeführte Sequenz umfaßt, und
(iii) einem HIV-nef-Genprodukt, welches die in SEQ ID NO: 10 aufgeführte Sequenz umfaßt,
wobei die Sequenz hinsichtlich der natürlichen Sequenz der Genprodukte mutiert ist und die Sequenz im wesentlichen alle der natürlich vorkommenden CD8+-T-Zell-Epitope der Genprodukte aufrechterhält, wobei das Polypeptid eine Aminosäuresequenz mit wenigstens 75% Identität zur vollen Länge von SEQ ID NO: 9 umfaßt.

2. Rekombinantes Polypeptid nach Anspruch 1, wobei die natürlich vorkommenden CD8+-T-Zell-Epitope der Genprodukte wie in p17 und p24 (gag), den Aminosäuren 1-440 von RT (pol) und nef, wie in Beispiel 8 gezeigt, definiert sind.

3. Rekombinantes Polypeptid nach Anspruch 1, wobei das Polypeptid eine Aminosäuresequenz mit wenigstens 95% Identität zur vollen Länge von SEQ ID NO: 9 umfaßt.

4. Rekombinantes Polypeptid nach einem der vorangegangenen Ansprüche, wobei die Epitope mit 100% Sequenzidentität den Epitopen in SEQ ID NO: 9 entsprechen und die verbleibenden Nicht-Epitop-Regionen der Sequenz wenigstens 75% Identität zu den korrespondierenden Regionen von SEQ ID NO: 9 haben.

5. Rekombinantes Polypeptid nach Anspruch 4, wobei das Polypeptid SEQ ID NO: 9 umfaßt.

6. Rekombinantes Polypeptid nach einem der vorangegangenen Ansprüche, wobei im wesentlichen alle der natürlich vorkommenden T-Helfer-Epitope der Genprodukte aufrechterhalten werden.

7. Rekombinantes Polypeptid nach Anspruch 6, wobei die natürlich vorkommenden T-Helfer-Epitope der Genprodukte wie in p17 und p24 (gag), den Aminosäuren 1-440 von RT (pol) und nef, wie in Beispiel 8 gezeigt, definiert sind.

8. Rekombinantes Polypeptid nach einem der vorangegangenen Ansprüche, wobei das Polypeptid von einem HIV-nef-Genprodukt abgeleitete Aminosäuresequenz umfaßt, wobei die rekombinante Polypeptidsequenz mutiert ist, um die Funktion der nef-Sequenz zu stören, wobei die nef-Sequenz weiterhin ein oder mehrere T-Helfer-Epitope umfaßt, die in dem natürlich vorkommenden nef-Gen nicht vorkommen und wobei das eine oder die mehreren T-Helfer-Epitop(e), das bzw. die in der natürlich vorkommenden nef-Sequenz nicht vorliegt bzw. vorliegen, in Figur 3A gezeigt ist bzw. sind und in Figur 3B fehlt bzw. fehlen.

9. Rekombinantes Polypeptid nach Anspruch 8, welches weiterhin alle natürlich vorkommenden CD8+-T-Zell-Epitope des nef-Genprodukts umfaßt.

10. Rekombinantes Polypeptid nach Anspruch 9, wobei die natürlich vorkommenden CD8+-T-Zell-Epitopde des nef-Genprodukts wie in Beispiel 8 definiert sind.

11. Rekombinantes Polypeptid nach einem der Ansprüche 8 bis 10, welches weiterhin alle natürlich vorkommenden humanen nef-T-Helfer-Epitope umfaßt.

12. Rekombinantes Polypeptid nach Anspruch 11, wobei die natürlich vorkommenden humanen nef-T-Helfer-Epitope wie in Beispiel 8 definiert sind.

13. Rekombinantes Polypeptid nach einem der Ansprüche 8 bis 12, wobei das Polypeptid eine Aminosäuresequenz, wie sie in SEQ ID NO: 6 gezeigt ist, oder eine Sequenz mit wenigstens 95% Identität dazu umfaßt.

14. Rekombinantes Polypeptid nach einem der vorangegangenen Ansprüche, wobei das Polypeptid von einem HIV-pol-Genprodukt abgeleitete Aminosäuresequenz umfaßt, wobei die rekombinante Polypeptidsequenz mutiert ist, um die reverse Transkriptase-Aktivität der pol-Sequenz zu stören, wobei im wesentlichen alle der CD8+-T-Zell-Epitope der natürlich vorkommenden pol-Sequenz in dem rekombinanten Polypeptid erhalten werden.

15. Rekombinantes Polypeptid nach Anspruch 14, wobei die CD8+-T-Zell-Epitope der natürlich vorkommenden pol-Sequenz wie in den Aminosäuren 1-440 von RT (pol), gezeigt in Beispiel 8, definiert sind.

16. Rekombinantes Polypeptid nach Anspruch 14 oder 15, wobei die reverse Transkriptase-Aktivität der pol-Sequenz durch Verdoppelung einer internen Sequenz, abgeleitet vom Zentrum des natürlich vorkommenden pol-Gens, und Austausch der amino- und carboxyterminalen Abschnitte der pol-Sequenz mutiert ist.

17. Rekombinantes Polypeptid nach Anspruch 16, wobei die verdoppelte interne Sequenz TPDKKHQKEPPF (SEQ ID NO: 14) umfaßt.

18. Rekombinantes Polypeptid nach Anspruch 16 oder Anspruch 17, wobei das Polypeptid eine Aminosäuresequenz wie in SEQ ID NO: 12 gezeigt oder eine Sequenz mit wenigstens 95% Identität dazu umfaßt.

19. Rekombinantes Polypeptid nach einem der vorangegangenen Ansprüche, wobei das Polypeptid von einem HIV-gag-Genprodukt abgeleitete Aminosäuresequenz umfaßt, wobei die rekombinante Polypeptidsequenz mutiert ist, so daß die Prozessierung des gag-Genprodukts gestört ist, und die gag-Sequenz weiterhin eine gestörte Myristoylierungsstelle umfaßt, wobei im wesentlichen alle der CD8+-T-Zell-Epitope der natürlich vorkommenden gag-Sequenz in dem rekombinanten Polypeptid erhalten werden.

20. Rekombinantes Polypeptid nach Anspruch 19, wobei die CD8+-T-Zell-Epitope der natürlich vorkommenden gag-Sequenz wie in p17 und p24 (gag), gezeigt in Beispiel 8, definiert sind.

21. Rekombinantes Polypeptid nach Anspruch 19 oder 20, wobei die Prozessierung von gag durch Austauschen der p17- und p24-Domänen gestört ist und wobei die Myristoylierungsstelle durch Mutation des zweiten Glycins zu Alanin gestört ist.

22. Rekombinantes Polypeptid nach einem der Ansprüche 19 bis 21, wobei das Polypeptid eine Aminosäuresequenz wie in SEQ ID NO: 13 gezeigt oder eine Sequenz mit wenigstens 95% Identität dazu umfaßt.

23. Rekombinantes Polypeptid nach einem der vorangegangenen Ansprüche, welches weiterhin eine Antikörper-Erkennungsmarkierung umfaßt, wobei die Markierung eine HA-Markierung ist, welche die Sequenz wie in SEQ ID NO: 8 gezeigt umfaßt.

24. Rekombinantes Polypeptid nach einem der vorangegangenen Ansprüche, welches weiterhin eine CD8+-T-Zell-Epitop-Markierung umfaßt, wobei die Markierung eine von gp160 abgeleitete Markierung ist, welche die Sequenz wie in SEQ ID NO: 7 gezeigt umfaßt.

25. Rekombinantes Polypeptid nach einem der vorangegangenen Ansprüche, wobei das Polypeptid die Sequenz wie in SEQ ID NO: 1 gezeigt umfaßt.

26. Rekombinantes Polypeptid nach einem der vorangegangenen Ansprüche, wobei der HIV ein HIV-Stamm B ist.

27. Rekombinante Nukleinsäure, welche ein Polypeptid nach einem der vorangegangenen Ansprüche codiert.

28. Rekombinante Nukleinsäuresequenz, welche SEQ ID NO: 11 umfaßt, oder eine Sequenz, welche sich nur durch stille Mutationen hinsichtlich des genetischen Codes unterscheidet, oder eine Sequenz mit wenigstens 95% Identität dazu.

29. Virusvektor, welcher ein Polypeptid nach einem der Ansprüche 1 bis 26 codiert, wobei der Virusvektor aus der Gruppe ausgewählt ist, bestehend aus Pockenviren, Adenoviren, AAV, AIphavirus, VSV, HSV und Sendai-Virus.

30. Virusvektor nach Anspruch 29, wobei der Vektor ein MVA- oder ein MVA-abgeleiteter Vektor ist.

31. Virusvektor nach Anspruch 29, wobei der Vektor ein Geflügelpocken- oder ein Geflügelpocken-abgeleiteter Virus ist.

32. Virusvektor nach Anspruch 31, wobei der Vektor ein FP9-Geflügelpockenvektor ist.

33. Nukleinsäurevektor, welcher eine Nukleinsäuresequenz nach Anspruch 27 oder Anspruch 28 umfaßt oder ein Polypeptid nach einem der Ansprüche 1 bis 26 codiert.

34. Adenovirusvektor, welcher eine Nukleinsäuresequenz nach Anspruch 27 oder Anspruch 28 umfaßt oder ein Polypeptid nach einem der Ansprüche 1 bis 26 codiert.

35. Pockenvirusvektor, welcher eine Nukleinsäuresequenz nach Anspruch 27 oder Anspruch 28 umfaßt oder ein Polypeptid nach einem der Ansprüche 1 bis 26 codiert.

36. Verwendung eines Polypeptids nach einem der Ansprüche 1 bis 26 bei der Herstellung eines Medikaments zur Immunisierung gegen HIV-Infektion.

37. Herpes-Simplex-Virus- (HSV-) Vektor, welcher eine Nukleinsäuresequenz nach Anspruch 27 oder Anspruch 28 umfaßt oder ein Polypeptid nach einem der Ansprüche 1 bis 26 codiert.

## Revendications

1. Polypeptide recombinant comprenant une séquence d'aminoacides dérivée
(i) d'un produit du gène gag du VIH comprenant la séquence indiquée dans la SEQ ID N° 2 et la SEQ ID N° 3 ;
(ii) d'un produit du gène pol du VIH comprenant la séquence indiquée dans la SEQ ID N° 5 ; et
(iii) d'un produit du gène nef du VIH comprenant la séquence indiquée dans la SEQ ID N° 10,
ladite séquence étant mutée par rapport à la séquence naturelle desdits produits de gènes, et ladite séquence conservant pratiquement la totalité des épitopes de lymphocytes T CD8+ naturels desdits produits de gènes, ledit polypeptide comprenant une séquence d'aminoacides présentant une identité d'au moins 75 % avec la totalité de la SEQ ID N° 9.

2. Polypeptide recombinant suivant la revendication 1, dans lequel les épitopes de lymphocytes T CD8+ naturels desdits produits de gènes sont tels que définis dans p17 et p24 (gag), les aminoacides 1-440 de RT (pol) et nef indiqués dans l'exemple 8.

3. Polypeptide recombinant suivant la revendication 1, ledit polypeptide comprenant une séquence d'aminoacides présentant une identité d'au moins 95 % avec la totalité de la SEQ ID N° 9.

4. Polypeptide recombinant suivant l'une quelconque des revendications précédentes, dans lequel les épitopes correspondent à une identité de séquence de 100 % avec les épitopes dans la SEQ ID N° 9 et les régions non épitopiques restantes de la séquence présentent une identité d'au moins 75 % avec les régions correspondantes de la SEQ ID N° 9.

5. Polypeptide recombinant suivant la revendication 4, ledit polypeptide comprenant la SEQ ID N° 9.

6. Polypeptide recombinant suivant l'une quelconque des revendications précédentes, dans lequel pratiquement la totalité des épitopes de lymphocytes T auxiliaires naturels desdits produits de gènes sont maintenus.

7. Polypeptide recombinant suivant la revendication 6, dans lequel les épitopes de lymphocytes T auxiliaires naturels desdits produits de gènes sont tels que définis dans p17 et p24 (gag), les aminoacides 1-440 de RT (pol) et nef indiqués dans l'exemple 8.

8. Polypeptide recombinant suivant l'une quelconque des revendications précédentes, ledit polypeptide comprenant une séquence d'aminoacides dérivée d'un produit du gène nef du VIH, ladite séquence de polypeptide recombinant étant mutée pour supprimer la fonction de ladite séquence nef, ladite séquence nef comprenant en outre un ou plusieurs épitopes de lymphocytes T auxiliaires qui ne sont pas présents dans le gène nef naturel et dans laquelle le ou les épitopes de lymphocytes T auxiliaires qui ne sont pas présents dans la séquence nef naturelle sont représentés sur la figure 3A et sont absents de la figure 3B.

9. Polypeptide recombinant suivant la revendication 8, comprenant en outre tous les épitopes de lymphocytes T CD8+ naturels du produit du gène nef.

10. Polypeptide recombinant suivant la revendication 9, dans lequel les épitopes de lymphocytes T CD8+ naturels du produit du gène nef sont tels que définis dans l'exemple 8.

11. Polypeptide recombinant suivant l'une quelconque des revendications 8 à 10, comprenant en outre tous les épitopes de lymphocytes T auxiliaires humains nef naturels.

12. Polypeptide recombinant suivant la revendication 11, dans lequel les épitopes de lymphocytes T auxiliaires humains nef naturels sont tels que définis dans l'exemple 8.

13. Polypeptide recombinant suivant l'une quelconque des revendications 8 à 12, ledit polypeptide comprenant une séquence d'aminoacides représentée dans la SEQ ID N° 6, ou une séquence présentant une identité d'au moins 95 % avec celle-ci.

14. Polypeptide recombinant suivant l'une quelconque des revendications précédentes, ledit polypeptide comprenant une séquence d'aminoacides dérivée d'un produit du gène pol du VIH, ladite séquence de polypeptide recombinant étant mutée pour supprimer l'activité de transcriptase inverse de la séquence de pol, pratiquement la totalité des épitopes de lymphocytes T CD8+ de la séquence de pol naturelle étant retenus dans ledit polypeptide recombinant.

15. Polypeptide recombinant suivant la revendication 14, dans lequel les épitopes de lymphocytes T CD8+ de la séquence de pol naturelle sont tels que définis dans les aminoacides 1-440 de RT (pol) représentés dans l'exemple 8.

16. Polypeptide recombinant suivant la revendication 14 ou 15, dans lequel l'activité de transcriptase inverse de ladite séquence de pol est mutée par duplication d'une séquence interne dérivée du centre du gène pol naturel et échange des portions amino- et carboxy-terminales de ladite séquence de pol.

17. Polypeptide recombinant suivant la revendication 16, dans lequel ladite séquence interne dupliquée comprend TPDKKHQKEPPF (SEQ ID N° 4).

18. Polypeptide recombinant suivant la revendication 16 ou la revendication 17, ledit polypeptide comprenant une séquence d'aminoacides représentée dans la SEQ ID N° 12 ou une séquence présentant une identité d'au moins 95 % avec celle-ci.

19. Polypeptide recombinant suivant l'une quelconque des revendications précédentes, ledit polypeptide comprenant une séquence d'aminoacides dérivée d'un produit du gène gag du VIH, ladite séquence de polypeptide recombinant étant mutée pour supprimer la maturation du produit du gène gag, et ladite séquence de gag comprenant en outre un site de myristoylation dissocié, où pratiquement la totalité des épitopes des lymphocytes T CD8+ de la séquence de gag naturelle sont retenus dans ledit polypeptide recombinant.

20. Polypeptide recombinant suivant la revendication 19, dans lequel les épitopes de lymphocytes T CD8+ de la séquence de gag naturelle sont tels que définis dans p17 et p24 (gag) indiqués dans l'exemple 8.

21. Polypeptide recombinant suivant la revendication 19 ou 20, dans lequel la maturation de gag est rompue par échange des domaines p17 et p24, et dans lequel le site de myristoylation est rompu par mutation du deuxième résidu glycine en un résidu alanine.

22. Polypeptide recombinant suivant l'une quelconque des revendications 19 à 21, ledit polypeptide comprenant une séquence d'aminoacides représentée dans la SEQ ID N° 13 ou une séquence présentant une identité d'au moins 95 % avec celle-ci.

23. Polypeptide recombinant suivant l'une quelconque des revendications précédentes, comprenant en outre un marqueur de reconnaissance d'anticorps, dans lequel ledit marqueur est un marqueur HA comprenant la séquence représentée dans la SEQ ID N° 8.

24. Polypeptide recombinant suivant l'une quelconque des revendications précédentes, comprenant en outre un marqueur d'épitope de lymphocytes T CD8+, dans lequel ledit marqueur est un marqueur dérivé de gp160 comprenant la séquence représentée dans la SEQ ID N° 7.

25. Polypeptide recombinant suivant l'une quelconque des revendications précédentes, ledit polypeptide comprenant la séquence représentée dans la SEQ ID N° 1.

26. Polypeptide recombinant suivant l'une quelconque des revendications précédentes, dans lequel le VIH est un VIH du clade B.

27. Acide nucléique recombinant codant pour un polypeptide suivant l'une quelconque des revendications précédentes.

28. Séquence d'acide nucléique recombinant comprenant la SEQ ID N° 11, ou une séquence qui diffère seulement par des mutations silencieuses en ce qui concerne le code génétique, ou une séquence présentant une identité d'au moins 95 % avec celle-ci.

29. Vecteur viral codant pour un polypeptide suivant l'une quelconque des revendications 1 à 26, ledit vecteur viral étant choisi dans le groupe consistant en des poxvirus, des adénovirus, l'AAV, un alphavirus, le VSV, le HSV et le virus Sendai.

30. Vecteur viral suivant la revendication 29, ledit vecteur étant un MVA ou un vecteur dérivé du MVA.

31. Vecteur viral suivant la revendication 29, ledit vecteur étant l'agent du *molluscum contagiosum* ou un vecteur qui en est dérivé.

32. Vecteur viral suivant la revendication 31, ledit vecteur étant un vecteur *molluscum contagiosum* FP9.

33. Vecteur d'acide nucléique comprenant une séquence d'acide nucléique suivant la revendication 27 ou la revendication 28 ou codant pour un polypeptide suivant l'une quelconque des revendications 1 à 26.

34. Vecteur adénoviral comprenant une séquence d'acide nucléique suivant la revendication 27 ou la revendication 28 ou codant pour un polypeptide suivant l'une quelconque des revendications 1 à 26.

35. Vecteur poxviral comprenant une séquence d'acide nucléique suivant la revendication 27 ou la revendication 28 ou codant pour un polypeptide suivant l'une quelconque des revendications 1 à 26.

36. Utilisation d'un polypeptide suivant l'une quelconque des revendications 1 à 26 dans la préparation d'un médicament destiné à l'immunisation contre l'infection par le VIH.

37. Vecteur du type virus Herpes simplex (HSV), comprenant une séquence d'acide nucléique suivant la revendication 27 ou la revendication 28 ou codant pour un polypeptide suivant l'une quelconque des revendications 1 à 26.
